# EUROPEAN PATENT SPECIFICATION

(11) **EP 4 499 869 B1**
(45) Date of publication and mention of the grant of the patent: **15.07.2026**
(21) Application number: 23713394.7
(22) Date of filing: 31.03.2023
(51) Int. Cl.: C12Q 1/686, C12Q 1/689

(54) **PRIMERS AND PROBES FOR DETECTING MYCOBACTERIA**
PRIMER UND SONDEN ZUM NACHWEIS VON MYCOBAKTERIEN
AMORCES ET SONDES POUR LA DÉTECTION DES MYCOBACTÉRIES

(30) Priority: 31.03.2022 EP 22305428
(43) Date of publication of application: 05.02.2025
(73) Proprietor: Sanofi R&D Vaccins, 69007 Lyon (FR)
(72) Inventor: FERNANDEZ, Clothilde, 75017 Paris (FR); MARIUS, Marine, 75017 Paris (FR)
(74) Representative: Ernest Gutmann - Yves Plasseraud S.A.S.
(86) International application number: PCT/EP2023/058475
(87) International publication number: WO 2023/187166

(56) References cited:
- US-A1- 2016 281 141
- DEGGIM-MESSMER VANESSA ET AL: "Diagnostic Molecular Mycobacteriology in Regions With Low Tuberculosis Endemicity: Combining Real-time PCR Assays for Detection of Multiple Mycobacterial Pathogens With Line Probe Assays for Identification of Resistance Mutations", EBIOMEDICINE, vol. 9, 1 July 2016 (2016-07-01), NL, pages 228 - 237, XP055960859, ISSN: 2352-3964, DOI: 10.1016/j.ebiom.2016.06.016
- BAHRMAND A R ET AL: "Polymerase chain reaction of bacterial genomes with single universal primer: application to distinguishing mycobacteria species", MOLECULAR AND CELLULAR PROBES, ACADEMIC PRESS, LONDON, GB, vol. 10, no. 2, 1 April 1996 (1996-04-01), pages 117 - 122, XP002448505, ISSN: 0890-8508, DOI: 10.1006/MCPR.1996.0016
- GARCÍA-QUINTANILLA ALBERT ET AL: "Simultaneous Identification of Mycobacterium Genus and Mycobacterium tuberculosis Complex in Clinical Samples by 5'-Exonuclease Fluorogenic PCR", vol. 40, no. 12, 1 December 2002 (2002-12-01), US, pages 4646 - 4651, XP055961249, ISSN: 0095-1137, Retrieved from the Internet <URL:https://www.ncbi.nlm.nih.gov/pmc/articles/PMC154622/pdf/0754.pdf> DOI: 10.1128/JCM.40.12.4646-4651.2002
- MORRIS CAITLIN ET AL: "Adventitious agent detection methods in bio-pharmaceutical applications with a focus on viruses, bacteria, and mycoplasma", CURRENT OPINION IN BIOTECHNOLOGY, LONDON, GB, vol. 71, 27 July 2021 (2021-07-27), pages 105 - 114, XP086825364, ISSN: 0958-1669, [retrieved on 20210727], DOI: 10.1016/J.COPBIO.2021.06.027
- VENKATESWARA RAO KARANAM ET AL: "Detection of indicator pathogens from pharmaceutical finished products and raw materials using multiplex PCR and comparison with conventional microbiological methods", JOURNAL OF INDUSTRIAL MICROBIOLOGY & BIOTECHNOLOGY, SPRINGER, BERLIN, DE, vol. 35, no. 9, 3 June 2008 (2008-06-03), pages 1007 - 1018, XP019596376, ISSN: 1476-5535
- KAWAI M. ET AL: "Bacterial population dynamics and community structure in a pharmaceutical manufacturing water supply system determined by real-time PCR and PCR-denaturing gradient gel electrophoresis", JOURNAL OF APPLIED MICROBIOLOGY, vol. 97, no. 6, 1 December 2004 (2004-12-01), GB, pages 1123 - 1131, XP055961335, ISSN: 1364-5072, DOI: 10.1111/j.1365-2672.2004.02396.x

## Description

The present invention is in the domain of detection of mycobacteria, especially in biological samples and in pharmaceutical products. The invention also relates to safety release tests regarding mycobacteria detection, which tests are required for all products expressed in or based on eukaryotic cells, *inter alia* for vaccines. The invention also concerns oligonucleotides, especially primers and probes, useful for specifically and exhaustively detecting mycobacteria in samples.

### Background of the invention

Mycobacteria are part of the Mycobacteriaceae family. The phylogeny and nomenclature of mycobacteria is rapidly evolving. Before February 2018, all species of mycobacteria were grouped into a single genus, namely *Mycobacterium.* Since then and the publication by Gupta R. *et al,* 2018, more than 188 species of mycobacteria have been referenced, grouped into 5 different genera, namely *Mycobacterium, Mycobacteroides, Mycolicibacterium, Mycolicibacter* and *Mycolicibacillus.*

The nomenclature has moreover recently been slightly modified (See Riojas M.A. *et al,* 2018). It is now admitted that there are 200 confirmed species of mycobacteria, 6 of which comprise subspecies or variants, and thus 217 confirmed species, subspecies or variants of mycobacteria. A subsequent change has modified the classification of the mycobacteria reconstituting the single *Mycobacterium* genus, however without modifying neither the strains considered as mycobacteria nor their number (Meehan CJ *et al.*, 2021)*.*

Amongst these different species, some of them are pathogenic for humans, such as *M*. *tuberculosis,* the causative pathogen of tuberculosis, and *M. leprae*, the causative pathogen of leprosy.

Other mycobacteria, named nontuberculous mycobacteria (NTM), may be opportunistic pathogens for humans, associated with both pulmonary and extra-pulmonary infections (as *M*. *avium* and *M*. *marinum* respectively). Other species are however commensal bacteria.

The growth kinetics of mycobacteria is very variable and much slower than growth kinetics of most other bacteria; indeed, the fast-growing mycobacteria have a reproductive cycle of around 48 to 72 hours whereas it can be between 10 days and 2 months for the slow-growing species. For comparison, the growth rate of *E. coli* is 20 minutes.

Mycobacteria are widely spread in various environments, such as water and soil, and may have several hosts (human, bovine, protozoa), which makes their detection highly challenging (Haig *et al,* 2018). Mycobacteria could potentially contaminate biopharmaceutical products, such as vaccines, during the production process, through the raw materials of animal or human origin used in the process, such as cells and sera. The contamination may also be brought by the operators, or by the environment, such as water.

Mycobacteria are thus screened in vaccine products, especially non-inactivated viral vaccine products, and in the banks of cells and viral seeds to be used in vaccine production.

Mycobacteria detection is a safety release test, that could be required for all biological or pharmaceutical products expressed in or based on eukaryotic cells. It is required for viral vaccines at the virus seed lots and virus harvest stages by the European pharmacopeia; equivalent requirements are to be found in the Japanese and Chinese pharmacopeia; mycobacteria testing is also performed in the US to ensure the safety of the pharmaceutical products.

The current method described in the European pharmacopeia as safety release test is based on microbiological culture (see European Pharmacopoeia. Chapter 2.6.2 and Technical Report Series - TRS 978 of WHO). It requires to inoculate the sample in triplicate onto two suitable solid media (Löwenstein-Jensen medium and Middlebrook 7H10 medium are considered suitable solid medium) and into one suitable liquid medium. All the media are to be incubated for 56 days at 36.5°C ± 1.5°C.

The main problems and limitations of the compendial culture-based assay described above are *inter alia* the availability and potential shortages of the specific media, the very long time to result (56 days), and the absence of detection of some mycobacteria, like intracellular mycobacteria such as *M*. *intracellulare.* An important human origin species, namely *M*. *leprae,* which is an obligatory intracellular parasite is not detected by the test mentioned above. There is thus moreover a safety risk associated with the absence of detection of this species according to the compendial method. The culture-based assays are also unable to detect fastidious species with specific growth needs, such as *M. genavense* that needs longer incubation times (i.e. 8-12 weeks) or *M*. *haemophilum* that needs iron medium supplementation.

Some alternative methods have been disclosed in the art, based on PCR (polymerase chain reaction) or qPCR (quantitative PCR), targeting different regions of the mycobacterial genome. These methods have the potential to overcome at least some of the limitations of the culture-based techniques, as they target bacterial DNA and therefore should not be constrained by the necessity to culture the microorganism.

Some of these alternative techniques developed for detecting mycobacteria based on a qPCR step were however coupled to days of culture, thus still requiring at least one month to be completed. These methods therefore do not solve the time limitation of the compendial culture-based assay nor the problem of detection of intracellular mycobacteria.

Other methods were developed using qPCR targeting the ITS sequences and 16S sequences, but on the previous taxonomy mycobacteria (2018). These methods generally display a lack of sensitivity and/or susceptibility, especially once applied on the new taxonomy (2018).

Another technology has been developed based on a filtration of high volumes (water samples), immunocapture and qPCR targeting the hsp65 gene. This technology however does not ensure sufficient sensitivity as not all mycobacteria are detected according to this method.

Indeed, in view of the lack of data regarding the corresponding sequences over the whole mycobacteria species, there are strong indications that the targeted sequences in the methods disclosed in the prior art do not allow an exhaustive detection of mycobacteria, representing an important safety risk. Finally, most of the sequences targeted by these alternative methods were not specific to mycobacteria, such that non mycobacterial sequences were also amplified, giving rise to false positives and unreliable results.

It is moreover stressed that most of the detection methods based on PCR disclosed in the art aim at diagnostic purposes, detecting only mycobacteria pathogenic for humans, such that exhaustivity over the *Mycobacterium* genus was not even an objective, as well as specificity. Similarly, most of the methods were intended for detection in samples obtained from patients, likely to present important concentrations of mycobacteria; such that sensitivity was not tested, and/or too low to be compatible with detection of contaminations in biopharmaceutical products, requiring detection at concentrations generally below 100 viable mycobacteria per mL.

None of the prior techniques was thus providing the required safety, efficiency and robustness, in order to meet the requirements of a safety release test. There is thus still an important need for an alternative to the compendial method with an improved exhaustivity and more rapid. There is thus still an important need for an alternative to the compendial method, providing the same specificity but with an improved exhaustivity, and which is efficient, robust and more rapid. There is thus still an important need for an alternative to the compendial method, providing the same specificity but with an improved exhaustivity, and which is efficient, robust and more rapid and which can be as sensitive as the compendial method. Using genome analysis and bioinformatics, applied on the new taxonomy, the inventors have unexpectedly found a combination of primers, adapted to PCR and which detect all the known strains and species of mycobacteria. The method based on this combination thus allows the detection of mycobacterial species, in a sample, in an exhaustive manner, i.e. any species of mycobacteria potentially present is detected, irrespective of the classification of the mycobacteria, irrespective of whether it is a human pathogen or not, irrespective of whether it is a fast-growing or slow-growing mycobacterium, irrespective whether the mycobacteria are at high concentrations like in a human body fluid sample, or at very low concentrations as a contaminant.

Moreover, the inventors have also found a combination of primers and probes, able to specifically detect all the known strains and species of mycobacteria. The method based on this combination thus allows the specific detection of mycobacterial species, in a sample, in an exhaustive and specific manner, i.e. any species, subspecies or variants of mycobacteria is detected, but no other bacteria, especially none of the phylogenetically close species, is detected.

US2016281141 discloses a kit comprising of a set of primers and probes used for specific detection of Mycobacterium tuberculosis and non-tuberculous Mycobacteria as well as primers for amplifying and probes for reporting 16S rRNA gene of non-tuberculous mycobacteria.

### Summary of the invention:

The invention is directed to a set of primers for amplifying by Polymerase Chain Reaction (PCR) a sequence of any mycobacterial *rrs* gene present in a sample, wherein said set comprises at least the three following pairs of primers:
1^{st} Pair:
   Forward primer F1 having the sequence: 5'-CCTGGTAGTCCACGCCGTAA-3' (SEQ ID NO:1) or modifications thereof,
   Reverse primer R1 having the sequence: 5'-CGGATCCCAAGGAAGGAAAC-3' (SEQ ID NO:2) or modifications thereof;
2^{nd} Pair:
   Forward primer F3 having the sequence: 5'-CACAGGACGCCGGTAGAGAT-3' (SEQ ID NO:4) or modifications thereof,
   Reverse primer R3 having the sequence: 5'-CATGCACCACCTGCACACA-3' (SEQ ID NO:5) or modifications thereof;
3^{rd} Pair:
   Forward primer F4 having the sequence: 5'-CCCTTATGTCCAGGGCTTCA-3' (SEQ ID NO:7) or modifications thereof,
   Reverse primer R4 having the sequence: 5'-GGCATCGCAGCCCTTTG-3' (SEQ ID NO:8) or modifications thereof;

wherein said modifications are the addition and/or the deletion of 1 to 4 nucleotides, at the 3' and/or 5' terminus, and/or the substitution of 1 or 2 nucleotides, and
wherein said three pairs of primers allow the amplification of a sequence from the *rrs* gene of any mycobacterial species.

According to particular embodiments, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
5) Forward primer F1-2: 5'-CCTGGTAGTCCACGCCGTAAA-3' (SEQ ID NO:10) Reverse primer R1-3: 5'- CACGGATCCCAAGGAAGGAAAC-3' (SEQ ID NO:27);
6) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12);
7) Forward primer F1-2: 5'-CCTGGTAGTCCACGCCGTAAA-3' (SEQ ID NO:10) Reverse primer R1-6: 5'-GGATCCCAAGGAAGGAAACC-3' (SEQ ID NO:13); and
8) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13).

According to particular embodiments, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
5) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15);
6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
7) Forward primer F3-1: 5'-CACAGGACGCCGGTAGAGAT-3' (SEQ ID NO:4) Reverse primer R3-4: 5'-TGCACCACCTGCACACAG-3' (SEQ ID NO:17). and
8) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17).

According to particular embodiments, the third pair of primers is chosen from:
1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
5) Forward primer F4-1: 5'-CCCTTATGTCCAGGGCTTCA-3' (SEQ ID NO:7), Reverse primer R4-5: 5'- GCATCGCAGCCCTTTGTA-3' (SEQ ID NO:28);
6) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
7) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
8) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

The invention is also directed to a kit for amplifying by PCR mycobacterial rrs gene sequences in a sample comprising a set of primers as defined above.

According to an embodiment, the kit is for the detection of mycobacterial rrs gene sequences amplified by PCR in a sample, and further comprises the following associated probes for the detection of the amplified product(s):
- Probe S1 having the sequence 5'-CGGTGGGTACTAGGTGTG-3' (SEQ ID NO:3) or modifications thereof, for detection of the sequences amplified by said primers F1 and R1;
- Probe S3 having the sequence: 5'-TCGGTTCCCTTGTGGC-3' (SEQ ID NO:6) or modifications thereof, for detection of the sequences amplified by said primers F3 and R3;
- Probe S4 having the sequence: 5'-ACATGCTACAATGGCCGG-3' (SEQ ID NO:9) or modifications thereof, for detection of the sequences amplified by said primers F4 and R4,
wherein said modifications are the addition and/or the deletion of 1 to 2 nucleotides, at the 3' and/or 5' terminus.

According to an embodiment, the probes are chosen:
a) For the probe S1, from the group consisting of :
   Probe S1-0: 5'-CGGTGGGTACTAGGTGTG-3' (SEQ ID NO:3),
   Probe S1-1: 5'-CGGTGGGTACTAGGTGT-3' (SEQ ID NO:22) and
   Probe S1-2: 5'-CGGTGGGTACTAGGTG-3' (SEQ ID NO:23);
   wherein when the Probe S1 is S1-1, the first pair of primers is chosen from:
      1) Forward primer F1-1 (SEQ ID NO:1) and reverse primer R1-1 (SEQ ID NO:2);
      2) Forward primer F1-1 (SEQ ID NO:1) and reverse primer R1-6 (SEQ ID NO:13);
      3) Forward primer F1-1 (SEQ ID NO:1) and reverse primer R1-5 (SEQ ID NO:12);
      4) Forward primer F1-2 (SEQ ID NO:10) and reverse primer R1-1 (SEQ ID NO:2); and
      5) Forward primer F1-3 (SEQ ID NO:11) and reverse primer R1-6 (SEQ ID NO:13);
   when the Probe S1 is S1-2, the first pair of primers is chosen from:
      1) Forward primer F1-1 (SEQ ID NO:1) and reverse primer R1-1 (SEQ ID NO:2);
      2) Forward primer F1-1 (SEQ ID NO:1) and reverse primer R1-6 (SEQ ID NO:13);
      3) Forward primer F1-1 (SEQ ID NO:1) and reverse primer R1-5 (SEQ ID NO:12);
      4) Forward primer F1-2 (SEQ ID NO:10) and reverse primer R1-1 (SEQ ID NO:2);
      5) Forward primer F1-2 (SEQ ID NO:10) and reverse primer R1-5 (SEQ ID NO:12); and
      6) Forward primer F1-3 (SEQ ID NO:11) and reverse primer R1-6 (SEQ ID NO:13);
b) For the probe S3, from the group consisting of :
   Probe S3-0: 5'-TCGGTTCCCTTGTGGC-3' (SEQ ID NO:6),
   Probe S3-1: 5'-ATCGGTTCCCTTGTGGC-3' (SEQ ID NO:24) and
   Probe S3-2: 5'-CGGTTCCCTTGTGGC-3' (SEQ ID NO:25);
   wherein when the Probe S3 is S3-1, the second pair of primers is chosen from:
      1) Forward primer F3-1 (SEQ ID NO:4) and reverse primer R3-1 (SEQ ID NO:5);
      2) Forward primer F3-3 (SEQ ID NO:14) and reverse primer R3-1 (SEQ ID NO:5);
      3) Forward primer F3-1 (SEQ ID NO:4) and reverse primer R3-2 (SEQ ID NO:15);
      4) Forward primer F3-3 (SEQ ID NO:14) and reverse primer R3-2 (SEQ ID NO:15); and
      5) Forward primer F3-3 (SEQ ID NO:14) and reverse primer R3-3 (SEQ ID NO:16);
   when the probe S3 is S3-2, the second pair of primers is chosen from:
      1) Forward primer F3-1 (SEQ ID NO:4) and reverse primer R3-1 (SEQ ID NO:5);
      2) Forward primer F3-3 (SEQ ID NO:14) and reverse primer R3-1 (SEQ ID NO:5);
      3) Forward primer F3-1 (SEQ ID NO:4) and reverse primer R3-2 (SEQ ID NO:15);
      4) Forward primer F3-1 (SEQ ID NO:4) and reverse primer R3-3 (SEQ ID NO:16);
      5) Forward primer F3-3 (SEQ ID NO:14) and reverse primer R3-4 (SEQ ID NO:17); and
      6) Forward primer F3-3 (SEQ ID NO:14) and reverse primer R3-3 (SEQ ID NO:16);
c) For the probe S4, from the group consisting of :
   Probe S4-0: 5'-ACATGCTACAATGGCCGG-3' (SEQ ID NO:9), and
   Probe S4-1: 5'-ACATGCTACAATGGCCGGT-3' (SEQ ID NO:26);
   wherein when the Probe S4 is S4-1, the third pair of primers is chosen from:
      1) Forward primer F4-1 (SEQ ID NO:7) and reverse primer R4-1 (SEQ ID NO:8);
      2) Forward primer F4-3 (SEQ ID NO:18) and reverse primer R4-1 (SEQ ID NO:8);
      3) Forward primer F4-5 (SEQ ID NO:19) and reverse primer R4-1 (SEQ ID NO:8);
      4) Forward primer F4-1 (SEQ ID NO:7) and reverse primer R4-3 (SEQ ID NO:20);
      5) Forward primer F4-3 (SEQ ID NO:18) and reverse primer R4-3 (SEQ ID NO:20);
      6) Forward primer F4-5 (SEQ ID NO:19) and reverse primer R4-3 (SEQ ID NO:20); and
      7) Forward primer F4-5 (SEQ ID NO:19) and reverse primer R4-6 (SEQ ID NO:21).

In an embodiment, the kit is for amplification by quantitative Polymerase Chain Reaction (qPCR) or by digital Polymerase Chain Reaction (dPCR).

The invention is also directed to a method for amplifying and detecting sequences of the *rrs* gene of any mycobacteria present in a sample, comprising the following steps, carried out simultaneously or sequentially:
a) Carrying out *in vitro* PCR on the nucleic acids extracted from said sample, with at least the set of three pairs of primers disclosed above;
b) Detecting the presence or absence of an amplified product.

The amplification step a) may for example be carried out by quantitative PCR or digital PCR.

According to an embodiment, the detection step b) is carried out with the following associated probes:
i. Probe S1 selected from the group consisting of:
   Probe S1-0: 5'-CGGTGGGTACTAGGTGTG-3' (SEQ ID NO:3),
   Probe S1-1: 5'-CGGTGGGTACTAGGTGT-3' (SEQ ID NO:22) and
   Probe S1-2: 5'-CGGTGGGTACTAGGTG-3' (SEQ ID NO:23);
   Wherein when the Probe S1 is S1-1, the first pair of primers is chosen from:
      1) Forward primer F1-1 (SEQ ID NO:1) and reverse primer R1-1 (SEQ ID NO:2);
      2) Forward primer F1-1 (SEQ ID NO:1) and reverse primer R1-6 (SEQ ID NO:13);
      3) Forward primer F1-1 (SEQ ID NO:1) and reverse primer R1-5 (SEQ ID NO:12);
      4) Forward primer F1-2 (SEQ ID NO:10) and reverse primer R1-1 (SEQ ID NO:2); and
      5) Forward primer F1-3 (SEQ ID NO:11) and reverse primer R1-6 (SEQ ID NO:13);
   when the Probe S1 is S1-2, the first pair of primers is chosen from:
      1) Forward primer F1-1 (SEQ ID NO:1) and reverse primer R1-1 (SEQ ID NO:2);
      2) Forward primer F1-1 (SEQ ID NO:1) and reverse primer R1-6 (SEQ ID NO:13);
      3) Forward primer F1-1 (SEQ ID NO:1) and reverse primer R1-5 (SEQ ID NO:12);
      4) Forward primer F1-2 (SEQ ID NO:10) and reverse primer R1-1 (SEQ ID NO:2);
      5) Forward primer F1-2 (SEQ ID NO:10) and reverse primer R1-5 (SEQ ID NO:12); and
      6) Forward primer F1-3 (SEQ ID NO:11) and reverse primer R1-6 (SEQ ID NO:13);
ii. Probe S3 selected from the group consisting of:
   Probe S3-0: 5'-TCGGTTCCCTTGTGGC-3' (SEQ ID NO:6),
   Probe S3-1: 5'-ATCGGTTCCCTTGTGGC-3' (SEQ ID NO:24) and
   Probe S3-2: 5'-CGGTTCCCTTGTGGC-3' (SEQ ID NO:25);
   wherein when the Probe S3 is S3-1, the second pair of primers is chosen from:
      1) Forward primer F3-1 (SEQ ID NO:4) and reverse primer R3-1 (SEQ ID NO:5);
      2) Forward primer F3-3 (SEQ ID NO:14) and reverse primer R3-1 (SEQ ID NO:5);
      3) Forward primer F3-1 (SEQ ID NO:4) and reverse primer R3-2 (SEQ ID NO:15);
      4) Forward primer F3-3 (SEQ ID NO:14) and reverse primer R3-2 (SEQ ID NO:15); and
      5) Forward primer F3-3 (SEQ ID NO:14) and reverse primer R3-3 (SEQ ID NO:16);
   when the Probe S3 is S3-2, the second pair of primers is chosen from:
      1) Forward primer F3-1 (SEQ ID NO:4) and reverse primer R3-1 (SEQ ID NO:5);
      2) Forward primer F3-3 (SEQ ID NO:14) and reverse primer R3-1 (SEQ ID NO:5);
      3) Forward primer F3-1 (SEQ ID NO:4) and reverse primer R3-2 (SEQ ID NO:15);
      4) Forward primer F3-1 (SEQ ID NO:4) and reverse primer R3-3 (SEQ ID NO:16);
      5) Forward primer F3-3 (SEQ ID NO:14) and reverse primer R3-4 (SEQ ID NO:17); and
      6) Forward primer F3-3 (SEQ ID NO:14) and reverse primer R3-3 (SEQ ID NO:16);
iii. Probe S4 selected from the group consisting of :
   Probe S4-0: 5'-ACATGCTACAATGGCCGG-3' (SEQ ID NO:9), and
   Probe S4-1: 5'-ACATGCTACAATGGCCGGT-3' (SEQ ID NO:26);
   wherein when the Probe S4 is S4-1, the third pair of primers is chosen from:
      1) Forward primer F4-1 (SEQ ID NO:7) and reverse primer R4-1 (SEQ ID NO:8);
      2) Forward primer F4-3 (SEQ ID NO:18) and reverse primer R4-1 (SEQ ID NO:8);
      3) Forward primer F4-5 (SEQ ID NO:19) and reverse primer R4-1 (SEQ ID NO:8);
      4) Forward primer F4-1 (SEQ ID NO:7) and reverse primer R4-3 (SEQ ID NO:20);
      5) Forward primer F4-3 (SEQ ID NO:18) and reverse primer R4-3 (SEQ ID NO:20);
      6) Forward primer F4-5 (SEQ ID NO:19) and reverse primer R4-3 (SEQ ID NO:20); and
      7) Forward primer F4-5 (SEQ ID NO:19) and reverse primer R4-6 (SEQ ID NO:21).

The method may comprise an initial step of extracting the nucleic acids from the sample, before carrying out step a).

The invention is also directed to a method for detecting the presence of mycobacterial contamination in a pharmaceutical product, without any step of culture, comprising the simultaneous following steps:
a) amplification *in vitro* by quantitative PCR (qPCR) or dPCR, carried out on the nucleic acids extracted from a sample of said product, with at least the three pairs of primers described above, wherein said primers hybridize to the *rrs* gene,
b) detection of the presence or absence of an amplified product by the following probes:
   i. Probe S1 selected from the group consisting of:
      Probe S1-0: 5'-CGGTGGGTACTAGGTGTG-3' (SEQ ID NO:3),
      Probe S1-1: 5'-CGGTGGGTACTAGGTGT-3' (SEQ ID NO:22) and
      Probe S1-2: 5'-CGGTGGGTACTAGGTG-3' (SEQ ID NO:23);
      wherein when the Probe S1 is S1-1, the first pair of primers is chosen from:
         1) Forward primer F1-1 (SEQ ID NO:1) and reverse primer R1-1 (SEQ ID NO:2);
         2) Forward primer F1-1 (SEQ ID NO:1) and reverse primer R1-6 (SEQ ID NO:13);
         3) Forward primer F1-1 (SEQ ID NO:1) and reverse primer R1-5 (SEQ ID NO:12);
         4) Forward primer F1-2 (SEQ ID NO:10) and reverse primer R1-1 (SEQ ID NO:2); and
         5) Forward primer F1-3 (SEQ ID NO:11) and reverse primer R1-6 (SEQ ID NO:13);
      when the Probe S1 is S1-2, the first pair of primers is chosen from:
         1) Forward primer F1-1 (SEQ ID NO:1) and reverse primer R1-1 (SEQ ID NO:2);
         2) Forward primer F1-1 (SEQ ID NO:1) and reverse primer R1-6 (SEQ ID NO:13);
         3) Forward primer F1-1 (SEQ ID NO:1) and reverse primer R1-5 (SEQ ID NO:12);
         4) Forward primer F1-2 (SEQ ID NO:10) and reverse primer R1-1 (SEQ ID NO:2);
         5) Forward primer F1-2 (SEQ ID NO:10) and reverse primer R1-5 (SEQ ID NO:12); and
         6) Forward primer F1-3 (SEQ ID NO:11) and reverse primer R1-6 (SEQ ID NO:13);
   ii. Probe S3 selected from the group consisting of:
      Probe S3-0: 5'-TCGGTTCCCTTGTGGC-3' (SEQ ID NO:6),
      Probe S3-1: 5'-ATCGGTTCCCTTGTGGC-3' (SEQ ID NO:24) and
      Probe S3-2: 5'-CGGTTCCCTTGTGGC-3' (SEQ ID NO:25);
      wherein when the Probe S3 is S3-1, the second pair of primers is chosen from:
         1) Forward primer F3-1 (SEQ ID NO:4) and reverse primer R3-1 (SEQ ID NO:5);
         2) Forward primer F3-3 (SEQ ID NO:14) and reverse primer R3-1 (SEQ ID NO:5);
         3) Forward primer F3-1 (SEQ ID NO:4) and reverse primer R3-2 (SEQ ID NO:15);
         4) Forward primer F3-3 (SEQ ID NO:14) and reverse primer R3-2 (SEQ ID NO:15); and
         5) Forward primer F3-3 (SEQ ID NO:14) and reverse primer R3-3 (SEQ ID NO:16);
      When the Probe S3 is S3-2, the second pair of primers is chosen from:
      1) Forward primer F3-1 (SEQ ID NO:4) and reverse primer R3-1 (SEQ ID NO:5);
      2) Forward primer F3-3 (SEQ ID NO:14) and reverse primer R3-1 (SEQ ID NO:5);
      3) Forward primer F3-1 (SEQ ID NO:4) and reverse primer R3-2 (SEQ ID NO:15);
      4) Forward primer F3-1 (SEQ ID NO:4) and reverse primer R3-3 (SEQ ID NO:16);
      5) Forward primer F3-3 (SEQ ID NO:14) and reverse primer R3-4 (SEQ ID NO:17); and
      6) Forward primer F3-3 (SEQ ID NO:14) and reverse primer R3-3 (SEQ ID NO:16);
   iii. Probe S4 selected from the group consisting of :
      Probe S4-0: 5'-ACATGCTACAATGGCCGG-3' (SEQ ID NO:9), and
      Probe S4-1: 5'-ACATGCTACAATGGCCGGT-3' (SEQ ID NO:26);
      wherein when the Probe S4 is S4-1, the third pair of primers is chosen from:
         1) Forward primer F4-1 (SEQ ID NO:7) and reverse primer R4-1 (SEQ ID NO:8);
         2) Forward primer F4-3 (SEQ ID NO:18) and reverse primer R4-1 (SEQ ID NO:8);
         3) Forward primer F4-5 (SEQ ID NO:19) and reverse primer R4-1 (SEQ ID NO:8);
         4) Forward primer F4-1 (SEQ ID NO:7) and reverse primer R4-3 (SEQ ID NO:20);
         5) Forward primer F4-3 (SEQ ID NO:18) and reverse primer R4-3 (SEQ ID NO:20);
         6) Forward primer F4-5 (SEQ ID NO:19) and reverse primer R4-3 (SEQ ID NO:20); and
         7) Forward primer F4-5 (SEQ ID NO:19) and reverse primer R4-6 (SEQ ID NO:21).

The amplification step a) may for example be carried out simultaneously with said three pairs of primers and said associated probes, in a single reaction mix.

According to still another embodiment, the application is also directed to a process for producing a biopharmaceutical product, comprising a step of testing for mycobacterial contamination by carrying out the method of the invention as disclosed above for detecting sequences of the *rrs* gene of any mycobacteria. The step of testing for mycobacterial contamination may be performed at any stage(s) during the biopharmaceutical product manufacture, for instance on the raw materials, such as on the cell substrate and/or the viral seed and/or the culture medium and/or the cell culture, and/or on the biopharmaceutical product harvest and/or on the formulated biopharmaceutical product and/or on the filled biopharmaceutical product. When a raw material or a biopharmaceutical product (i.e. a sample from a raw material or a biopharmaceutical product) is detected as being contaminated by mycobacteria according to the method of the invention as disclosed above, the raw material or the biopharmaceutical product is discarded from the production process. A product, such as a raw material or a biopharmaceutical product determined as devoid of mycobacterial contamination (i.e. from which a sample is determined as devoid of mycobacterial contamination) by testing according to the method of the invention as disclosed above, is maintained in the production process. Such a process for producing a biopharmaceutical product may further comprise a step of releasing the biopharmaceutical product having passed the test based on the absence of detection of any amplified product by using the three pairs of primers and associated probes of the invention.

The process for producing a biopharmaceutical product may be a process for producing a biopharmaceutical product devoid of mycobacterial contamination, comprising the step of providing a biopharmaceutical product and the step of carrying out, on the biopharmaceutical product, the method of the invention as disclosed above for detecting sequences of the *rrs* gene of any mycobacteria.

The biopharmaceutical product may be a biological or pharmaceutical product; a suitable product is a vaccine.

Additional uses and methods based on the primers and probes are also part of the invention.

### Definitions:

Mycobacteria: A mycobacterium according to the invention is a bacterium of the mycobacteriaceae family according to the classification of February 2018 (Gupta *et al,* 2018) completed by newly identified species. Currently, at the date of the invention, this family comprises 217 species, subspecies or variants, from the previously defined genera *Mycobacteroides*, *Mycolicibacterium*, *Mycolicibacter*, *Mycolicibacillus* and *Mycobacterium*; this represents 217 different sequences. These different species and subspecies or variants are detailed in table 4, corresponding to mycobacteria for which a sequence has been validated by a publication; these bacteria are considered as mycobacteria in the present invention, irrespective of their classification within the mycobacteria family.

Mycobacteria-specific amplification of a target sequence refers to amplification of a target sequence in all of the relevant species of Mycobacteria (i.e. in the 217 species, subspecies or variants).

A mycobacterial species in the following refers to any of these 217 species, subspecies or variants.

### Legend of the Figures:

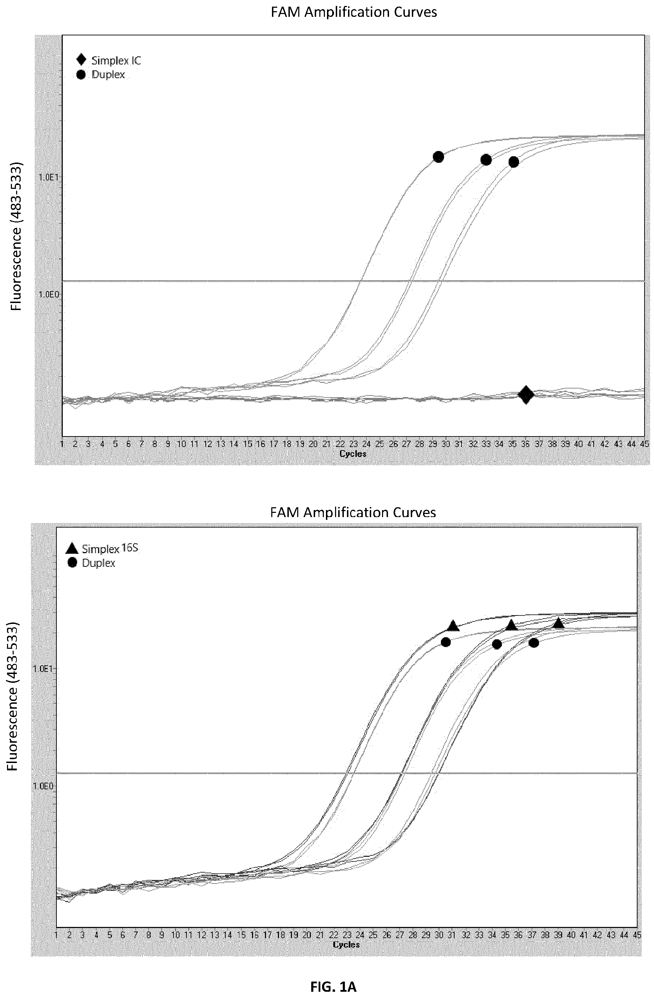

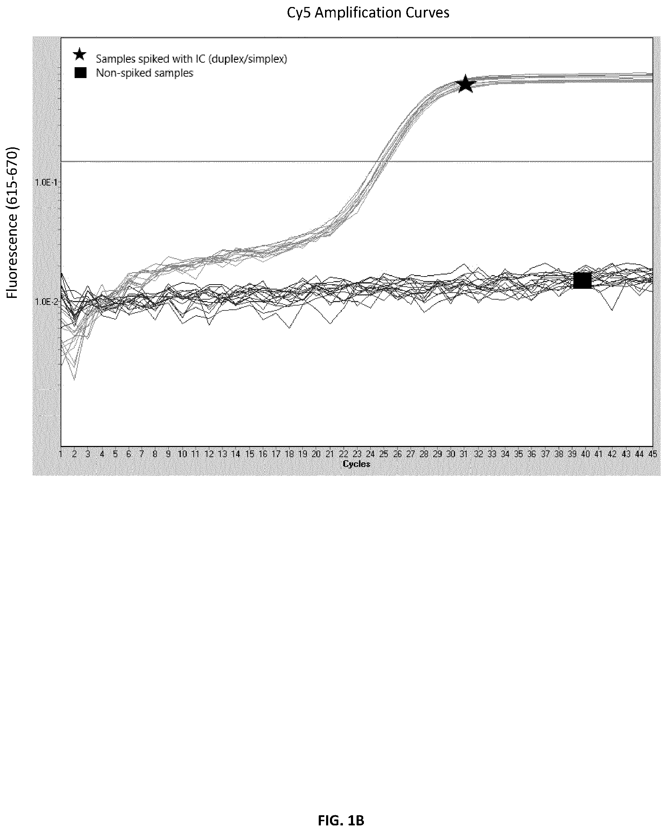

**FIG.1A** **and** **1B****:** Comparison of the amplification curves in simplex and duplex amplification.
   These figures represent the level of fluorescence at the wavelength corresponding to the fluorophore FAM (FIG.1A), i.e. the fluorophore of the probe S1-0, and at the wavelength corresponding to the fluorophore Cy5 (FIG. 1B), i. e. the fluorophore of the probe for the internal control (IC), as a function of the number of PCR cycles, for amplification/detection carried out in simplex (using only F1-1/R1-1/S1-0 or only primers/probe of the internal control IC) or duplex (using both F1-1/R1-1/S1-0 and primers/probe of the internal control IC), on samples comprising extracted DNA from *M*. *tuberculosis* var BCG at different concentrations, with the internal control (IC) at a fixed concentration.
   The x-axis represents the number of cycles of PCR, and the Y-axis the level of fluorescence in logarithmic scale.
   FIG. 1A : upper panel: amplification in simplex (diamond ◆) with the primers/probe of the internal control (simplex IC) and in duplex (circle ●) with the primers/probe of the internal control and F1-1/R1-1/S1-0. The sample is spiked with the internal control, and DNA of *M*. *tuberculosis* var BCG, at different concentrations.
   Lower panel: amplification in simplex (diamond ◆) with the primers/probe F1-1/R1-1/S1-0 (simplex 16S) and in duplex (circle ●) with the primers/probe of the internal control and F1-1/R1-1/S1-0. The sample is spiked with the internal control, and DNA of M. tuberculosis var BCG, at different concentrations. FIG. 1B: amplification in simplex (with the primers/probe of the internal control) and in duplex (with the primers/probe of the internal control and F1-1/R1-1/S1-0) of a sample spiked with the internal control and DNA of M. tuberculosis var BCG (star *) and non-spiked (square ■).

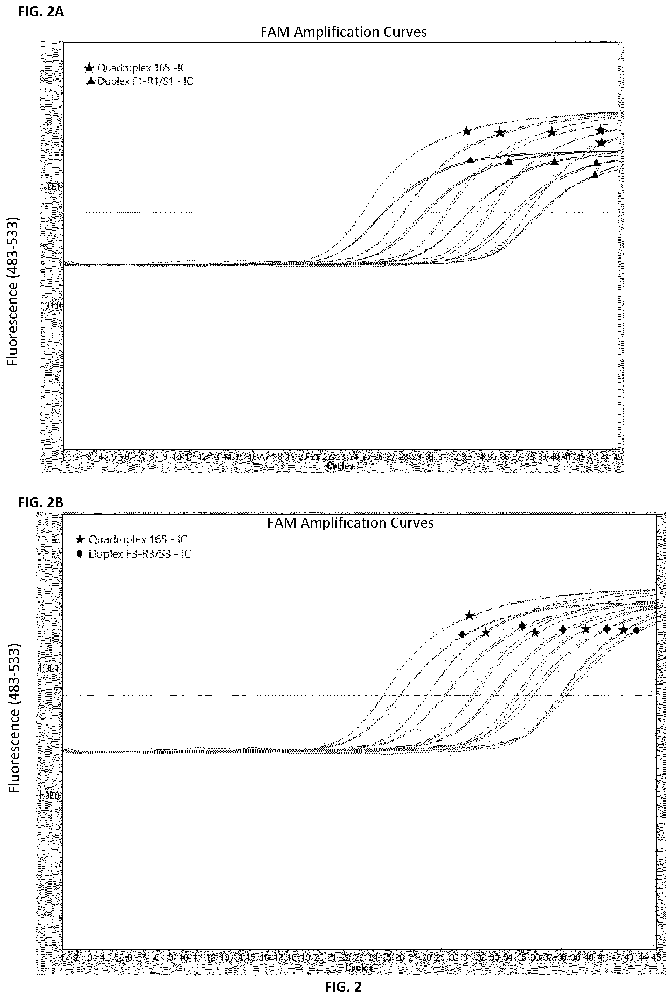

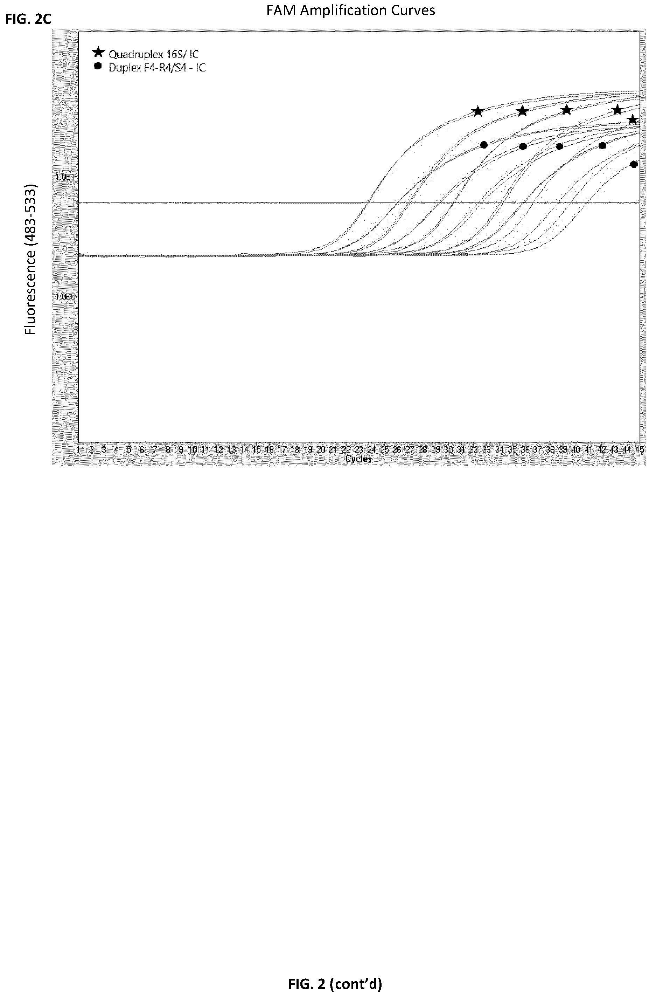

**FIG. 2****:** Comparison of the amplification curves in duplex and quadruplex amplification.
   This figure represents the level of fluorescence at the wavelength corresponding to the fluorophore FAM, i.e. the fluorophore of the probes S1-0, S3-0 and S4-0, as a function of the number of PCR cycles, for amplification/detection carried out in duplex or in quadruplex, on samples comprising DNA of *M*. *tuberculosis* var BCG at different concentrations, with the internal control.
   The x-axis represents the number of cycles of PCR, and the Y-axis the level of fluorescence in logarithmic scale.
      **FIG.2A****:** comparison of the amplification curves in quadruplex, and in duplex with only the 1^{st} system (F1-R1/S1 corresponding to F1-1/R1-1/S1-0) and the internal control and associated primers and probes (IC).
      **FIG.2B****:** comparison of the amplification curves in quadruplex, and in duplex with only the 3^{rd} system (F3-R3/S3 corresponding to F3-1/R3-1/S3-0) and the internal control and associated primers and probes (IC).
      **FIG.2C****:** comparison of the amplification curves in quadruplex, and in duplex with only the 4^{th} system (F4-R4/S4 corresponding to F4-1/R4-1/S4-0) and the internal control and associated primers and probes (IC).
**FIG.3A****-F:** Amplification curves of the *rrs* gene of different mycobacteria (detection with FAM).
   The curves obtained by the extracted DNA "pure", as well as the different dilutions 10⁻¹, 10⁻² and 10⁻³ are indicated.

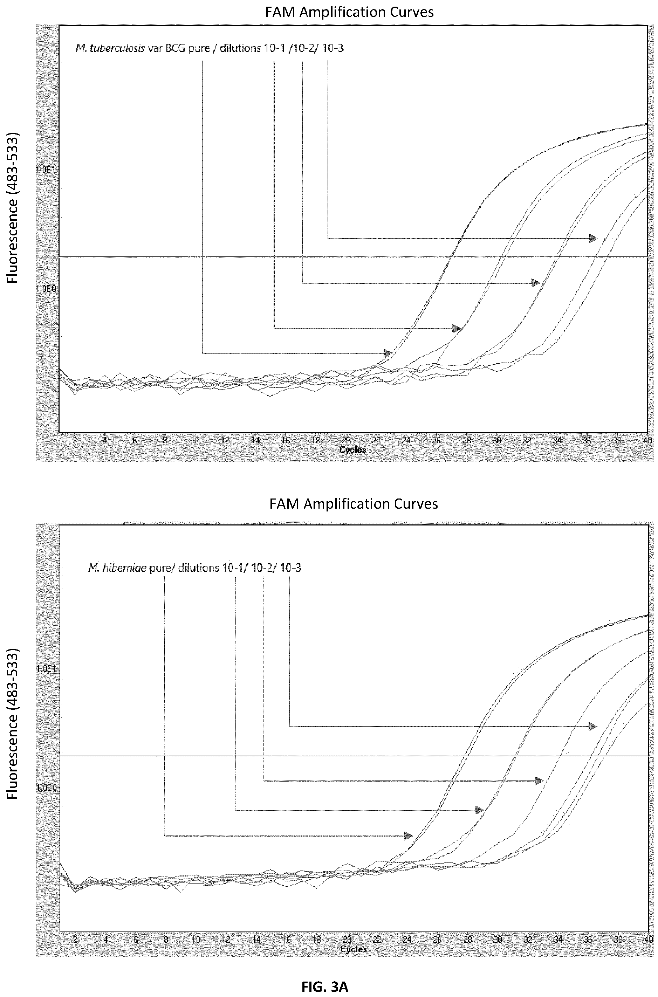

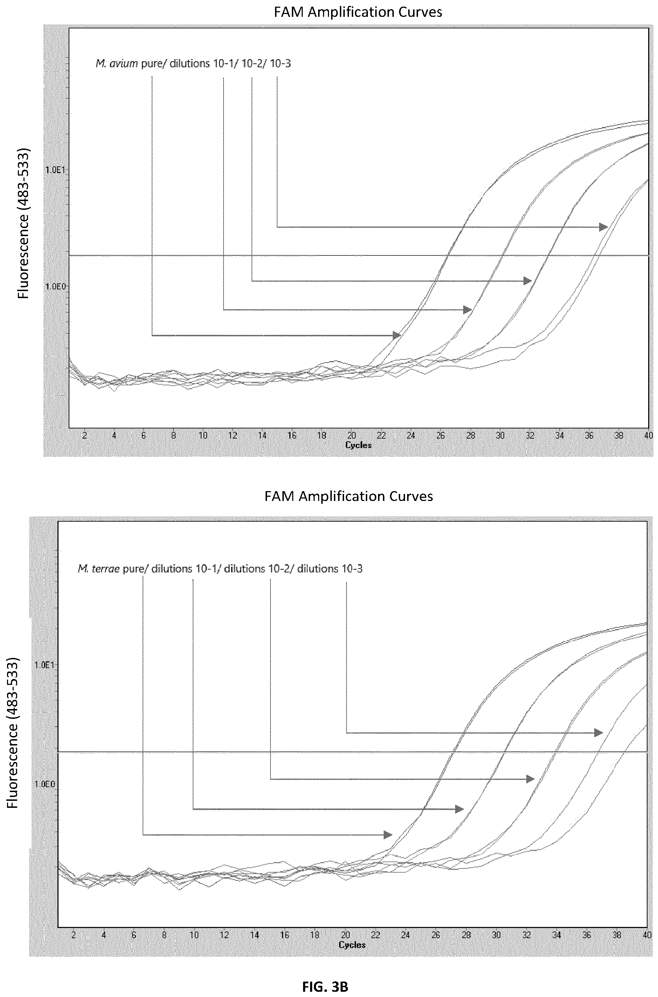

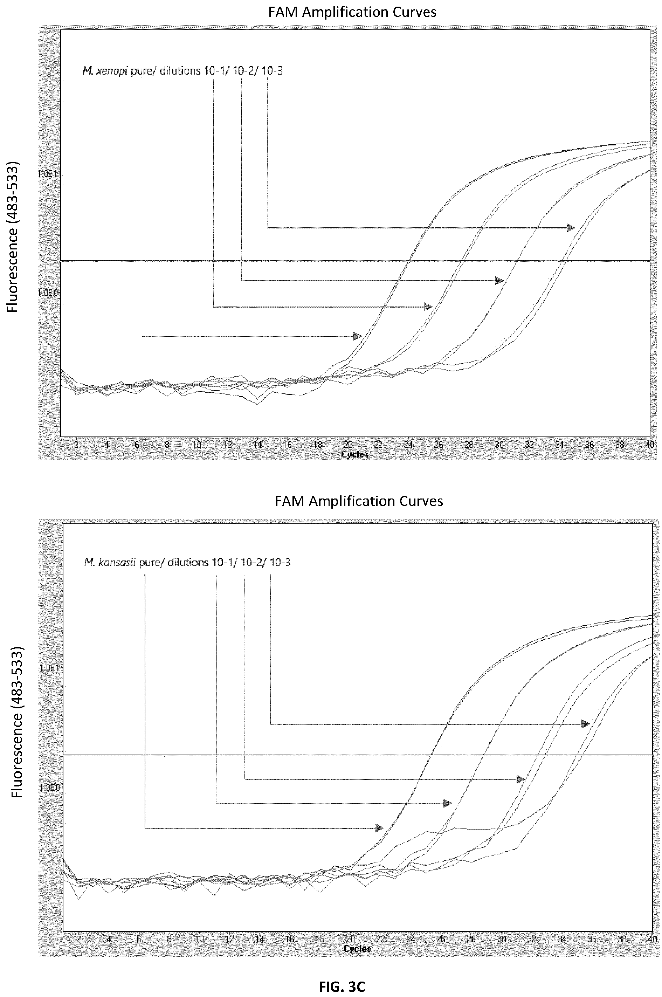

   **FIG.3A****-C:** 6 slow-growing mycobacteria: *M. tuberculosis* var BCG and *M. hiberniae* (FIG. 3A), *M. avium* and *M*. *terrae* (FIG. 3B), *M. xenopi* and *M. kansaii* (FIG. 3C).

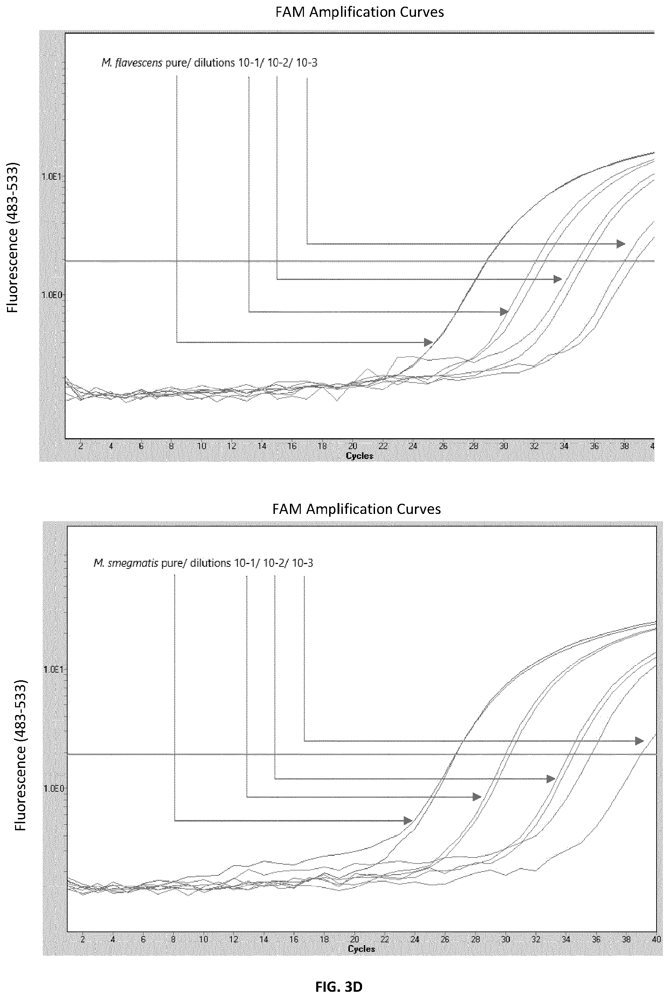

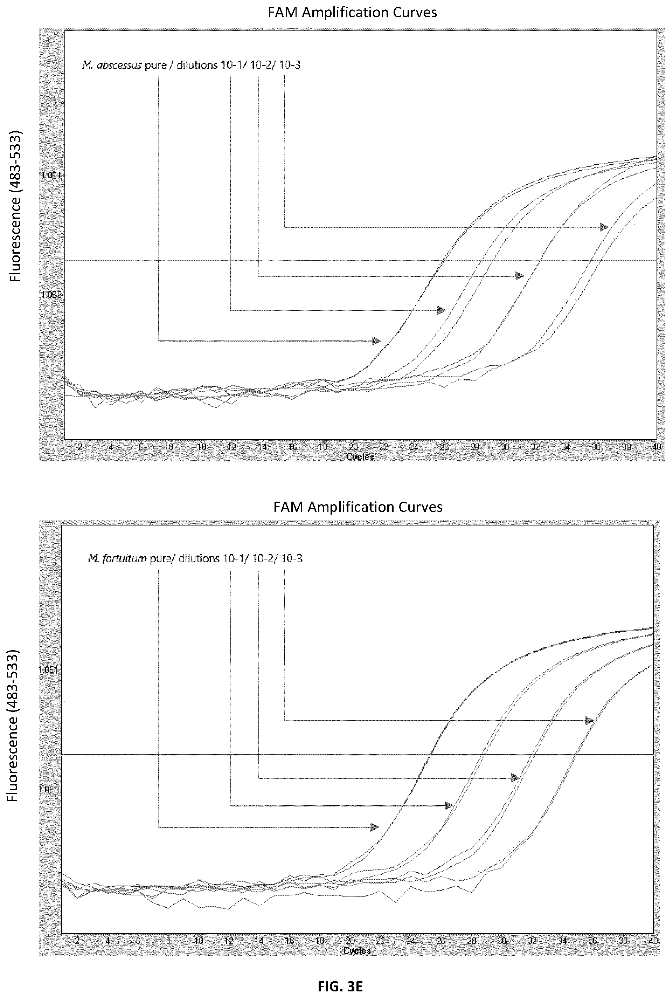

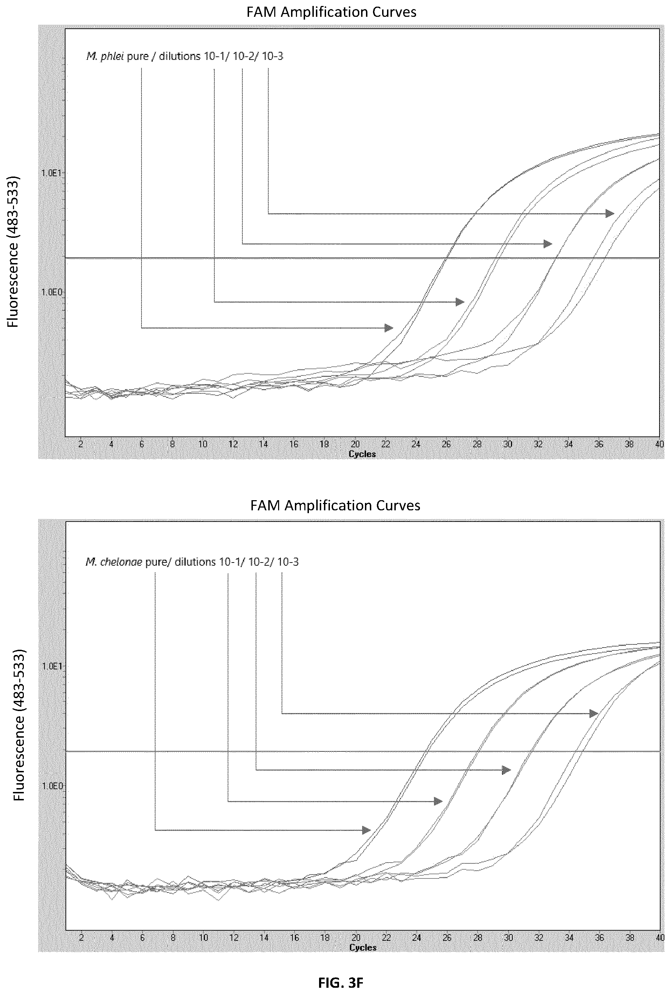

   **FIG.3D****-F:** 6 fast -growing mycobacteria: *M*. *flavescens* and *M*. *smegmatis* (FIG. 3D), *M*. *abscessus* and *M*. *fortuitum* (FIG. 3E), *M. phlei* and *M*. *chelonae* (FIG. 3F).

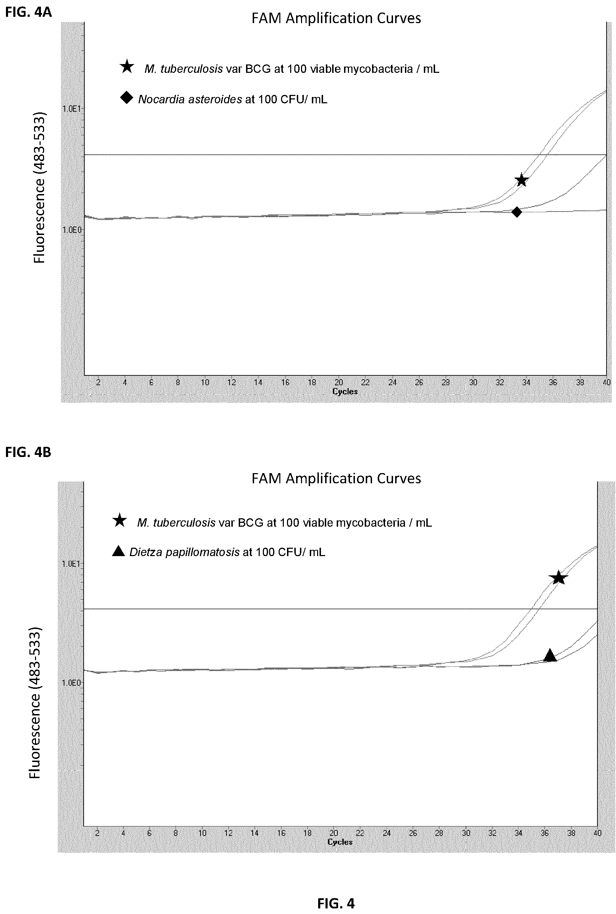

**FIG.4****:** Amplification curves of the *rrs* gene in different samples comprising *M*. *tuberculosis* var BCG (positive control) at 100 viable mycobacteria per mL and different actinomyces bacteria also at 100 Colony Forming Units (CFU) per mL (detection with FAM).
   The x-axis represents the number of cycles of PCR, and the Y-axis the level of fluorescence in logarithmic scale.
   **FIG.4A****:** comparison of the amplification curves obtained with *M. tuberculosis* var BCG and N. *asteroides* at 100 CFU/mL. No amplification of *N. asteroides* by the mycobacteria qPCR (Cycle threshold >40).
   **FIG.4B****:** comparison of the amplification curves obtained with *M. tuberculosis* var BCG and *D. papillomatosis* at 100 CFU/mL. No amplification of *D. papillomatosis* by the mycobacteria qPCR (Cycle threshold >40).

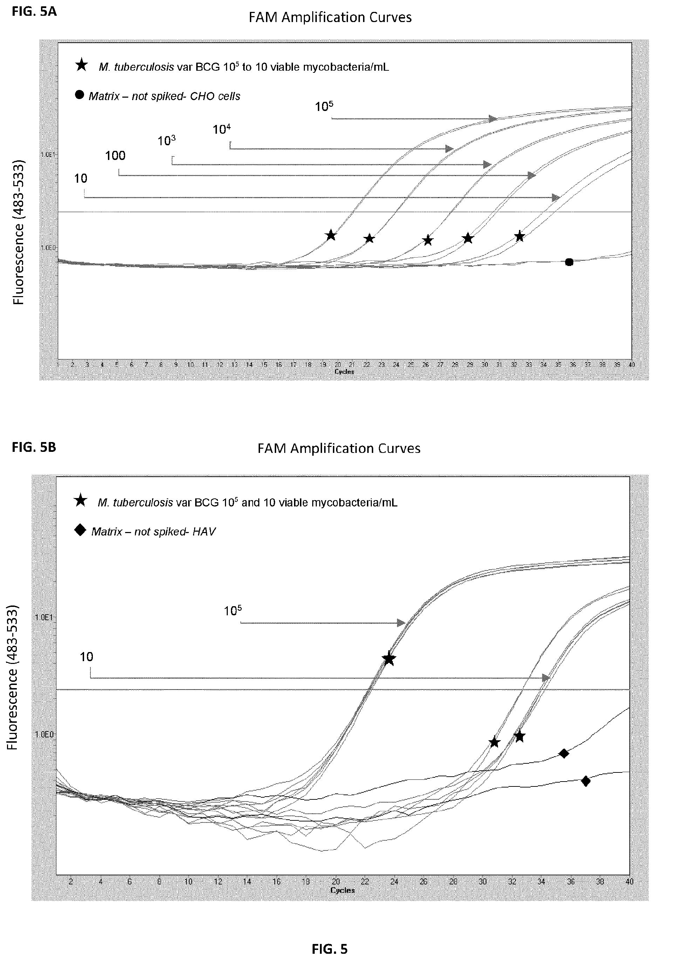

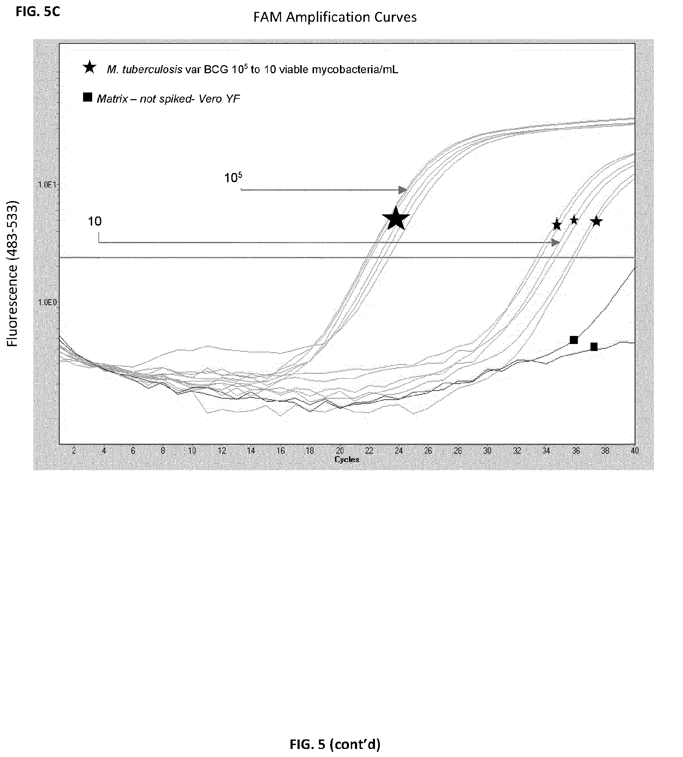

**FIG.5****:** Amplification curves of the *rrs* gene in different matrices spiked or not spiked with *M*. *tuberculosis* var BCG at different concentrations (from 10⁵ to 10 viable mycobacteria per mL) (detection with FAM). The x-axis represents the number of cycles of PCR, and the Y-axis the level of fluorescence in logarithmic scale.
   **FIG.5A****:** comparison of the amplification curves obtained with the matrix corresponding to supernatant of CHO cell used for CMV production, spiked with *M*. *tuberculosis* var BCG at different concentrations (10, 100, 10³, 10⁴ and 10⁵ viable mycobacteria per mL, two replicates per concentration) (star *) or not spiked (circle ●).
   **FIG.5B****:** comparison of the amplification curves obtained with the matrix corresponding to crude harvest of drug substance from HAV produced on MRC5 cells, spiked with *M. tuberculosis* var BCG at two different concentrations (10 and 10⁵ viable mycobacteria per mL, six replicates per concentration) (star *) or not spiked (diamond ◆).
   **FIG.5C****:** comparison of the amplification curves obtained with the matrix corresponding to crude harvest of drug substance from Yellow Fever virus vaccine produced on Vero cells, spiked with *M. tuberculosis* var BCG at two different concentrations (10 and 10⁵ viable mycobacteria per mL, six replicates per concentration) (star *) or not spiked (square ■).

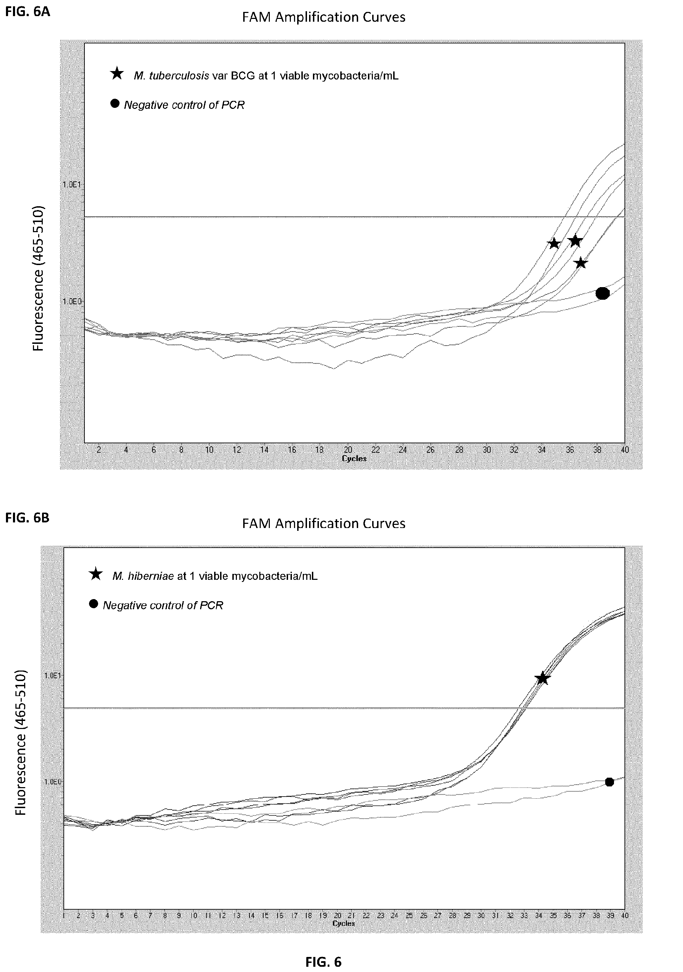

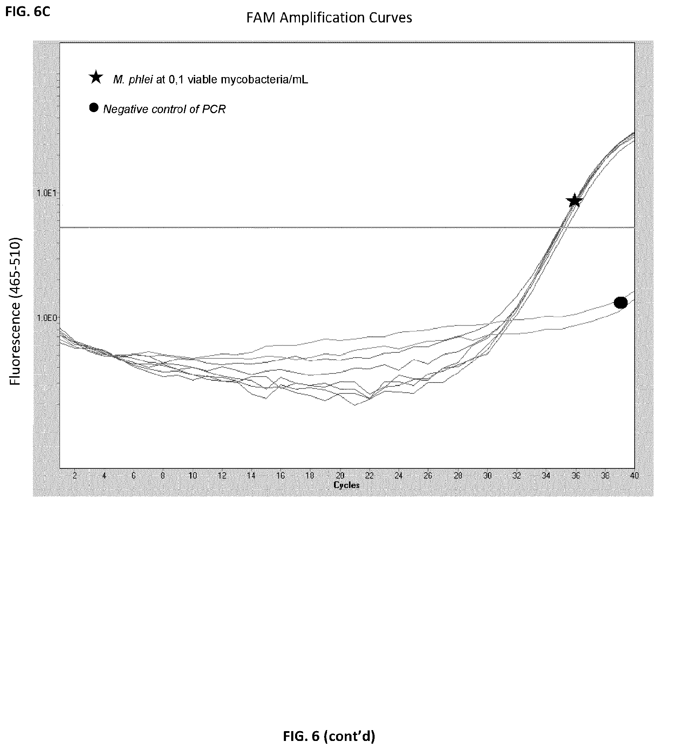

**FIG.6****:** Amplification curves of the *rrs* gene of different mycobacteria, present at 1 or 0.1 viable mycobacteria per mL of matrix (Vero cells) (detection with FAM).
   The x-axis represents the number of cycles of PCR, and the Y-axis the level of fluorescence in logarithmic scale.
   **FIG.6A****:** comparison of the amplification curves obtained with the matrix corresponding to Vero cells, spiked with *M*. *avium* at 1 viable mycobacteria per mL (star *) or not spiked (circle ●).
   **FIG.6B****:** comparison of the amplification curves obtained with the matrix corresponding to Vero cells, spiked with *M*. *hiberniae* at 1 viable mycobacteria per mL (star *) or not spiked (circle ●).
   **FIG.6C****:** comparison of the amplification curves obtained with the matrix corresponding to Vero cells, spiked with *M. phlei* at 0.1 viable mycobacteria per mL (star *) or not spiked (circle ●).

### Detailed Description of the invention:

The inventors have unexpectedly found a combination of primers, adapted to PCR and giving rise to a mycobacteria specific amplification of a target gene, namely *rrs* gene. The method based on this combination of primers thus allows the detection of mycobacterial species, namely all the known strains and species of mycobacteria, in a sample, in an exhaustive manner, i.e. any species, subspecies or variants of mycobacteria potentially present is detected. The minimal combination of primers moreover comprises only 6 primers, such that simultaneous amplification with all the primers is made possible.

According to a first aspect, the present invention is directed to a set of primers for amplifying by Polymerase Chain Reaction (PCR) a sequence of the *rrs* gene of mycobacteria, more specifically to a set of primers, amplifying the *rrs* gene of any potential bacteria classified as mycobacteria. The set of primers thus covers the entire mycobacteria family according to the classification of February 2018 (Gupta *et al,* 2018) as well as new bacteria classified as mycobacteria; and also all the mycobacteria of the *Mycobacterium* genus according to the latest classification (Meehan CJ *et al,* 2021).

The set of primers of the invention covers the entire mycobacteria family, i.e. the 217 species, subspecies or variants of table 4, for which a sequence has been validated by a publication.

The set of primers thus allows amplification by PCR of the *rrs* gene of any mycobacteria present in a sample. The set of primers according to the invention consists in or comprises at least the three following pairs of primers:
1^{st} Pair:
   Forward primer F1 having the sequence: 5'-CCTGGTAGTCCACGCCGTAA-3' (SEQ ID NO:1) or modifications thereof,
   Reverse primer R1 having the sequence: 5'-CGGATCCCAAGGAAGGAAAC-3' (SEQ ID NO:2) or modifications thereof;
2^{nd} Pair:
   Forward primer F3 having the sequence: 5'-CACAGGACGCCGGTAGAGAT-3' (SEQ ID NO:4) or modifications thereof,
   Reverse primer R3 having the sequence: 5'-CATGCACCACCTGCACACA-3' (SEQ ID NO:5) or modifications thereof;
3^{rd} Pair:
   Forward primer F4 having the sequence: 5'-CCCTTATGTCCAGGGCTTCA-3' (SEQ ID NO:7) or modifications thereof,
   Reverse primer R4 having the sequence: 5'-GGCATCGCAGCCCTTTG-3' (SEQ ID NO:8) or modifications thereof.

The set of primers of the invention may only comprise these 3 pairs of primers, i.e. 6 primers in total, or may comprise additional pairs of primers, or alternative forward or reverse primers. The minimal number of primers is thus six, for amplifying the *rrs* gene of any mycobacterial origin, i.e. in an exhaustive manner, irrespective of whether the mycobacterial strain is pathogenic or not, infect humans or not, is from the tuberculosis complex or not. This very small number of primers is particularly advantageous, as it necessitates less reagents and allows simultaneous amplification with all 6 primers. The inventors have thus defined a minimal combination of primers, allowing a Mycobacteria-specific amplification of the *rrs* gene.

The inventors have indeed designed three pairs of primers, corresponding to SEQ ID NO:1 and SEQ ID NO:2 for the 1^{st} pair of primers, SEQ ID NO:4 and SEQ ID NO:5 for the 2^{nd} pair of primers, and SEQ ID NO:7 and SEQ ID NO:8 for the 3^{rd} pair of primers, which allow to amplify the *rrs* gene of any mycobacterial species. According to a particular embodiment, the set of primers thus consists of or comprises the following three pairs of primers:
1^{st} Pair:
   Forward primer F1-1 having the sequence: 5'-CCTGGTAGTCCACGCCGTAA-3' (SEQ ID NO:1),
   Reverse primer R1-1 having the sequence: 5'-CGGATCCCAAGGAAGGAAAC-3' (SEQ ID NO:2); 2^{nd} Pair:
   Forward primer F3-1 having the sequence: 5'-CACAGGACGCCGGTAGAGAT-3' (SEQ ID NO:4),
   Reverse primer R3-1 having the sequence: 5'-CATGCACCACCTGCACACA-3' (SEQ ID NO:5);
3^{rd} Pair:
   Forward primer F4-1 having the sequence: 5'-CCCTTATGTCCAGGGCTTCA-3' (SEQ ID NO:7),
   Reverse primer R4-1 having the sequence: 5'-GGCATCGCAGCCCTTTG-3' (SEQ ID NO:8).

The inventors have moreover demonstrated that some modifications of the sequence of these primers do not change the properties of the pairs of primers, i.e. they still amplify the *rrs* gene of the same mycobacteria, such that, collectively, the three pairs of primers allow to amplify the *rrs* gene of any mycobacterial species.

The present invention thus also concerns modifications of SEQ ID NO:1 and SEQ ID NO:2 for the 1^{st} pair of primers, of SEQ ID NO:4 and SEQ ID NO:5 for the 2^{nd} pair of primers, and of SEQ ID NO:7 and SEQ ID NO:8 for the 3^{rd} pair of primers, to the extent that the resulting 3 pairs of primers still allow amplification of the *rrs* gene of any mycobacterial species. Example 7 in the following experimental section details how to design suitable modifications of the sequences SEQ ID NO:1, SEQ ID NO:2, SEQ ID NO:4, SEQ ID NO:5, SEQ ID NO:7 and SEQ ID NO:8, preserving the exhaustivity.

The modifications are *inter alia* the addition and/or the deletion of 1 to 4 nucleotides, at the 3' and/or 5' terminus, and/or the substitution of 1 or 2 or 3 nucleotides. According to a particular embodiment, the deletion is of 1 to 4 nucleotides, at the 3' terminus, at the 5' terminus, or at the 3' and 5' terminus. The substitution is in particular a substitution of 1 or 2 nucleotides, and particularly not at the 3' and 5' terminus, and especially not at the 3' terminus, and more generally not in the last 5 nucleotides at the 3' terminus.

According to an embodiment, the modifications are the addition and/or the deletion of 1 to 4 nucleotides, at the 3' and/or 5' terminus of the primer sequence.

Given the modifications detailed above, according to different embodiments of the present invention, the first pair of primers may have any of the following sequences:
a) Forward primer F1-1: 5'-CCTGGTAGTCCACGCCGTAA-3' (SEQ ID NO:1)
   Reverse primer R1-1: 5'-CGGATCCCAAGGAAGGAAAC-3' (SEQ ID NO:2); corresponding to the 1^{st} pair of primers without modifications, i.e. the original 1^{st} pair of primers,
b) Forward primer F1-1: 5'-CCTGGTAGTCCACGCCGTAA-3' (SEQ ID NO:1)
   Reverse primer R1-6: 5'-GGATCCCAAGGAAGGAAACC-3' (SEQ ID NO:13);
c) Forward primer F1-1: 5'-CCTGGTAGTCCACGCCGTAA-3' (SEQ ID NO:1);
   Reverse primer R1-5: 5'-ACGGATCCCAAGGAAGGAAAC-3' (SEQ ID NO:12);
d) Forward primer F1-2: 5'-CCTGGTAGTCCACGCCGTAAA-3' (SEQ ID NO:10)
   Reverse primer R1-1: 5'-CGGATCCCAAGGAAGGAAAC-3' (SEQ ID NO:2);
e) Forward primer F1-2: 5'-CCTGGTAGTCCACGCCGTAAA-3' (SEQ ID NO:10)
   Reverse primer R1-3: 5'- CACGGATCCCAAGGAAGGAAAC-3' (SEQ ID NO:27);
f) Forward primer F1-2: 5'-CCTGGTAGTCCACGCCGTAAA-3' (SEQ ID NO:10)
   Reverse primer R1-5: 5'-ACGGATCCCAAGGAAGGAAAC-3' (SEQ ID NO:12);
g) Forward primer F1-2: 5'-CCTGGTAGTCCACGCCGTAAA-3' (SEQ ID NO:10)
   Reverse primer R1-6: 5'-GGATCCCAAGGAAGGAAACC-3' (SEQ ID NO:13); or
h) Forward primer F1-3: 5'-CTGGTAGTCCACGCCGTAAA-3' (SEQ ID NO:11)
   Reverse primer R1-6: 5'-GGATCCCAAGGAAGGAAACC-3' (SEQ ID NO:13).

As demonstrated in the experimental section of the present application, all these different pairs of primers allow the amplification of the same mycobacterial species as the original 1^{st} pair of primers. Moreover, the combination of any one of these pairs of primers, with the pairs of primers corresponding to SEQ ID NO:4, SEQ ID NO:5, SEQ ID NO:7 and SEQ ID NO:8, or with variants thereof, allows the amplification of a sequence of the *rrs* gene of any mycobacterial species.

Irrespective of the above, the choice of the first pair of primers, especially of the sequences of the forward and reverse primers of this set is made independently of the choice of the sequences for the 2^{nd} and 3^{rd} pairs of primers.

Given the modifications detailed above, according to other independent embodiments of the present invention, the second pair of primers may have any of the following sequences:
a) Forward primer F3-1: 5'-CACAGGACGCCGGTAGAGAT-3' (SEQ ID NO:4)
   Reverse primer R3-1: 5'-CATGCACCACCTGCACACA-3' (SEQ ID NO:5); corresponding to the 2^{nd} pair of primers without modifications, *i.e.* the original 2^{nd} pair of primers,
b) Forward primer F3-3: 5'-CACAGGACGCCGGTAGAGATA-3' (SEQ ID NO:14)
   Reverse primer R3-1: 5'-CATGCACCACCTGCACACA-3' (SEQ ID NO:5);
c) Forward primer F3-1: 5'-CACAGGACGCCGGTAGAGAT-3' (SEQ ID NO:4)
   Reverse primer R3-2: 5'-CATGCACCACCTGCACACAG-3' (SEQ ID NO:15);
d) Forward primer F3-1: 5'-CACAGGACGCCGGTAGAGAT-3' (SEQ ID NO:4)
   Reverse primer R3-3: 5'-ATGCACCACCTGCACACAG-3' (SEQ ID NO:16);
e) Forward primer F3-3: 5'-CACAGGACGCCGGTAGAGATA-3' (SEQ ID NO:14)
   Reverse primer R3-2: 5'-CATGCACCACCTGCACACAG-3' (SEQ ID NO:15);
f) Forward primer F3-3: 5'-CACAGGACGCCGGTAGAGATA-3' (SEQ ID NO:14)
   Reverse primer R3-3: 5'-ATGCACCACCTGCACACAG-3' (SEQ ID NO:16);
g) Forward primer F3-1: 5'-CACAGGACGCCGGTAGAGAT-3' (SEQ ID NO:4)
   Reverse primer R3-4: 5'-TGCACCACCTGCACACAG-3' (SEQ ID NO:17); or
h) Forward primer F3-3: 5'-CACAGGACGCCGGTAGAGATA-3' (SEQ ID NO:14)
   Reverse primer R3-4: 5'-TGCACCACCTGCACACAG-3' (SEQ ID NO:17).

As demonstrated in the experimental section of the present application, all these different pairs of primers allow the amplification of the same mycobacterial species as the original 2^{nd} pair of primers. Moreover, the combination of any one of these pairs of primers, with the pairs of primers corresponding to SEQ ID NO:1, SEQ ID NO:2, SEQ ID NO:7 and SEQ ID NO:8, or with variants thereof, allows the amplification of a sequence of the *rrs* gene of any mycobacterial species.

Irrespective of the above, the choice of the second pair of primers, especially of the sequences of the forward and reverse primers of this set is made independently of the choice of the sequences for the 1^{st} and 3^{rd} pairs of primers.

According to still other independent embodiments of the present invention, the third pair of primers may have any of the following sequences:
a) Forward primer F4-1: 5'-CCCTTATGTCCAGGGCTTCA-3' (SEQ ID NO:7),
   Reverse primer R4-1: 5'-GGCATCGCAGCCCTTTG-3' (SEQ ID NO:8); corresponding to the 3^{rd} pair of primers without modifications, *i.e.* the original 3^{rd} pair of primers,
b) Forward primer F4-3: 5'-CCCCTTATGTCCAGGGCTTC-3' (SEQ ID NO:18),
   Reverse primer R4-1: 5'-GGCATCGCAGCCCTTTG-3' (SEQ ID NO:8);
c) Forward primer F4-5: 5'-GCCCCTTATGTCCAGGGC-3' (SEQ ID NO:19),
   Reverse primer R4-1: 5'-GGCATCGCAGCCCTTTG-3' (SEQ ID NO:8);
d) Forward primer F4-1: 5'-CCCTTATGTCCAGGGCTTCA-3' (SEQ ID NO:7),
   Reverse primer R4-3: 5'-CGGCATCGCAGCCCTT-3' (SEQ ID NO:20);
e) Forward primer F4-1: 5'-CCCTTATGTCCAGGGCTTCA-3' (SEQ ID NO:7),
   Reverse primer R4-5: 5'- GCATCGCAGCCCTTTGTA-3' (SEQ ID NO:28);
f) Forward primer F4-3: 5'-CCCCTTATGTCCAGGGCTTC-3' (SEQ ID NO:18),
   Reverse primer R4-3: 5'-CGGCATCGCAGCCCTT-3' (SEQ ID NO:20);
g) Forward primer F4-5: 5'-GCCCCTTATGTCCAGGGC-3' (SEQ ID NO:19),
   Reverse primer R4-3: 5'-CGGCATCGCAGCCCTT-3' (SEQ ID NO:20); or
h) Forward primer F4-5: 5'-GCCCCTTATGTCCAGGGC-3' (SEQ ID NO:19),
   Reverse primer R4-6: 5'-GGCATCGCAGCCCTTTGTA-3' (SEQ ID NO:21).

As demonstrated in the examples, all these different pairs of primers allow the amplification of the same mycobacterial species as the original 3^{rd} pair of primers. Moreover, the combination of any one of these pairs of primers, with the pairs of primers corresponding to SEQ ID NO:1, SEQ ID NO:2, SEQ ID NO:4 and SEQ ID NO:5, or with variants thereof, allows the amplification of a sequence of the *rrs* gene of any mycobacterial species.

Irrespective of the above, the choice of the third pair of primers, especially of the sequences of the forward and reverse primers of this set is made independently of the choice of the sequences for the 1^{st} and 2^{nd} pairs of primers.

Irrespective of the above-mentioned modifications of the primer sequences, which are modifications in the sequence hybridizing with the target sequence to be amplified, the primers of the invention may also comprise additional sequence or moiety(ies) not directly involved in the interaction with the target sequence. The primers as disclosed may *inter alia* comprise loops as required in the design of primers adapted to LAMP (loop mediated isothermal amplification) technique. The sequences of the primers as disclosed in the present description thus relate to the sequences hybridizing with the target mycobacterial sequence.

The 3 pairs of primers disclosed above allow to amplify a sequence from the *rrs* gene of any mycobacterial species, i.e. irrespective of the mycobacterial species, subspecies or variants. The 3 pairs of primers do not target the same portion of the *rrs* gene; they target sequences of the *rrs* gene which are conserved between the different species of mycobacteria, or between a subgroup of mycobacteria. The *rrs* gene of any mycobacterial species will thus be targeted by at least one pair of primers of the set of primers of the invention, giving rise to an amplification product. The *rrs* gene of some mycobacterial species may however be targeted by 2 pairs of primers of the set of the invention. The *rrs* gene of any mycobacterial species will thus be hybridized by at least one pair of primers, but not necessarily only one pair of primers, of the set of primers according to the invention.

The absence of amplification when using the set of 3 pairs of primers of the invention, in suitable conditions, in a sample comprising DNA extracted from bacteria, means that the sample does not comprise sequences corresponding the *rrs* gene of a mycobacterial species, and thus that the sample does not comprise any species of mycobacteria.

In a further aspect, the present invention is directed to a kit for amplifying by PCR mycobacterial *rrs* gene sequences in a sample. Such a kit comprises a set of primers as disclosed above, namely at least 3 pairs of primers as disclosed above.

Different amplification methods based on Polymerase Chain Reaction (PCR) can be used, combined with appropriate detection methods, with the kit of the present invention. The conditions of the amplification cycles and the detection methods may however vary depending on the chosen amplification method. The skilled person would know how to adapt such conditions.

The amplification technique may be *inter alia* the LAMP (loop mediated isothermal amplification) technique.

Another technique particularly suitable in the context of the present invention is amplification by qPCR (quantitative PCR, also known as real-time PCR). Quantitative PCR (or qPCR) is based on PCR, and is suitable for amplifying short sequences, and for real time detection. Insofar as the *rrs* sequences amplified by the pairs of primers disclosed in the present application are indeed short sequences, this technique is particularly adapted to the present situation. In the context of the present invention, this technique can be used for detecting the amplification (or absence of amplification) of a sequence, or for the quantification of the amplified product.

Regarding the detection of the amplified product by qPCR, different technologies are available.

One of such technologies is the use of a DNA intercalating agent, such as SYBRGreen. It inserts non-specifically into the minor groove of the double-stranded DNA structure and emits fluorescence. The increase in the fluorescence signal thus indicates an increase in the number of amplified sequences, and thus the presence of the target sequence.

Another mean to detect amplification is the technology based on hydrolysis probes (such as TaqMan^{®} probes), with a dual-labeled probe having a sequence complementary to the amplified target sequence. The probe is coupled to a fluorophore and a quencher. As long as the fluorophore and the quencher are in proximity, the fluorescence signal of the fluorophore is quenched. During the hybridization step of the qPCR, the primers and the probe hybridize to the target sequence. During the elongation, due to the 5'-3' exonuclease activity of the polymerase; the probe is degraded, releasing the fluorophore, thus relieving the quenching effect of the quencher and allowing fluorescence of the fluorophore.

The use of hydrolysis probes generally increases the specificity of quantitative PCR.

Sequences of suitable probes for this invention are disclosed below.

Amplification can also be carried out by digital PCR or dPCR. dPCR is a technique providing an absolute quantification of nucleic acid target sequences; it is an end-point measurement that enables to quantify nucleic acids without the use of standard curves. The PCR reaction mix is portioned in thousand independent reactions. Each reaction is analyzed to determinate if it is positive or negative by a specific software. With a statistic law of Poisson, an absolute quantification of initial copies number/µL is provided.

The detection can be made with hydrolysis probes, or by intercalating agents such as EvaGreen.

The main advantage of this technique is the absence of a reference scale, as the quantification is absolute.

The kit of the invention may advantageously comprise further components, especially components necessary for the amplification by PCR, such as polymerase, particularly DNA polymerase and deoxyribonucleotide triphosphates. This list is not exhaustive. The polymerase is, according to a preferred embodiment, a TaqPolymerase. Many other suitable polymerases are well known to the skilled person and are not detailed here. They will depend on the chosen amplification method.

According to an embodiment, the kit according to the invention does not comprise any further primer in addition to the three pairs of primers disclosed above; or does not comprise any further primer or any further pair of primers hybridizing to a mycobacterial sequence.

According to another embodiment, the kit of the invention may comprise additional primers. Advantageously, the kit may indeed comprise a further pair of primers, namely a forward and reverse primers, suitable to amplify a nucleic acid sequence, used as control. Such a nucleic acid sequence, used as an exogenous control is to be added to the sample, at a defined concentration; or such a nucleic acid sequence, used as an endogenous control is present in the sample at a defined concentration. The exogenous control can be any DNA sequence either already extracted or present in a microorganism unrelated to the mycobacteria to be detected. The endogenous control can be any DNA sequence known as being in the sample and unrelated to the mycobacteria to be detected, for instance in a test of a cell bank, the endogenous control can be a DNA sequence from a gene of the cell, not present in the mycobacterial genome.

According to a further embodiment, the nucleic acid control is part of the kit of the invention and is to be added at a defined concentration to the sample to be tested.

Moreover, the kit may also comprise additional primers for the detection of other contaminants than mycobacteria; especially detection of detrimental or infectious agents such as virus, bacteria, fungus. In order to incorporate these potential additional primers, it is thus an important advantage of the invention to have designed only 3 pairs of primers for the amplification of the whole mycobacteria family.

In a particular embodiment, a kit according to the invention not only comprises a set of primers for amplifying sequences of mycobacterial species, if present in a sample, but also comprises probes to detect the amplification product(s), if any.

According to such an embodiment, the kit of the invention advantageously comprises a Probe S1 having the sequence 5'-CGGTGGGTACTAGGTGTG-3' (SEQ ID NO:3) or modifications thereof, for detection of the sequences amplified by the forward primer F1 and the reverse primer R1 of the 1^{st} pair of primers disclosed above.

In a particular embodiment, the kit of the invention also comprises a probe S3 having the sequence: 5'-TCGGTTCCCTTGTGGC-3' (SEQ ID NO:6) or modifications thereof, for detection of the sequences amplified by the forward primer F3 and the reverse primer R3 of the 2^{nd} pair of primers disclosed above.

In a particular embodiment, the kit of the invention also comprises a probe S4 having the sequence: 5'-ACATGCTACAATGGCCGG-3' (SEQ ID NO:9) or modifications thereof, for detection of the sequences amplified by the forward primer F4 and the reverse primer R4 of the 3^{rd} pair of primers disclosed above. The present inventors have indeed shown in the example section that the probes having SEQ ID NO:3, SEQ ID NO:6 and SEQ ID NO:9 are suitable for detecting the amplified products obtained by amplification with the 1^{st} pair of primers, the 2^{nd} pair of primers and the 3^{rd} pair of primers respectively, either with the original design of the primers, or with the modified sequences as detailed.

They have moreover demonstrated that modifications of the sequence of these probes may nevertheless preserve the ability to detect the amplification products obtained by amplification with the 1^{st} pair of primers, the 2^{nd} pair of primers and the 3^{rd} pair of primers according to the invention.

The modifications of the probes are for example addition and/or deletion of 1-3 nucleotides, particularly only one or two nucleotides, at the 3' terminus and/or at the 5' terminus of the probes.

The probes as defined above have a length entirely compatible with the length of the expected amplified product, when the primers of the invention are used for amplifying a sequence corresponding to the *rrs* gene of a mycobacterial species.

Given the modifications detailed above, according to different embodiments of the present invention, the first probe S1 may have any of the following sequences:
Probe S1-0: 5'-CGGTGGGTACTAGGTGTG-3' (SEQ ID NO:3) corresponding to the original design without modification,
Probe S1-1: 5'-CGGTGGGTACTAGGTGT-3' (SEQ ID NO:22) and
Probe S1-2: 5'-CGGTGGGTACTAGGTG-3' (SEQ ID NO:23).

According to other independent embodiments, the probe S3 is chosen from the group of:
Probe S3-0: 5'-TCGGTTCCCTTGTGGC-3' (SEQ ID NO:6) corresponding to the original design without modification,
Probe S3-1: 5'-ATCGGTTCCCTTGTGGC-3' (SEQ ID NO:24) and
Probe S3-2: 5'-CGGTTCCCTTGTGGC-3' (SEQ ID NO:25).

According to still other independent embodiments, the probe S4 is chosen from the group of:
Probe S4-0: 5'-ACATGCTACAATGGCCGG-3' (SEQ ID NO:9) corresponding to the original design without modification, and
Probe S4-1: 5'-ACATGCTACAATGGCCGGT-3' (SEQ ID NO:26).

According to particular combinations, the probe S1-0 is used in combination with any one of the pairs of primers F1-1 (SEQ ID NO:1) & R1-1 (SEQ ID NO:2); F1-1 & R1-6 (SEQ ID NO:13); F1-1 & R1-5 (SEQ ID NO:12); F1-2 (SEQ ID NO:10) & R1-1; F1-2 & R1-3 (SEQ ID NO:27); F1-2 & R1-5; F1-2 & R1-6 and F1-3 (SEQ ID NO:11) & R1-6.

According to other combinations, the probe S1-1 is used in combination with any one of the pairs of primers F1-1 & R1-1; F1-1 & R1-6; F1-1 & R1-5; F1-2 & R1-1 and F1-3 & R1-6.

According to still other combinations, the probe S1-2 is used in combination with any one of the pairs of primers F1-1 & R1-1; F1-1 & R1-6; F1-1 & R1-5; F1-2 & R1-1, F1-2 & R1-5 and F1-3 & R1-6.

According to particular combinations, the probe S3-0 is used in combination with any one of the pairs of primers F3-1 (SEQ ID NO:4) & R3-1 (SEQ ID NO:5); F3-3 (SEQ ID NO:14) & R3-1; F3-1 & R3-2 (SEQ ID NO:15); F3-1 & R3-3 (SEQ ID NO:16); F3-1 & R3-4 (SEQ ID NO:17); F3-3 & R3-2; F3-3 & R3-3; and F3-3 & R3-4.

According to other combinations, the probe S3-1 is used in combination with any one of the pairs of primers F3-1 & R3-1; F3-3 & R3-1; F3-1 & R3-2; F3-3 & R3-2 and F3-3 & R3-3.

According to still other combinations, the probe S3-2 is used in combination with any one of the pairs of primers F3-1 & R3-1; F3-3 & R3-1; F3-1 & R3-2; F3-1 & R3-3; F3-3 & R3-3; and F3-3 & R3-4.

According to particular combinations, the probe S4-0 is used in combination with any one of the pairs of primers F4-1 (SEQ ID NO:7) & R4-1 (SEQ ID NO:8); F4-3 (SEQ ID NO:18) & R4-1; F4-5 (SEQ ID NO:19) & R4-1; F4-1 & R4-3 (SEQ ID NO:20); F4-1 & R4-5 (SEQ ID NO:28); F4-3 & R4-3; F4-5 & R4-3 and F4-5 & R4-6 (SEQ ID NO:21).

According to other combinations, the probe S4-1 is used in combination with any one of the pairs of primers F4-1 & R4-1; F4-3 & R4-1; F4-5 & R4-1; F4-1 & R4-3; F4-3 & R4-3; F4-5 & R4-3 and F4-5 & R4-6.

In an embodiment, the kit comprises the forward primer F1-1 (SEQ ID NO:1), the reverse primer R1-1 (SEQ ID NO:2) and the probe S1-0 (SEQ ID NO:3).

In another embodiment, the kit comprises the forward primer F3-1 (SEQ ID NO:4), the reverse primer R3-1 (SEQ ID NO:5) and the probe S3-0 (SEQ ID NO:6).

In still another embodiment, the kit comprises the forward primer F4-1 (SEQ ID NO:7), the reverse primer R4-1 (SEQ ID NO:8) and the probe S4-0 (SEQ ID NO:9).

According to a particular embodiment, the kit comprises F1-1, R1-1, S1-0, F3-1, R3-1, S3-0, F4-1, R4-1 and S4-0.

The inventors have indeed demonstrated that the combinations of pairs of primers and associated probes as herein disclosed allow not only to amplify sequences of the *rrs* gene of any mycobacterial species, but moreover allow the detection of only sequences of mycobacterial species, and not of any other bacterial origin. The kit of the invention, with the combination of the specific pairs of primers and corresponding probes provides thus not only the exhaustivity of the mycobacterial detection, but also the specificity of such a detection, with respect to other bacteria. The kit of the invention, with the corresponding probes, is thus a kit of detection of mycobacterial species, providing exhaustivity and specificity.

The kit of the invention may also comprise a probe designed for detecting the sequences amplified from the nucleic acid control, by the further pair of primers disclosed above.

The kit of the invention, with the at least 3 pairs of primers and associated probes disclosed, is particularly suitable for amplification by qPCR or by dPCR. The detection may be carried out with the probes, in order to confer specificity to the mycobacterial detection.

The probes of the kit of the invention are advantageously linked, either directly or indirectly, to a label. Such a label may be any kind of label, especially any kind of fluorophore label, suitable for detecting the probes and adapted to the thermocycler. Particular labels are dye or fluorophore markers. A particular marker illustrated in the example is 6-Carboxyfluorescein (6-FAM or FAM). Several other markers or fluorophores well-known to the skilled person and which can be used are for example Cy3 dye, Cy5 dye, VIC, YAK (Yakima Yellow), Red, HEX, TET, Cyan dyes, etc..

In specific embodiments, the probes are also linked, directly or indirectly, to a quencher, in order to be used as hydrolysis probes.

According to other embodiments, when the kit comprises the probes S1, S3 and S4 detailed above, the 3 probes are linked, directly or indirectly, to an identical label, e.g. a fluorophore marker. Indeed, insofar as the kit allows the exhaustive and selective amplification of sequences of the *rrs* gene of any mycobacterial species, the detection of an amplified product by the probes is indicative of the presence of mycobacterial sequences in the sample; whether the amplified product is detected by the probe S1, S3 and S4 is not necessarily informative. It is to be reminded in this regard that a given *rrs* gene of a mycobacterial species may moreover be amplified and detected by more than one combination of primers and probes.

When the kit further comprises a pair of primers for amplifying a nucleic acid control as well as a probe for detecting the corresponding amplification product, said probe for the nucleic acid control may also be linked directly or indirectly to a label, which is advantageously a label different from the label of the probes S1, S3 and S4. In this way, the amplification resulting from the control system can be immediately distinguished from the amplification resulting from the presence of *rrs* gene sequences of mycobacterial origin. A quencher is also linked to the probe for the control.

The kit as disclosed is for use with samples likely to comprise mycobacterial DNA from mycobacteria, especially obtained after DNA extraction. The mycobacterial DNA must be freely accessible in the sample, i.e. not trapped within a cell, not fragmented, and corresponds to the whole DNA; namely DNA extracted from live mycobacteria, according to protocols not damaging the DNA. A control of DNA extraction may be added if appropriate and can be part of the kit.

The kit according to the invention comprising the specific combination of a set of primers and corresponding probes as disclosed is such that the detection of an amplified product with any of the probes S1, S3 and S4, is indicative of the specific presence of DNA of at least one species of mycobacteria in the sample, more particularly at least one species amongst the 217 different species or subspecies or variants of mycobacteria known to date, irrespective of their classification.

By way of contrast, due to the exhaustivity conferred by the set of primers of the kit, the absence of detection of an amplified product when using the kit of the invention, is indicative of the absence of DNA of any species of mycobacteria in the sample, i.e. the absence of the target sequence to be amplified. According to a particular embodiment, an internal control is added in the kit, in order to ensure that the lack of amplification and detection is indeed due to the absence of the target sequence.

The internal control is for example to be added to the samples at the extraction step. It allows to control this extraction step, in case there is no amplification of mycobacteria, it ensures that this is not linked to a defective extraction step.

According to still further embodiment, a kit according to the invention comprises polymerase chain reaction agents and at least the set of 3 primer pairs according to the invention; with or without the associated probes, and potentially also comprises a DNA extraction kit.

In another aspect, the present invention is directed to different methods for amplifying and detecting sequences of the *rrs* gene of any mycobacteria, using the kit of the invention as described in the preceding sections. Indeed, in view of the exhaustivity of the combination of primers, and the sensitivity of the PCR technique, the invention provides highly sensitive detection tests of mycobacteria, applicable to several domains, especially for the detection of mycobacteria in samples having a very low content of mycobacteria, e.g. below 100 viable mycobacteria per mL, even below 10 viable mycobacteria per mL, or even around 5 or 1 viable mycobacteria per mL.

The invention is *inter alia* directed to such a method, for amplifying and detecting sequences of the *rrs* gene of any mycobacteria present in a sample, comprising the following steps:
a) Carrying out *in vitro* PCR on the nucleic acids extracted from said sample, with at least the set of three pairs of primers as disclosed above and
b) Detecting the presence or absence of an amplified product.

Depending on the method used for the amplification by PCR, it is immediately apparent that the two steps can be carried out simultaneously or sequentially. As explained above, techniques such as qPCR indeed allow to simultaneously carry out amplification and detection of the amplified product.

The step of amplification may be carried out with only three pairs of primers, especially the 1^{st}, 2^{nd} and 3^{rd} pairs of the invention, without additional primers hybridizing to mycobacterial sequences. Such an embodiment does not exclude the amplification of a nucleic acid control, with a pair of primers specific to this control, and unrelated to mycobacterial sequences (internal control). According to such an embodiment, the amplification step a) is carried out with an additional control pair of primers comprising a forward primer and a reverse primer, designed for amplification of a nucleic acid control present or added to the sample; such a nucleic acid control is unrelated to any mycobacterial sequence and is not amplified by the 3 pairs of primers of the invention.

According to particular embodiments, the three pairs of primers comprise or consist in the following primers:
1^{st} Pair:
   Forward primer F1-1: 5'-CCTGGTAGTCCACGCCGTAA-3' (SEQ ID NO:1)
   Reverse primer R1-1: 5'-CGGATCCCAAGGAAGGAAAC-3' (SEQ ID NO:2)
2^{nd} Pair:
   Forward primer F3-1: 5'-CACAGGACGCCGGTAGAGAT-3' (SEQ ID NO:4)
   Reverse primer R3-1: 5'-CATGCACCACCTGCACACA-3' (SEQ ID NO:5)
3^{rd} Pair:
   Forward primer F4-1: 5'-CCCTTATGTCCAGGGCTTCA-3' (SEQ ID NO:7)
   Reverse primer R4-1: 5'-GGCATCGCAGCCCTTTG-3' (SEQ ID NO:8).

Primers having modified sequences with respect to F1-1, F3-1, F4-1, R1-1, R3-1 and R4-1 are also suitable for use in the method of the invention. Suitable modifications have been extensively discussed in connection with the set of primers of the invention and are similarly applicable to this aspect of the invention.

As disclosed in the preceding aspect in connection with the set of primers of the invention, the different pairs of primers may also independently be chosen:
- For the 1^{st} pair, in the list of pairs of primers consisting of F1-1 (SEQ ID NO:1) & R1-1 (SEQ ID NO:2); F1-1 & R1-6 (SEQ ID NO:13); F1-1 & R1-5 (SEQ ID NO:12); F1-2 (SEQ ID NO:10) & R1-1; F1-2 & R1-3 (SEQ ID NO:27); F1-2 & R1-5; F1-2 & R1-6 and F1-3 (SEQ ID NO:11) & R1-6;
- For the 2^{nd} pair, in the list of pairs of primers consisting of F3-1 (SEQ ID NO:4) & R3-1 (SEQ ID NO:5); F3-3 (SEQ ID NO:14) & R3-1; F3-1 & R3-2 (SEQ ID NO:15); F3-1 & R3-3 (SEQ ID NO:16); F3-3 & R3-2; F3-3 & R3-3; F3-1 & R3-4 (SEQ ID NO:17) and F3-3 & R3-4; and
- For the 3^{rd} pair, in the list of pairs of primers consisting of F4-1 (SEQ ID NO:7) & R4-1 (SEQ ID NO:8); F4-3 (SEQ ID NO:18) & R4-1; F4-5 (SEQ ID NO:19) & R4-1; F4-1 & R4-3 (SEQ ID NO:20); F4-1 & R4-5 (SEQ ID NO:28); F4-3 & R4-3; F4-5 & R4-3 and F4-5 & R4-6 (SEQ ID NO:21).

According to the method of the invention, the absence of detection of an amplified product is indicative of the absence of any species of mycobacteria in the sample, or at least any of the 217 species, subspecies and variants of table 4, insofar as the set of primers used in the method confers the exhaustivity to the method, i.e. irrespective of the mycobacterial species potentially present in the sample, its *rrs* gene will be amplified by at least one pair of primers.

Moreover, as detailed in connection with the kits of the invention, the amplification step a) can be carried out by any appropriate PCR method, but highly suitable methods according to the invention are quantitative PCR and digital PCR. Quantitative PCR is illustrated in the example section of the application; similar results are obtained by dPCR.

As immediately apparent from the preceding, the method of the invention does not require any step of culture of mycobacteria. The method can be carried out directly on a sample to be tested, once the nucleic acids are extracted from said sample, without any need for culture. The method is thus particularly rapid, especially with respect to the method based on culture of mycobacteria; the time to results can indeed by reduced by more than 90%, in particular by 96%. The method is also more rapid than methods requiring a step of preculture of the mycobacteria, before amplification. The claimed method requires no culture step at all. The method presents also the advantage of being able to detect any and all mycobacteria, including the fastidious and intracellular mycobacteria species.

According to a particular embodiment, the amplifications with said three pairs of primers are carried out simultaneously, in the same reaction mix, i.e. in triplex. Moreover, when a further pair of primer is also added, for example designed for amplification of a nucleic acid control present or added to the sample, the amplification with said additional pair of primers is also advantageously carried out simultaneously, i.e. in quadruplex. The inventors have indeed demonstrated in example 5 that such an amplification in quadruplex is possible, without negative interaction, thus preserving the exhaustivity of the detection method, and also its specificity, when the adequate probes are used, whilst improving its efficiency. The amplification step a) of the method of the invention is to be carried out in presence of all the appropriate components, necessary for the chosen technique of amplification, namely with the appropriate deoxyribonucleotide triphosphates and polymerase, under conditions appropriate for amplification.

Regarding step b) of detection, this step may be carried out with the associated probes corresponding to the pairs of primers, as already disclosed in connection with the kits of the invention. In this regard, the detection is advantageously carried out with a probe S1, having the sequence 5'-CGGTGGGTACTAGGTGTG-3' (SEQ ID NO:3) or modifications thereof, for detection of the sequences amplified by said primers F1 and R1; with a probe S3 having the sequence: 5'-TCGGTTCCCTTGTGGC-3' (SEQ ID NO:6) or modifications thereof, for detection of the sequences amplified by said primers F3 and R3; and with a probe S4 having the sequence: 5'-ACATGCTACAATGGCCGG-3' (SEQ ID NO:9) or modifications thereof, for detection of the sequences amplified by said primers F4 and R4. The suitable modifications have already been detailed in a previous section and are applicable to this aspect of the invention.

According to a particular embodiment, the probe is chosen:
- for the Probe S1, from the group consisting of :
   Probe S1-0: 5'-CGGTGGGTACTAGGTGTG-3' (SEQ ID NO:3),
   Probe S1-1: 5'-CGGTGGGTACTAGGTGT-3' (SEQ ID NO:22) and
   Probe S1-2: 5'-CGGTGGGTACTAGGTG-3' (SEQ ID NO:23);
   wherein when the Probe S1 is S1-0, the first pair of primers is chosen from:
      a) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
      b) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
      c) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
      d) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
      e) Forward primer F1-2: 5'-CCTGGTAGTCCACGCCGTAAA-3' (SEQ ID NO:10) Reverse primer R1-3: 5'- CACGGATCCCAAGGAAGGAAAC-3' (SEQ ID NO:27);
      f) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12);
      g) Forward primer F1-2: 5'-CCTGGTAGTCCACGCCGTAAA-3' (SEQ ID NO:10) Reverse primer R1-6: 5'-GGATCCCAAGGAAGGAAACC-3' (SEQ ID NO:13); and
      h) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13),
   wherein when the Probe S1 is S1-1, the first pair of primers is chosen from:
      a) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
      b) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
      c) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
      d) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2); and
      e) Forward primer F1-3: 5'-CTGGTAGTCCACGCCGTAAA-3' (SEQ ID NO:11)
         Reverse primer R1-6: 5'-GGATCCCAAGGAAGGAAACC-3' (SEQ ID NO:13),
   wherein when the Probe S1 is S1-2, the first pair of primers is chosen from:
      a) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
      b) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
      c) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
      d) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
      e) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12); and
      f) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13).
- For the Probe S3, from the group consisting of :
   Probe S3-0: 5'-TCGGTTCCCTTGTGGC-3' (SEQ ID NO:6),
   Probe S3-1: 5'-ATCGGTTCCCTTGTGGC-3' (SEQ ID NO:24) and
   Probe S3-2: 5'-CGGTTCCCTTGTGGC-3' (SEQ ID NO:25);
   wherein when the Probe S3 is S3-0, the second pair of primers is chosen from:
      a) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
      b) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
      c) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
      d) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
      e) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15);
      f) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
      g) Forward primer F3-1: 5'-CACAGGACGCCGGTAGAGAT-3' (SEQ ID NO:4) Reverse primer R3-4: 5'-TGCACCACCTGCACACAG-3' (SEQ ID NO:17); and
      h) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17),
   wherein when the Probe S3 is S3-1, the second pair of primers is chosen from:
      a) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
      b) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
      c) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
      d) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15); and
      e) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16),
   wherein when the Probe S3 is S3-2, the second pair of primers is chosen from:
      a) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
      b) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
      c) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
      d) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
      e) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17). And
      f) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16).
- For the Probe S4, from the group consisting of :
   Probe S4-0: 5'-ACATGCTACAATGGCCGG-3' (SEQ ID NO:9), and
   Probe S4-1: 5'-ACATGCTACAATGGCCGGT-3' (SEQ ID NO:26);
   Wherein when the Probe S4 is S4-0, the third pair of primers is chosen from:
      a) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
      b) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
      c) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
      d) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
      e) Forward primer F4-1: 5'-CCCTTATGTCCAGGGCTTCA-3' (SEQ ID NO:7), Reverse primer R4-5: 5'- GCATCGCAGCCCTTTGTA-3' (SEQ ID NO:28);
      f) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
      g) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
      h) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21),
   wherein when the Probe S4 is S4-1, the third pair of primers is chosen from:
      i. Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
      ii. Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
      iii. Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
      iv. Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
      v. Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
      vi. Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
      vii. Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

In an embodiment, the method is carried out with the forward primer F1-1 (SEQ ID NO:1), the reverse primer R1-1 (SEQ ID NO:2) and the probe S1-0 (SEQ ID NO:3).

In another embodiment, the method is carried out with the forward primer F3-1 (SEQ ID NO:4), the reverse primer R3-1 (SEQ ID NO:5) and the probe S3-0 (SEQ ID NO:6).

In still another embodiment, the method is carried out with the forward primer F4-1 (SEQ ID NO:7), the reverse primer R4-1 (SEQ ID NO:8) and the probe S4-0 (SEQ ID NO:9).

According to a particular embodiment, the method is carried out with F1-1, R1-1, S1-0, F3-1, R3-1, S3-0, F4-1, R4-1 and S4-0.

The detection step b) of the method may also be carried out by using a DNA intercalating agent, such as SYBRGreen, as detailed above. In order to confer the specificity, the detection is however in particular embodiments carried out with a probe as disclosed above.

When probes are used for the detection of the potentially amplified products, according to a particular embodiment, the amplification step a) and detection step b) are carried out, in a single reaction mix, comprising the different pairs of primers, namely F1, R1, F3, R3, F4 and R4, as well as the associated probes S1, S3 and S4. The inventors have indeed demonstrated that the simultaneous amplification and detection with three pairs of primers and associated three probes gives a satisfying result.

They have moreover shown that the amplification and detection can also simultaneously be carried out with an additional pair of primers and associated probe, particularly primers and probe specifically designed to amplify and detect a nucleic acid control. The method of the invention thus may also comprise an additional pair of primers and associated probe designed for detection of a nucleic acid control present or added to the sample.

As detailed in connection with the kits of the invention, the probes may be linked, either directly or indirectly, to a label, such as a dye or fluorophore marker. Adequate markers are illustrated in the examples and include 6-FAM.

Moreover, all the particular embodiments already disclosed in connection with the kits of the invention, are entirely applicable to this aspect of the invention in connection with the methods. In this respect, as already detailed, the probes S1, S3 and S4 are linked directly or indirectly to an identical label, and the probe of the control system is linked directly or indirectly to a different label. The reasons underlying this choice have been exposed.

As detailed above, the amplification and detection steps may be carried out simultaneously or sequentially. Step a) may also comprise at least several cycles of amplification by PCR, before step b) is carried out, or before the readout corresponding to step b). There are particularly at least 30 cycles of PCR, for example at least 35 cycles; for example, about 37 cycles, in particular at least or around 40 cycles. A suitable number of cycles can be easily determined by a skilled person, depending on the experimental conditions, in order to discriminate between specific amplification of mycobacterial nucleic acids and non-specific amplification of non-mycobacterial nucleic acids or noise. Determination of an adequate threshold for the number of PCR cycles is illustrated inter alia in Example 8.

The method according to the invention may advantageously be used with different types of samples. The sample may indeed be for example a biopharmaceutical sample, likely to be contaminated by mycobacteria, e.g. due to the use of eukaryotic cells in its production process, or the sample may also be an environmental sample, e.g. water sample from an installation; the sample may also be a biological sample, obtained from an animal or human being likely to be contaminated by mycobacteria. The sample may also be a medium for cell culture, or matrices used for the production of a pharmaceutical product, virus seeds, starting material for cell culture, cell culture or supernatants therefore, intermediate products like crude harvest of drug substance, formulated drug product, or final product. The sample can be from any step of any pharmaceutical product production such as for example CHO cells, used for recombinant protein production, e.g. cytomegalovirus protein antigens, crude harvest of drug substance from MRC5 cells, e.g. used for virus production, such as HAV (hepatitis A virus), crude harvest of drug substance from Vero cells, e.g. used for virus production such as Yellow Fever virus or Rabies virus. The inventors have indeed demonstrated that the specific and exhaustive amplification of any potentially present mycobacterium in these matrices can be carried out according to the method of the invention.

In a particular embodiment, the method is carried out on a biopharmaceutical sample, from a medicament or a vaccine. at any step of the pharmaceutical product production, inter alia on the final product, on any intermediate matrices, on virus seeds, or on starting material, such as cell culture or media.

By the method of the invention, the detection of an amplified product with any of the probes S1, S3 and S4 is indicative of the specific presence of at least one species of mycobacteria in the sample, amongst at least 200 different species of mycobacteria, more particularly amongst the 217 species, subspecies and variants of mycobacteria.

Depending on the expected concentration of mycobacterial DNA and on the PCR mix provider's user manual recommendations, the skilled person will adapt the concentration of the different primers F1, R1, F3, R3, F4 and R4. In the case of a biopharmaceutical sample and detection by qPCR, the concentrations to be used are generally in the range between 100 nM and 400 nM for the primers in the reaction mix.

Similarly, the concentration of the probes will be adapted depending *inter alia* on the expected concentration of mycobacterial DNA, the number of PCR amplification cycles before the detection, and the type of detection technique. In the case of a biopharmaceutical sample, and detection by qPCR, the probes S1, S3 and S4 are generally to be added at concentrations in the range between 50 nM and 250 nM.

According to a particular embodiment, steps a) and b) are carried out simultaneously, in a same reaction mix. The DNA extract of the sample is thus contacted with the three pairs of primers as well as the 3 associated probes simultaneously, in the same reaction mix. As detailed above, a fourth pair of primers for amplifying a control sequence is also advantageously added, still to the same reaction mix, as well as the associated probe. Three or four different pairs of primers and associated probes are thus added to the DNA extract, and the amplification and detection are carried out in this single reaction mix. The inventors have indeed demonstrated that reaction in quadruplex is possible, without losing the exhaustivity and specificity of the detection, even in presence of the associated probes. For a triplex or quadruplex amplification and detection, the PCR technique is for example qPCR or dPCR, and especially qPCR.

Carrying out in triplex or quadruplex the amplification and detection is a major gain of time and also allows to save materials, reagents, machine-time and costs.

The inventors have also demonstrated that the method as disclosed above can detect very small quantities of mycobacteria species, and is thus a highly sensitive method, suitable for use in the pharmaceutical field. The method can indeed detect mycobacterial DNA at concentrations below 10 fg/µL, below 1fg/µL, and even below 0.3 fg/µL, and generally below 0.03 fg/µL, for most strains. By adjusting the DNA extraction step, it is thus possible to detect mycobacteria with a sensitivity essentially similar to, or better than the sensitivity of the compendial method, fulfilling the requirements of Ph Eur. 2.6.2 with respect to mycobacteria detection. The invention provides highly sensitive detection tests of mycobacteria, applicable to several domains, especially for the detection of mycobacteria in samples having a very low content of mycobacteria, e.g. below 100 viable mycobacteria per mL, even below 10 viable mycobacteria per mL, or even around 5 or 1 viable mycobacteria per mL.

The method is thus advantageously an alternative to the compendial method, which moreover can be performed in less than 48 hours, and even in less than 24 hours.

The method is thus highly valuable for biopharmaceutical products testing, especially for vaccine testing, as this method can replace the compendial method, with the same or even increased sensitivity and comparable or increased specificity (see example 8.1), and with a broader range of detection.

According to still another aspect, the present invention is also directed to the use of the set of primers detailed according to a previous aspect, or the use of the kit also detailed, for amplifying by PCR mycobacterial rrs gene sequences present in a sample. It is immediately apparent that all the potential or particular embodiments disclosed in respect of the set of primers and kits of the invention are also applicable to this further aspect. In this regard, adequate pairs of primers have already been disclosed and are also adequate for this aspect; the same is true for the associated probes. Similarly, the different PCR techniques which can be used have already been detailed in connection with the previous aspects; particular techniques are qPCR and dPCR, especially qPCR. The use of the kit of the invention can be for amplifying and detecting mycobacterial rrs gene sequences present in a sample, if appropriate probes for the detection are present in the kit.

The use according to the invention gives rise to an exhaustive amplification of any mycobacterial rrs gene potentially present in the sample. Moreover, by using the kit of the invention, with the associated probes, the use gives rise to a specific detection of the mycobacterial rrs gene, the other bacterial *rrs* genes potentially present in the sample not being detected.

This use according to the invention can be either *in vivo*, *ex vivo* or *in vitro*; but particularly *in vitro.* The use is moreover to be carried out on a sample.

According to a specific embodiment, the invention is directed to the *in vitro* use of at least the three following pairs of primers and associated probes:
System 1:
   Forward primer F1-1: 5'-CCTGGTAGTCCACGCCGTAA-3' (SEQ ID NO:1)
   Reverse primer R1-1: 5'-CGGATCCCAAGGAAGGAAAC-3' (SEQ ID NO:2)
   Probe S1-0: 5'-CGGTGGGTACTAGGTGTG-3' (SEQ ID NO:3)
System 3:
   Forward primer F3-1: 5'-CACAGGACGCCGGTAGAGAT-3' (SEQ ID NO:4)
   Reverse primer R3-1: 5'-CATGCACCACCTGCACACA-3' (SEQ ID NO:5)
   Probe S3-0: 5'-TCGGTTCCCTTGTGGC-3' (SEQ ID NO:6)
System 4:
   Forward primer F4-1: 5'-CCCTTATGTCCAGGGCTTCA-3' (SEQ ID NO:7)
   Reverse primer R4-1: 5'-GGCATCGCAGCCCTTTG-3' (SEQ ID NO:8)
   Probe S4-0: 5'-ACATGCTACAATGGCCGG-3' (SEQ ID NO:9),
for specifically amplifying and detecting by PCR mycobacteria nucleic acids present in a sample without amplifying other bacterial nucleic acids.

In a particular embodiment, only the three pairs of primers and associated probes disclosed are used, potentially in association with other probes or primers unrelated to mycobacterial species.

In the different uses according to the invention, the primers and probes of the three systems are advantageously used simultaneously, i.e. in a single amplification step. A fourth system, related to amplification and detection of a control nucleic acid molecule can also be combined with the first three systems, in these uses.

As for the other aspects of the invention, the PCR technique can be any suitable technique, but is for example qPCR or dPCR, and especially qPCR.

According to another specific embodiment, the invention also concerns a method for detecting the presence of mycobacterial contamination in a pharmaceutical product, e.g. in a vaccine, without any step of culture of mycobacteria, comprising the following steps:
a) amplification *in vitro* by quantitative PCR (qPCR) or dPCR, carried out on the nucleic acids extracted from a sample of said product, with the 1^{st}, 2^{nd} and 3^{rd} pairs of primers described above, which hybridize to the *rrs* gene;
b) detection of the presence or absence of an amplified product by the following probes:
   i) Probe S1 for detection of the sequences amplified by the first set of primers, selected from the group consisting of:
      Probe S1-0: 5'-CGGTGGGTACTAGGTGTG-3' (SEQ ID NO:3),
      Probe S1-1: 5'-CGGTGGGTACTAGGTGT-3' (SEQ ID NO:22) and
      Probe S1-2: 5'-CGGTGGGTACTAGGTG-3' (SEQ ID NO:23);
      wherein when the Probe S1 is S1-1, the first pair of primers is chosen from:
         1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
         2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
         3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
         4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2); and
         5) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
      when the Probe S1 is S1-2, the first pair of primers is chosen from:
         1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
         2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
         3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
         4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
         5) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12); and
         6) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
   ii) Probe S3 for detection of the sequences amplified by the second set of primers, selected from the group consisting of:
      Probe S3-0: 5'-TCGGTTCCCTTGTGGC-3' (SEQ ID NO:6),
      Probe S3-1: 5'-ATCGGTTCCCTTGTGGC-3' (SEQ ID NO:24) and
      Probe S3-2: 5'-CGGTTCCCTTGTGGC-3' (SEQ ID NO:25);
      wherein when the Probe S3 is S3-1, the second pair of primers is chosen from:
         1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
         2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
         3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
         4) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15); and
         5) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
         When the Probe S3 is S3-2, the second pair of primers is chosen from:
         1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
         2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
         3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
         4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
         5) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17); and
         6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
      iii) Probe S4 for detection of the sequences amplified by the third set of primers, selected from the group consisting of :
         Probe S4-0: 5'-ACATGCTACAATGGCCGG-3' (SEQ ID NO:9), and
         Probe S4-1: 5'-ACATGCTACAATGGCCGGT-3' (SEQ ID NO:26);
         wherein when the Probe S4 is S4-1, the third pair of primers is chosen from:
            1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
            2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
            3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
            4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
            5) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
            6) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
            7) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

The steps can be carried out sequentially or simultaneously, for example partially simultaneously. The first pair of primer is for example F1-1 and R1-1. In such a case, the first probe can be any of S1-0, S1-1 and S1-2.

The second pair of primers is for example F3-1 and R3-1. In such a case, the second probe can be any of S3-0, S3-1 and S3-2.

The third pair of primers is for example F4-1 and R4-1. In such a case, the third probe can be any of S4-0 and S4-1.

The probe S1 may be S1-0; in such a case, the 1^{st} pair of primers is chosen from F1-1 & R1-1; F1-1 & R1-6; F1-1 & R1-5, F1-2 & R1-1; F1-2 & R1-3; F1-2 & R1-5; F1-2 & R1-6 and F1-3 & R1-6.

The probe S3 may be S3-0; in such a case, the 2^{nd} pair of primers is chosen from F3-1 & R3-1; F3-3 & R3-1; F3-1 & R3-2; F3-1 & R3-3; F3-1 & R3-4; F3-3 & R3-2; F3-3 & R3-3; and F3-3 & R3-4.

The probe S4 may be S4-0; in such a case, the 3^{rd} pair of primers is chosen from F4-1 & R4-1; F4-3 & R4-1; F4-5 & R4-1; F4-1 & R4-3; F4-1 & R4-5; F4-3 & R4-3; F4-5 & R4-3 and F4-5 & R4-6.

In a particular embodiment, the first pair of primers is F1-1 and R1-1 and the associated probe S1-0; the second pair of primers is F3-1 and R3-1, and the associated probe S3-0 and the third pair of primers is F4-1 and R4-1, and the associated probe S4-0.

The inventors have indeed demonstrated, in the experimental section, that the specificity and sensitivity of the method, primers and probes is not lost in presence of biopharmaceutical matrices, such as cellular supernatants, crude harvests of drug substance or media (see *inter alia* examples 8 and 9).

According to a particular embodiment of this specific method, a fourth pair of primers is added, with an associated probe, amplifying an exogenous nucleic acid molecule, used as control. This fourth pair of primers and associated probe is thus a control system, to be used in combination with the three systems providing amplification and detection of mycobacterial *rrs* gene.

The three pairs of primers and associated probes, hybridizing to the *rrs* gene as well as the fourth pair and associated probe, hybridizing to a control molecule, are for example to be used in quadruplex, in a same reaction mix, as already detailed in connection with the other aspects of the invention.

The method according to the invention is thus such that the detection of an amplified product is indicative of the specific presence of at least one species of mycobacteria in the pharmaceutical product, amongst at least 217 different species, subspecies or variants of mycobacteria and the absence of detection of an amplified product is indicative of the absence of any species of mycobacteria in the pharmaceutical product.

Such a method is thus suitable as an alternative safety release test, and can be used as such, after product validation according to regulatory requirements.

Regarding the three pairs of primers and associated probes, hybridizing to the *rrs* gene, different alternative systems are described with respect to other aspects of the invention and are fully applicable to this method.

The invention is also directed to different processes, especially process to releasing pharmaceutical products having passed the safety release test as disclosed above, based on the absence of detection of any amplified product by using the three pairs of primers and associated probes of the invention.

The invention also concerns a process for producing a biopharmaceutical product, comprising a step of testing for mycobacterial contamination by carrying out the method for detecting sequences of the *rrs* gene of any mycobacteria according to the invention.

The invention also concerns a process for producing a biopharmaceutical product, devoid of mycobacterial contamination, comprising:
- providing a biopharmaceutical product and
- carrying out on the product the method for detecting sequences of the *rrs* gene of any mycobacteria according to the invention.

According to this method, it is to be concluded that the product is devoid of mycobacterial contamination, if no amplification product is detected by the method of the invention.

The biopharmaceutical product may be a biological or pharmaceutical product; a suitable product is a vaccine.

For this aspect also, all the particular features already disclosed in connection with previous aspects of the invention are also applicable.

### EXAMPLES:

Mycobacteria detection is a safety release test, that could be required for biopharmaceutical products expressed in or based on eukaryotic cells. The current method described in the Pharmacopeia is based on microbiological culture (see European Pharmacopoeia. Chapter 2.6.2 and Technical Report Series - TRS 978 section B.11.3.3 of WHO). It requires to inoculate the sample to be tested in triplicate onto two different suitable solid media and into one suitable liquid medium. All the media are to be incubated for 56 days.

The main problems and limitations of the compendial culture-based assay mentioned above are *inter alia* the availability and potential shortages of the specific media, the very long testing turnaround time of 56 days, and the absence of detection of an important human origin species, namely *M*. *leprae,* which is an obligatory intracellular parasite, as well as absence of detection of other intracellular mycobacteria or fastidious species with specific growth needs, and thus a lack of exhaustivity of this assay.

The inventors have thus developed an alternative method, which is:
- specific and exhaustive, i.e. capable of detecting low levels of all mycobacteria species but not other species than mycobacteria;
- rapid, i.e. the result is obtainable in a couple of days, and even less; and
- sufficiently sensitive to replace the compendial method.

Such a method is moreover compatible with the Good Manufacturing Practices.

The different mycobacteria known to date and taken into consideration by the inventors are listed in table 4.

### Example 1: Material and Methods

### Strains:

Strains of mycobacteria which have been used for validating the method, include slow-growing strains and fast-growing strains and are detailed in table 1. The rationale underlying the selection of these strains is explained in example 5.

The growth speed is defined as fast (also defined as rapid) when the reproductive cycle is 7 days or less. The growth speed is defined as slow for mycobacteria having a reproductive cycle of more than 7 days.

**Table 1: list of strains, their reference in the banks of cells, their potential alternative name and their growth speed.**

| Strain | Reference | Heterosynonym* | Growth speed |
|---|---|---|---|
| *Mycobacterium tuberculosis* var. BCG | Internal strain | *Mycobacterium bovis* strain BCG | slow |
| *Mycobacterium avium* | *Mycobacterium avium* subsp. *Avium* Chester (ATCC 15769) | N/A | slow |
| *Mycolicibacter terrae* | ATCC 19616 | *Mycobacterium terrae* | slow |
| *Mycolicibacter hiberniae* | ATCC 49874 | *Mycobacterium hiberniae* | slow |
| *Mycobacterium xenopi* | ATCC 19250 | N/A | slow |
| *Mycobacterium kansasii* | ATCC 12479 | N/A | slow |
| *Mycolicibacterium phlei* | ATCC 11758 | *Mycobacterium phlei* | fast |
| *Mycolicibacterium fortuitum* | ATCC 13756 | *Mycobacterium fortuitum* | fast |
| *Mycolicibacterium smegmatis* | ATCC 10143 | *Mycobacterium smegmatis* | fast |
| *Mycobacteroides chelonae* | ATCC 35752 / 19549/ 19535 | *Mycobacterium chelonae* | fast |
| *Mycobacteroides abscessus* | ATCC 23003 | *Mycobacterium abscessus* | fast |
| *Mycolicibacterium flavescens* | ATCC 23035 | *Mycobacterium flavescens* | fast |

| | | | |
|---|---|---|---|
| * the alternative name or heterosynonym takes account of the two nomenclatures for the Mycobacterium genus, namely according to Gupta *et al,* and to Tortoli *et al.* | | | |

### Production and characterization of a bank of mycobacteria.

The mycobacteria of the bank have been characterized and calibrated with flow cytometry and digital PCR (dPCR).

### DNA extraction and purification

For DNA purification, the QIAmp DNA Micro kit (for the preliminary steps of designs of primers and determination of LOQ) and the QIAamp UCP DNA Micro Kit (Qiagen) are used, according to the manufacturer's recommendations.

Alternative commercially available kits have also been tested, such as the EliGene^{®} MTB Isolation kit (Elisabeth Pharmacon).

### Amplification:

### PCR amplification

The amplification is carried out with ready to use commercially available Master Mix for probe detection, containing DNA Taq polymerase and oligonucleotides appropriate to the PCR technique used, for instance qPCR.

### Control of extraction/ amplification:

An exogenous **internal control** for the extraction and amplification is to be used. The 2 following internal controls have been tested:
- DiaControlDNA^{™}: Diagenode, ref DICD-CY-L100 with a detection with Cy5.

This kit comprises the primers and probe, and the seal Herpesvirus at a concentration of 1000 TCID50 per mL, unrelated to the mycobacterial genes and to the product to be evaluated.
- The second internal control is a plasmid comprising the gene coding for GFP, unrelated to the mycobacterial genes and to the product to be evaluated.

Suitable primers and a corresponding probe have been designed for detecting this control.

### Primers and probes

Primers and probes used for amplifying and detecting amplification product in the *rrs* gene (16S rRNA) are detailed in table 2.

**Table 2: Primers and probes specific to the mycobacterial rrs gene. The fluorophore linked to the probes S1-0, S3-0 and S4-0, at the 5' terminus, is FAM; the quencher (at the 3' terminus) is MGB (Minor Groove Binding).**

| **System 1** | | | | |
|---|---|---|---|---|
| Type | Sequence (5' - 3') | SEQ ID No | Length (bp) | Tm (°C) |
| Forward Primer F1-1 | CCTGGTAGTCCACGCCGTAA | 1 | 20 | 60 |
| Reverse Primer R1-1 | CGGATCCCAAGGAAGGAAAC | 2 | 20 | 59 |
| Probe S1-0 | CGGTGGGTACTAGGTGTG | 3 | 18 | 70 |

| **System 3** | | | | |
|---|---|---|---|---|
| Type | Sequence (5' - 3') | SEQ ID No | Length (bp) | Tm (°C) |
| Forward Primer F3-1 | CACAGGACGCCGGTAGAGAT | 4 | 20 | 59 |
| Reverse Primer R3-1 | CATGCACCACCTGCACACA | 5 | 19 | 60 |
| Probe S3-0 | TCGGTTCCCTTGTGGC | 6 | 16 | 69 |

| **System 4** | | | | |
|---|---|---|---|---|
| Type | Sequence (5' - 3') | SEQ ID No | Length (bp) | Tm (°C) |
| Forward Primer F4-1 | CCCTTATGTCCAGGGCTTCA | 7 | 20 | 59 |
| Reverse Primer R4-1 | GGCATCGCAGCCCTTTG | 8 | 17 | 60 |
| Probe S4-0 | ACATGCTACAATGGCCGG | 9 | 18 | 69 |

The probe used for the detection of the internal control is linked at the 5' terminus to the fluorophore Cy5, and the quencher at the 3' terminus is BHQ1.

### Example 2: Choice of the target, primer and amplification method.

With a view to developing an alternative method to the compendial method for detecting mycobacteria, being more rapid, more exhaustive, and easier to perform, the inventors have tested different methods based on molecular biology rather than culture, and more specifically on sequence amplification.

The inventors have thus first designed *in silico* potentially adequate primers, have tested and compared different amplification methods, and have constructed calibrated banks of mycobacteria, for validating the primers, the specificity and exhaustivity of the detection method, and for evaluating the sensitivity.

### 2.1. Choice of the target to be amplified.

### Genome-wide approach

One approach to define an appropriate target with a view to defining an alternative approach to the compendial method, is to target a single gene or a single sequence, strictly specific to mycobacteria species. For this purpose, sequence alignments, carried out on the whole genome of all species are to be performed. This approach thus necessitates that the whole sequence of all mycobacteria species be available, with a sufficient quality. This is however not the case for all species, jeopardizing the exhaustivity of the method. This approach has thus been disregarded by the inventors.

### Gene- or region-specific approach

Different genes or regions are reported in the art as prime targets for detecting mycobacteria (Bhatnagar, *et al* 2017). After study of these genes or regions, the inventors have however come to the conclusion that, in most cases, these genes are only present in tuberculous mycobacteria, or only in non-tuberculous mycobacteria (NTM). Moreover, the sequence of these genes is not available or reported for all the species of the genus, which is not compatible with the exhaustivity required.

The intergenic sequence between the *rrs* and *rrl* genes, coding for the 23S RNA (ITS1 region) has also been investigated and initially retained. However, the corresponding sequences were not available for all mycobacteria jeopardizing the exhaustivity of the detection; moreover, the ITS1 target appears to be not sufficiently specific for the required specificity.

The *rrs* gene is a gene coding for 16S rRNA and comprises around 1400 base pairs. This is a prime target for detecting specific microorganism, as this gene comprises conserved regions, which are shared by several bacterial species, as well as variable regions, depending on the species. Moreover, the *rrs* gene appears to be well documented for the different species of mycobacteria and was thus retained by the inventors.

A file comprising the sequences of the *rrs* gene in the FASTA format, was created, from the Ezbiocloud database, comprising only sequences, the quality of which has been checked. This file comprises 195 sequences, corresponding to all different species and subspecies referenced as mycobacteria.

These 195 sequences, then completed to cover the 217 sequences known to date, have been aligned with the Clustal omega program, adapted for alignment of multiple sequences, with a view to determining adequate primers.

### 2.2. Design of the primers and probes:

### Single pair of primers approach:

The alignment of all the available *rrs* gene sequences of mycobacteria has been carried out. Once the sequences have been aligned, they are used in the Primer express software (Thermo Fisher Scientific) in order to design pairs of primers, with associated MGB probes (Minor Groove Binder). Different potential pairs are proposed by the software, and the different sequences are then compared with Primer Blast (NCBI) and de Silva test Prime (The German network for bioinformatic infrastructure).

This step is essential not only to check the mycobacterial sequences recognized by the pairs or primers, but also for ensuring the specificity, i.e. the absence of amplification and detection of other bacterial species.

No mismatch of the primers is allowed on the database.

The results obtained by the selected primers are disappointing, as other species than mycobacteria are amplified, insofar as the targeting regions for the 195 species or subspecies are conserved regions in bacteria.

It was thus not possible to design a single pair of primers in order to amplify only and all sequences from mycobacteria.

### Multiplex approach:

As it was not possible to design a single pair of primers in order to amplify only *rrs* sequences from mycobacteria, but all of them, the inventors have then decided to design different groups of mycobacteria, depending on their phylogenetic evolution, before undertaking a search for appropriate pairs of primers. The rationale underlying this approach, is to identify target regions of the *rrs* gene which are more variable but specific to mycobacteria.

Moreover, the inventors have decided for designing the different groups to take advantage of phylogenetic trees elaborated according to the previously defined *Mycobacterium* genus, as the species which are phylogenetically nearer are more likely to present common conserved regions, specific to their group (Gupta *et al,* 2018).

In a first step, two pairs of primers, covering 175 different species have been designed. The first pair, F1-1 & R1-1, allows the detection of 151 species of mycobacteria. The second pair allows to detect 24 additional species, but around one hundred species are amplified by both pairs.

The 20 remaining sequences in the FASTA format are realigned with another alignment software, namely Seaview. The Primer Express (version3) software generates new primers suggestions, which are then compared to the different database. Two additional primer pairs are thus obtained (F3-1 & R3-1 and F4-1 & R4-1), covering 17 additional species, and being specific to *Mycobacterium* genus.

Four different pairs of primers have thus been generated, targeting the same *rrs* gene but on different regions.

Further to the update of the classification, and to a revision of the software defining the primers and probes, it was determined by the inventors that the 2^{nd} pair of primers was not necessary to cover all the mycobacteria, as the systems 1, 3 and 4 cover all species referenced to date.

The list of mycobacteria species aligned, including the accession numbers and the primer pairs giving rise to amplification are detailed in table 4. This table is based on the List of Prokaryotic names with Standing in Nomenclature (LPSN) and on the publications Parte *et al,* 2018, Parte *et al* 2020, Parte et *al* 2014 and Euzéby 1997.

**Table 4:**

| Species | Sub-species | n°Accession GenBank | Detection by F1-1 & R1-1 | Detection by F3-1 & R3-1 | Detection by F4-1 & R4-1 |
|---|---|---|---|---|---|
| **Mycobacteroides** / **fast growing** | | | | | |
| *M*. *abscessus* | subsp. Abscessus | CU458896 | YES | NO | NO |
| | subsp. Bolletii | AHAS01000006 | YES | NO | NO |
| | subsp. Massiliense | AKVF01000003 | YES | NO | NO |
| *M. chelonae* | subsp. Gwanakae | MG780327.1 | YES | NO | NO |
| | subsp. Bovis | KY593892.1 | YES | NO | NO |
| | subsp. Chelonae | CP010946 | YES | NO | YES |
| *M. immunogenum* | | CP011530 | YES | NO | NO |
| *M. salmoniphilum* | | DQ866768 | YES | NO | NO |
| *M. stephanolepidis* | | AB971866 | YES | NO | NO |
| *M. franklinii* | | MAFQ01000001 | YES | NO | NO |
| *M. saopaulense* | | CP010271 | YES | NO | NO |

| **Mycolicibacterium / fast growing** | | | | | |
|---|---|---|---|---|---|
| *M. fortuitum* | subsp. Fortuitum | ALQB01000301 | YES | NO | YES |
| | subsp. Acetamidolyticum | BCSZ01000080 | YES | NO | YES |
| *M. acapulcense* | | LT592223 | YES | NO | YES |
| *M. agri* | | AJ429045 | NO | NO | YES |
| *M. aichiense* | | X55598 | YES | NO | YES |
| *M. alvei* | | AF023664 | YES | NO | YES |
| *M. anyangense* | | KJ855063 | YES | NO | YES |
| *M. aquaticus* | | MVHF01000088 | YES | NO | NO |
| *M. aquiterrae* | | FJ796421.2 | YES | NO | YES |
| *M. arabiense* | | KC010491 | YES | NO | NO |
| *M. arcueilense* | | KP644745 | YES | NO | YES |
| *M. aromaticivorans* | | JALN02000001 | YES | NO | YES |
| *M. aubagnense* | | AY859683 | YES | NO | YES |
| *M. aurum* | | CVQQ01000002 | YES | NO | YES |
| *M. austroafricanum* | | HG964453 | YES | NO | YES |
| *M. bacteremicum* | | FJ172308 | YES | NO | YES |
| *M. boenickei* | | FUWC01000003 | YES | NO | YES |
| *M. brisbanense* | | AY012577 | YES | NO | NO |
| *M. brumae* | | AF480576 | YES | NO | YES |
| *M. canariasense* | | BCSY01000102 | YES | NO | YES |
| *M. celeriflavum* | | KJ607136 | YES | NO | YES |
| *M. chitae* | | X55603 | YES | NO | YES |
| *M*. *chlorophenolicum* | | JYNL01000068 | YES | NO | YES |
| *M. chubuense* | | JYNX01000034 | YES | NO | YES |
| *M. conceptionense* | | LQOP01000038 | YES | NO | YES |
| *M. confluentis* | | LQOQ01000001.1 | YES | NO | YES |
| *M. cosmeticum* | | CCBB010000001 | YES | NO | YES |
| *M. crocinum* | | DQ534008 | YES | NO | NO |
| *M. diernhoferi* | | X55593 | YES | NO | YES |
| *M. doricum* | | AF264700 | YES | NO | YES |
| *M. duvalii* | | U94745 | YES | NO | YES |
| *M. elephantis* | | AJ010747 | YES | NO | NO |
| *M. fallax* | | AF480600 | YES | NO | YES |
| *M. farcinogenes* | | HG964483 | YES | NO | YES |
| *M*. *flavescens* | | X52932 | YES | NO | YES |
| *M*. *fluoranthenivorans* | | AJ617741 | YES | NO | YES |
| *M*. *frederiksbergense* | | AJ276274 | YES | NO | YES |
| *M. gadium* | | X55594 | YES | NO | YES |
| *M. gilvum* | | X81996 | YES | NO | YES |
| *M. goodii* | | AY457079 | YES | NO | YES |
| *M. grossiae* | | CP043474.1 | YES | NO | YES |
| *M. hassiacum* | | KB903880 | NO | NO | NO ** |
| *M. helvum* | | KM216314 | YES | NO | YES |
| *M. hippocampi* | | FN430736 | YES | NO | NO |
| *M. hodleri* | | X93184 | YES | NO | YES |
| *M. holsaticum* | | AJ310467 | YES | NO | YES |
| *M. houstonense* | | FJVO01000061 | YES | NO | YES |
| *M. insubricum* | | EU605695 | YES | NO | YES |
| *M. iranicum* | | HQ009482 | YES | NO | YES |
| *M. komaniense* | | KJ873240.2 | YES | NO | YES |
| *M. komanii* | | CVTA01000034 | YES | NO | YES |
| *M. komossense* | | X55591 | YES | NO | YES |
| *M. kyogaense* | | MH401762 | YES | NO | YES |
| *M. lehmannii* | | NR_159196.1 (NCBI Ref Seq) | YES | NO | YES |
| *M. litorale* | | NR_117568.1 (NCBI Ref Seq) | YES | NO | YES |
| *M. llatzerense* | | AJ746070 | YES | NO | NO |
| *M. lutetiense* | | KP676904 | YES | NO | YES |
| *M. madagascariense* | | X55600 | YES | NO | YES |
| *M. mageritense* | | CCBF010000003 | YES | NO | YES |
| *M*. *malmesburyense* | | KJ873241 | NO | NO | YES |
| *M. monacense* | | AF107039 | YES | NO | YES |
| *M. montmartrense* | | KP676919 | YES | NO | NO |
| *M. moriokaense* | | AJ429044 | YES | NO | YES |
| *M. mucogenicum* | | AY457074 | YES | NO | YES |
| *M. murale* | | Y08857 | YES | NO | YES |
| *M. neglectum* | | AB741461.1 | YES | NO | YES |
| *M. neoaurum* | | JMDW01000030 | YES | NO | YES |
| *M. neumannii* | | NR_159197 (NCBI Ref Seq) | YES | NO | YES |
| *M. neworleansense* | | CWKH01000003 | YES | NO | YES |
| *M. novocastrense* | | U96747 | YES | NO | YES |
| *M*. *obuense* | | JYNU01000003 | YES | NO | YES |
| *M. oryzae* | | LN846834 | NO | NO | YES |
| *M. palauense* | | NZ_NVQF010001 59.1 | YES | NO | YES |
| *M. pallens* | | DQ370008 | YES | NO | YES |
| *M. parafortuitum* | | X93183 | YES | NO | YES |
| *M. peregrinum* | | AF058712 | YES | NO | YES |
| *M. phlei* | | ANBO01000010 | YES | NO | YES |
| *M. phocaicum* | | AY859682 | YES | NO | YES |
| *M. porcinum* | | AY457077 | YES | NO | YES |
| *M. poriferae* | | AF480589 | YES | NO | YES |
| *M. psychrotolerans* | | AJ534886 | YES | NO | YES |
| *M. pulveris* | | AJ429046 | YES | NO | YES |
| *M. pyrenivorans* | | AJ431371 | YES | NO | YES |
| *M. rhodesiae* | | AJ429047 | YES | NO | YES |
| *M. rufum* | | JROA01000001 | YES | NO | YES |
| *M. rutilum* | | LT629971 | YES | NO | YES |
| *M. sarraceniae* | | KM216315 | YES | NO | YES |
| *M. sediminis* | | KC010490 | YES | NO | NO |
| *M. senegalense* | | AY457081 | YES | NO | YES |
| *M. septicum* | | HG322957 | YES | NO | YES |
| *M*. *setense* | | JTJW01000014 | YES | NO | YES |
| *M. smegmatis* | | LN831039 | YES | NO | YES |
| *M. sphagni* | | FR733719 | YES | NO | YES |
| *M.syngnathidarum* | | MG015886 | YES | NO | YES |
| *M. thermoresistibile* | | AGVE01000014 | NO | NO | NO ** |
| *M. tokaiense* | | AF480590 | YES | NO | YES |
| *M. tusciae* | | AF058299 | YES | NO | YES |
| *M. vaccae* | | JH814714 | YES | NO | YES |
| *M. vanbaalenii* | | CP000511 | YES | NO | YES |
| *M. vulneris* | | EU834055 | YES | NO | YES |
| *M. wolinskyi* | | Y12873 | YES | NO | YES |

| **Mycolicibacter** / **slow growing** | | | | | |
|---|---|---|---|---|---|
| *M. terrae* | | X52925 | NO | NO | YES |
| *M. algericus* | | GU564404 | NO | NO | YES |
| *M. arupensis* | | DQ157760 | NO | YES | NO |
| *M. engbaekii* | | AF480577 | NO | YES | NO |
| *M. heraklionense* | | GU084182 | NO ** | NO | NO ** |
| *M. hiberniae* | | X67096 | NO | YES | NO |
| *M*. *kumamotonensis* | | AB239925 | NO | YES | YES |
| *M. longobardus* | | JN571166 | NO | NO | YES |
| *M. minnesotensis* | | MVHZ01000040 | NO | YES | NO |
| *M*. *nonchromogenicus* | | X52928 | YES | YES | NO |
| *M. paraterea* | | EU919229 | NO | YES | NO |
| *M.senuensis* | | DQ536408 | NO | YES | NO |
| *M. virginiensis* | | KR025879 | NO | YES | NO |

| **Mycolicibacillus** / **slow growing** | | | | | |
|---|---|---|---|---|---|
| *M. trivialis* | | X88924 | YES | NO | YES |
| *M. koreensis* | | JF271826 | YES | NO | YES |
| *M. parakoreensis* | | JF271823 | YES | NO | YES |

| **Mycobacterium** / **slow growing** | | | | | |
|---|---|---|---|---|---|
| *M. tuberculosis* | var. tuberculosis | AL123456 | YES | NO | YES |
| | var. bovis | MWXE01000056 | YES | NO | YES |
| | var. BCG | AM408590 | YES | NO | YES |
| | var. caprae | AJ131120 | YES | NO | YES |
| | var. africanum | AF480605 | YES | NO | YES |
| | var. canetii | NZ_JACEGU010000027.1 | YES | NO | YES |
| | var. microti | AF480584 | YES | NO | NO |
| | var. mungi | NZ_LXTB01000088.1 | YES | NO | YES |
| | var. pinnipedii | AF502574 | YES | NO | NO |
| *M. alsense* | | AJ938169 | YES | NO | YES |
| *M. angelicum* | | AM884328 | YES | NO | YES |
| *M. arosiense* | | EF054881 | YES | NO | YES |
| *M. asiaticum* | | HG964937 | YES | NO | YES |
| *M. attenuatum* | | LS999934 | YES | NO | YES |
| *M. avium* | subsp. Avium | ACFI01000053 | YES | NO | YES |
| | subsp. Paratuberculosis | AGAR01000357 | YES | NO | YES |
| | subsp. Silvaticum | AYOC01000269 | YES | NO | YES |
| | subsp. Hominissuis | LVCS01000100 | YES | NO | YES |
| *M. basiliense* | | LR216719.1 | YES | NO | YES |
| *M. bohemicum* | | CSTD01000001 | YES | NO | YES |
| *M. botniense* | | AJ012756 | NO | NO | YES |
| *M*. *bouchedurhonense* | | EF591053 | YES | NO | YES |
| *M. bourpelatii* | | HF674384 | YES | NO | YES |
| *M. branderi* | | X82234 | YES | NO | YES |
| *M. celatum* | | BBUN01000124 | YES | NO | YES |
| *M. chimaera* | | MNAM01000009 | YES | NO | YES |
| *M. colombiense* | | AFVW01000001 | YES | NO | YES |
| *M. conspicuum* | | X88922 | YES | NO | YES |
| *M. cookii* | | AF480598 | YES | NO | YES |
| *M. decipiens* | | NZ_NCXP01000009.1 | YES | NO | YES |
| *M. eburneum ** | | KX879093 | NO | NO | YES |
| *M. europaeum* | | HM022196 | YES | NO | YES |
| *M. florentinum* | | AJ616230 | NO | NO | YES |
| *M. fragae* | | JQ898451 | YES | NO | YES |
| *M. gastri* | | AZYN01000124 | YES | NO | YES |
| *M. genavense* | | JAGZ01000001 | NO ** | NO | NO ** |
| *M. gordonae* | | X52923 | YES | NO | YES |
| *M. haemophilum* | | CP011883 | YES | NO | YES |
| *M. heckshornense* | | AF174290 | NO | NO | YES |
| *M.heildelbergense* | | AJ000684 | YES | NO | YES |
| *M. helveticum* | | VMQU01000043.1 | YES | NO | YES |
| *M. innocens* | | LS999933 | YES | NO | YES |
| *M. interjectum* | | FJVQ01000132 | YES | NO | YES |
| *M. intermedium* | | X67847 | YES | NO | YES |
| *M. intracellulare* | subsp. Intracellulare | ABIN01000139 | YES | NO | YES |
| | subsp. Yongonense | CP003347 | YES | NO | YES |
| *M. kansasii* | | ACBV01000020 | YES | NO | YES |
| *M. kubicae* | | AF133902 | YES | NO | YES |
| *M. kyorinense* | | AB370111 | YES | NO | YES |
| *M. lacus* | | AF406783 | YES | NO | YES |
| *M. lentiflavum* | | AF480583 | NO | NO | YES |
| *M. leprae* | | AL450380 | YES | NO | YES |
| *M. lepraemurium* | | CP021238.1 | YES | NO | YES |
| *M. malmoense* | | X52930 | YES | NO | YES |
| *M. mantenii* | | FJ042897 | YES | NO | YES |
| *M. marinum* | | AJ536032 | YES | NO | YES |
| *M. marseillense* | | EU266632 | YES | NO | YES |
| *M. montefiorense* | | AF330038 | NO | NO | YES |
| *M. nebraskense* | | AY368456 | YES | NO | YES |
| *M. noviomagense* | | EU239955 | YES | NO | YES |
| *M. palustre* | | AJ308603 | YES | NO | YES |
| *M. paraense* | | KJ948996 | YES | NO | YES |
| *M. paraffinicum* | | GQ153270 | YES | NO | YES |
| *M. paragordonae* | | KC525204 | YES | NO | YES |
| *M*. *paraintracellulare* | | KP670329 | YES | NO | YES |
| *M*. *parascrofulaceum* | | ADNV01000350 | YES | NO | YES |
| *M. paraseoulense* | | DQ536404 | YES | NO | YES |
| *M. parmense* | | AF466821 | YES | NO | YES |
| *M. persicum* | | MVIF01000072 | YES | NO | YES |
| *M. pseudokansasii* | | LS999932.1 | YES | NO | YES |
| *M. pseudoshotsii* | | BCND01000025 | YES | NO | NO |
| *M. riyadhense* | | EU274642 | YES | NO | YES |
| *M*. *saskatchewanense* | | AY208856 | YES | NO | YES |
| *M. scrofulaceum* | | AF480604 | YES | NO | YES |
| *M. seoulense* | | DQ536403 | YES | NO | YES |
| *M. sherrisii* | | AY353699 | NO | NO | YES |
| *M. shigaense* | | AB547401 | NO | NO | YES |
| *M. shimoidei* | | AJ005005 | NO | NO | YES |
| *M. shinjukuense* | | AB268503 | NO | NO | YES |
| *M. shottsii* | | AY005147 | YES | NO | YES |
| *M. simiae* | | CBMJ020000103 | NO | NO | YES |
| *M. stomatepiae* | | AM884331 | NO ** | NO | NO ** |
| *M.szulgai* | | X52926 | YES | NO | YES |
| *M. talmoniae* | | KX008970 | NO | NO | YES |
| *M. timonense* | | EF591054 | YES | NO | YES |
| *M. triplex* | | HG964446 | NO | NO | YES |
| *M. ulcerans* | | AB548725 | YES | NO | NO |
| *M. vicinigordonae* | | CP059165.1 | YES | NO | YES |
| *M. xenopi* | | AJ536033 | NO | NO | YES |

| | | | | | |
|---|---|---|---|---|---|
| ** Not a 100% detecting according to *in silico* study, but the detection has been validated by *in vitro* testing. ***: *M. eburneum :* close to slow growing mycobacteria (*M. paraense* et *M. cookii*) but fast growing mycobacteria. | | | | | |

As can be seen in table 4, for 5 species, namely *M*. *genavense*, *M. stomatepiae*, *M. hassiacum*, *M. thermoresistible* and *M*. *heraklionense*, there is a mismatch between one of the primers and the *rrs* gene sequence of these species. Given the position of these mismatches on the primer, and the fact that the mismatches concern primers and not probes, it is to be deduced that such a mismatch should not hinder the amplification. This point has been confirmed by the inventors (see example 3).

*Conclusions*: The 3 pairs of primers F1-1 & R1-1, F3-1 & R3-1 and F4-1 & R4-1, allow to detect the *rrs* gene of mycobacteria and to cover all the mycobacteria species.

### Probes and confirmation of the specificity:

Using the same software, probes specific to the sequences amplified by the 3 pairs of primers F1-1 & R1-1, F3-1 & R3-1 and F4-1 & R4-1 have been designed, namely S1-0, S3-0 and S4-0 respectively. A perfect match between these probes and the amplified sequences is necessary.

The inventors have moreover conducted an analysis using the Primer-BLAST software, developed at NCBI to help users make primers that are specific to intended PCR target. This program uses BLAST and global alignment algorithm to screen primers against user-selected database in order to avoid primer pairs (all combinations including forward-reverse primer pair, forward-forward as well as reverse-reverse pairs) that can cause non-specific amplifications. In the present case, the selected database was RefSeq Representative Genome Database (Organism limited to Bacteria).

The parameters of Primer BLAST were set as follows:

| **Search parameters and other details** | |
|---|---|
| Entrez query | |
| Min total mismatches | **2** |
| Min 3' end mismatches | 2 |
| Defined 3' end region length | 5 |
| Mismatch threshold to ignore targets | 1 |
| Max target size | 4000 |
| Max number of Blast target sequences | 50000 |
| Blast E value | 30000 |
| Blast word size | 7 |
| Max candidate primer pairs | 500 |
| Min PCR product size | 60 for F1-1 & R1-1 |
| | 59 for F3-1 & R3-1 |
| | 57 for F4-1 & R4-1 |
| Max PCR product size | 1000 |
| Min Primer size | 15 |
| Opt Primer size | 20 |
| Max Primer size | 25 |
| Min Tm | 57 |
| Opt Tm | 60 |
| Max Tm | 63 |
| Max Tm difference | 3 |
| Repeat filter | AUTO |
| Low complexity filter | Yes |

The number of Blast hits analyzed was 36477 for F1-1 & R1-1, 8072 for F3-1 & R3-1 and 8971 for F4-1 & R4-1.

The results are as follows:

### For F1-1 & R1-1:

With 2 mismatches authorized, the specificity is 98.72%.

Two of the 156 amplified sequences are indeed not from mycobacteria. These two sequences are from:
- *Lujinxingia litoralis* strain B210 B210. The amplified fragment comprises however more than 1000 basepairs, and is thus not compatible with qPCR. In any event, this amplified fragment does not hybridize with the probe S1-0.
- *Virgibacillus indicus* strain P2-C2. No amplification is however detected with the probe S1-0.

The specificity is thus 100% with the chosen probe S1-0.

### For F3-1 & R3-1:

With 2 mismatches authorized, the specificity is 100 %.

13 sequences are amplified, all from mycobacteria.

### For F4-1 & R4-1:

With 2 mismatches authorized, the specificity is 95,65%.

Eight of the 184 amplified sequences are indeed not from mycobacteria, but do not hybridize with the probe S4-0.

Conclusions: The 3 pairs of primers F1-1 & R1-1, F3-1 & R3-1 and F4-1 & R4-1, with the corresponding probes S1-0, S3-0 and S4-0, allow to specifically detect all the mycobacteria species without detecting other bacteria.

### 2.3. Elaboration of calibrated mycobacteria banks

The production of mycobacteria cell banks is performed during the exponential phase of growth. Characterization of viable mycobacteria concentration is performed by flow cytometry method, and the number of Genome copy (GC) (alive and dead bacteria) is determined by classical methods known to the person skilled in the art (like qPCR, dPCR, fluorimetry or UV detection) in mono copy reference gene.

A ratio between GC/mL and viable mycobacteria/mL is calculated and must be as close as possible to 1 (mainly viable microorganisms) in order not to favor the method based on genome amplification with respect to the method based on culture.

This calibration method provides suitable mycobacteria cell bank for PCR use.

### 2.4. Comparison of the sensitivity obtained with different amplification methods.

Quantitative PCR (qPCR), also known as real-time PCR, and digital PCR (dPCR) have been evaluated in parallel.

For the pairs of primers F1-1 & R1-1 and F4-1 & R4-1 (as well as F2-R2, although this pair was finally not retained), these pairs amplify a sequence which is present in *M*. *bovis* genome (now classified as *M. tuberculosis* var BCG further to the last evolution of the nomenclature). This mycobacterium was thus used for the determination of the limit of detection for these pairs of primers, according to the amplification method, i.e. qPCR or dPCR, on the basis of the evaluated concentration as detailed in section 2.3. The mycobacterial DNA was extracted with commercial suitable kits, used according to the manufacturer's recommendations.

For the pair F3-1 & R3-1, corresponding DNA of *M*. *hiberniae* was used.

### Amplification by dPCR

This technique was first tested by the inventors with primers targeting the ITS1 region.

The inventors have then tested this technique with the pairs of primers targeting the *rrs* gene (F1-1 & R1-1, F2 & R2 and F4-1 & R4-1) on *M*. *bovis* DNA, with a view to evaluating the limit of detection of this technique.

Dilutions of the samples are carried out, until extinction of the signal, i.e. no positive droplet is detected. The limit of detection is equivalent for the 3 pairs of primers tested (including the pair F2-R2, however not retained), irrespective of the detection system, i.e. by probe or by Evagreen.

The limit of detection is around a dilution of up to 10⁻⁴ (for *M*. *tuberculosis* var BCG) according to the initial tests carried out by the inventors (on a bank roughly calibrated by optical density).

By optimizing the steps of DNA extractions, PCR conditions, including the number of cycles, an improved sensitivity is to be expected.

### Amplification by qPCR

In order to evaluate the limit of detection for this technique, an initial reference range was prepared, allowing to estimate the quantity of DNA present from the detected amplification.

For this purpose, for each chosen target, a corresponding synthetic reference nucleotide sequence was synthesized and introduced into a plasmid and quantified.

Using the pairs of primers F1-1 & R1-1, F2-R2 and F4-1 & R4-1, the associated probes S1-0, S2 and S4-0 respectively, and the synthetic sequences, for which the quantity in ng/mL is known, the inventors were then able to set a reference range for the detection of the targeted sequences by qPCR with a probe.

The limit of detection is around a dilution of up to 10⁻⁵ (for *M*. *tuberculosis* var BCG) according to the initial tests carried out by the inventors (on a bank roughly calibrated by optical density).

In view of the sensitivity and rapidity of qPCR, which is also less expensive than -dPCR, qPCR has been selected by the inventors for the next steps. This technique moreover fulfils the requirements of Good Manufacturing Practice (GMP).

### Example 3: Validation of the exhaustivity.

For 5 species, namely *M*. *genavense*, *M. stomatepiae*, *M. hassiacum*, *M. thermoresistible* and *M*. *heraklionense*, there is a mismatch between one of the primers and the *rrs* gene sequence of these species. Given the position of these mismatches on the primer, and the fact that the mismatches concern primers and not probes, it is to be deduced that such a mismatch should not hinder the amplification. The inventors have confirmed this point by using a synthetic rrs gene, corresponding to each one of these 5 species, in order to test the 3 sets of primers.

The results obtained by the inventors have confirmed that F1-1 & R1-1 and/or F4-1 & R4-1 are able to amplify the *rrs* gene of these 5 species. These results are reported in table 4, "NO*" means no amplification on the basis of *in silico* study, but amplification *in vitro.*

The combination of the 3 pairs of primers disclosed in example 2 thus allows the amplification of the *rrs* gene of every and all mycobacteria.

### Example 4: Validation of multiplex amplification.

In a first step, the inventors have carried out a duplex amplification. Samples of *M*. *tuberculosis* var BCG at different concentrations have been prepared with or without the internal control (IC), i.e. spiked with either the internal control of the Diagenode kit (spiking at 10 TCID50) or with the internal control GFP plasmid (spiking at 10⁶ copies of plasmid per 5 µL). Both tested internal controls were working well (data not shown), but for further steps, the inventors used the GFP IC.

The different samples (comprising the extracted DNA from *M*. *tuberculosis* var BCG at different concentrations with the internal control) have been analyzed by qPCR in the following conditions:
- Simplex: only the pair of primers F1-1 & R1-1 for amplifying the *rrs* gene, or only the pair of primers for amplifying the internal control with the suitable probe, and
- Duplex: simultaneously with the pair of primers F1-1 & R1-1 and the pair of primers of IC, with the suitable probe.

The results obtained show that it is possible to simultaneously detect both the targeted *rrs* gene and the internal control. There is no difference in the detection of the targeted *rrs* gene or the internal control, between the simplex and duplex settings, as illustrated in FIG.1A and B.

As can be also seen on these figures, there is no fluorescence interference between the probes used for the *rrs* gene (fluorophore FAM) and the probe used for the internal control (fluorophore Cy5) as the wavelength are sufficiently distinct, namely:
FAM: excitation at 490 nm / Emission at 520 nm
Cy5: excitation at 650 nm / emission at 670 nm.

In order to confirm the above results, the experiments have been reproduced with the 3 different pairs of primers and associated probes.
*M. tuberculosis* var BCG has been extracted at 10⁵, 10⁴ and 10³ mycobacteria per mL and the GFP plasmid has been spiked at the concentration of 10⁵ copies per 5µL. The same has been carried out with the DNA of *M*. *hiberniae* at a concentration of 5.3 pg/µL and 5.3 fg/µL.

Using qPCR, the 3 systems (primers and corresponding probes) disclosed in example 2 as well as the primers and probe for GFP plasmid have first been tested in simplex reaction and then in duplex.

The inventors have then concluded that the amplification in simplex or duplex does not modify the results obtained by qPCR.

### Detection in multiplex

The inventors have first tested a duplex qPCR amplification, with the targeted M. *tuberculosis* var BCG and both the first system (F1-1, R1-1 and S1-0) and 4^{th} system (F4-1, R4-1 and S4-0).

The results show that the duplex qPCR is possible and allows a better detection of the mycobacteria. Indeed, these systems of detection, i.e. the first and 4^{th} system, amplify a distinct region of the *rrs* gene, such that there is no competition between the primers with regard to the targeted DNA. The other components of the master mix (e.g. dNTP, polymerase, etc...) are in excess, also ensuring the absence of competition between the primers with respect to the other resources. Insofar as the probes of both systems are detected by the same fluorophore, the signal is even amplified, as there is a twofold increase of the amplified sequences. This amplification of the signal is expected for all the species likely to be amplified by at least two of the three systems disclosed in example 2.

The inventors have then added the internal control, namely GFP internal control, with the associated primers and probe, and conducted the qPCR in triplex. The results obtained have shown that the triplex reaction does not negatively impact the detection of the internal control. Similarly, the detection of the targeted *rrs* gene is homogenous.

The inventors have therefore tested a quadruplex qPCR. In this respect, 4 different reactions have been carried out:
- 3 duplex reactions, corresponding to one of the 3 systems (system 1, system 3 or system 4) and the internal control (GFP plasmid) and associated primers and probe;
- 1 quadruplex with the 3 systems and the internal control.

A side-by-side comparison of the results obtained for each system, in combination either only with the internal control, or with the internal control and the two additional systems, allows to conclude that the quadruplex amplification gives satisfying results with an adequate sensitivity and specificity. Indeed, as can be seen in FIG.2, for each system (1^{st} system: FIG. 2A; 3^{rd} system FIG.2B and 4^{th} system FIG.2C), the detection of the targeted sequence is either earlier or amplified in quadruplex, with respect to the detecting in duplex, with only the control.

### Example 5: Validation of multiplex amplification on representative species.

The detection method disclosed in example 2 has then been experimentally validated by the inventors on mycobacteria, in quadruplex conditions detailed in example 4.

The 12 species/strains selected in the development and validation of the detection method are listed in example 1, table 1. They represent a sample of the different genera comprised in the mycobacteria (according to the former classification, as the single *Mycobacterium* genus has now been reconstituted according to the latest classification, however without modifying the bacteria considered as mycobacteria); indeed, 4 out of the 5 different mycobacterial genera are represented, namely *Mycobacteroides, Mycolicibacterium, Mycolicibacter* and *Mycobacterium.* The *Mycolibacillus* is not represented as it comprises only 3 non usual mycobacteria having only a very low occurrence.

*M. xenopi* and *M*. *chelonae* being available in insufficient quantities for this experiment, the inventors have used a synthetic *rrs* gene coding to the 16S rRNA from these species. Regarding *M. hiberniae,* the inventors have worked with DNA already extracted from this mycobacterium for safety reasons.

All the strains, including those only represented by DNA, have been extracted by one of the kit mentioned in example 1; the samples are then spiked with the internal control (GFP plasmid at 10⁵ copies per 5µL).

The samples are then analyzed by qPCR, using a commercial kit, in quadruplex.

A standard range is obtained in order to quantify the extracted samples, for defining the limit of detection of the test, for each tested strain. This standard range is calibrated in pg/µL of DNA and is obtained with 6 points and 3 replications. The error margin must be below 0.1 and the linear regression coefficient must be as close as possible to 1.

Each strain extract is first analyzed by qPCR without dilution, and then diluted up to 10⁻³, in order to estimate the limit of detection for each strain.

The results are depicted on FIG. 3 (FIG.3A: slow-growing mycobacteria; FIG.3B: fast-growing mycobacteria).

In order for a given dilution to be considered as "quantifiable" in quadruplex condition, it is necessary that both replicates are similar (a difference inferior to 0.8 Cp) and the result regarding Cp in coherence with the previous dilution, i.e. 3.3 Cp apart from the previous dilution.

Cp means "Crossing point", it is also named Cq, and is the number of amplification cycles beyond which the amplification is considered as significative, i.e. above the threshold level. The threshold level is illustrated by a line on FIG.1, 2 and 3.

Table 5 summarizes the minimal quantity of DNA amplified for each strain, by the quadruplex detection system. This is not the limit of detection of the method, but the proof that the method is suitable for detecting very small DNA quantities, for each species.

**Table 5:**

| Species | Type | Minimal detection fg/µL |
|---|---|---|
| *M. tuberculosis* var BCG | strain | 0.03 |
| *M. terrae* | strain | 0.03 |
| *M. hiberniae* | DNA | 0.26 |
| *M. xenopi* | DNA | 0.03 |
| *M. avium* | strain | 0.006 |
| *M. kansasii* | strain | 0.01 |
| *M. flavescens* | strain | 0.01 |
| *M. fortuitum* | strain | 0.02 |
| *M. smegmatis* | strain | 0.03 |
| *M. phlei* | strain | 0.01 |
| *M*. *abscessus* | strain | 0.01 |
| *M. chelonae* | DNA | 0.02 |

In conclusion, the inventors have shown that, using standard extraction conditions, the detection test detailed in example 2 can detect the targeted mycobacteria as well as the internal control, within a quadruplex pool.

The sensitivity is comprised between 0.03 and 0.005 fg/µL, for all strains but *M*. *hiberniae* (0.26 fg/µL).

### Conclusions of examples 2-5:

The inventors have demonstrated that the technique based on qPCR, with the primers, probes and conditions disclosed in these examples, provides a suitable test for detecting presence of mycobacteria without culture. This technique provides results more rapidly than the culture-based compendial method, as it provides results in 2 days or even far less. The time to results can thus be reduced by up to 96% with respect to the compendial method.

qPCR is also a technique compatible with GMP environment.

The above results moreover show that the detection of the mycobacteria is exhaustive, as illustrated *in silico* and with the proof of concept with a representative number of mycobacteria.

The test as detailed in the examples is moreover particularly efficient, as it allows the simultaneous detection with the three systems, and with the internal control.

The specificity of the detection has also been demonstrated with the alignments with other bacterial sequences; this specificity is provided by the probes.

The sensitivity of the method can be further improved by optimizing the extraction step and the conditions of the qPCR.

Finally, although the proof of concept has been established with qPCR, the inventors have also initiated tests with other techniques such as dPCR, which seems also applicable to detection, especially as exhaustivity and specificity rely on the design of the primers and probes, not on the amplification method as such.

### Example 6: Detection test for mycobacteria

Given the results obtained in examples 1-5, the inventors have thus defined a suitable method for detecting by qPCR mycobacteria in a sample, which is exhaustive and specific.

This method indeed can detect any mycobacteria, namely mycobacteria from any of the 5 following genera:
- *Mycolicibacterium*
- *Mycobacteroides*
- *Mycolicibacter*
- *Mycolicibacillus*
- *Mycobacterium*
which represents 200 species and 217 species, subspecies or variants in total (see table 4). The new classification has no impact on this number of mycobacteria.

The method relies on 3 different pairs of primers and the associated probe, designed as detailed in example 2, in order to amplify a region of the sequence of the *rrs* gene of any mycobacteria. The 3 different pairs of primers, or systems, target different regions of the *rrs* gene. The 3 associated probes are coupled to a FAM fluorophore.

A fourth pair of primers and associated probe is used for detecting an internal exogenous control. The probe of the control is coupled to Cy5, or to another fluorophore different from FAM.

The internal exogenous control can be any DNA sequence either already extracted or present in a microorganism unrelated to the mycobacteria to be detected. The control is in particular added to the samples at the extraction step. It allows to control this extraction step, in case there is no amplification of mycobacteria, it ensures that this is not linked to a defective extraction step.

All the primers, i.e. the four pairs of primers, and all the probes, i.e. the four probes, are mixed together in a single reaction mix for the PCR. A single PCR reaction is thus sufficient for detecting all the mycobacteria potentially present in a sample, corresponding to a quadruplex detection.

The primers F1-1, R1-1, F3-1, R3-1, F4-1 and R4-1 are those disclosed in example 1, table 2.

The primers and the probe suitable for the internal control are also to be added; the fluorophore coupled to the probe detecting the internal control must be different from the FAM fluorophore, in order to distinguish the amplification from the systems 1, 3 and 4, indicative of the presence of mycobacteria, from the amplification from the control system, indicative of the presence of the control.

For the qPCR, the reaction mix is prepared depending on the number of samples to be analyzed and the necessary volume for each well (between 20µL and 50 µL), with:
- Commercial Master Mix qPCR with probes : TaqPolymerase, dNTPs, Mg2+, buffer (non exhaustive list),
- Primers at a concentration between 100nM and 400nM
- Probes at a final concentration between 50nM and 250nM
- Primers and probe adapted to the internal control of extraction
- Nuclease free water qsp

The composition of the master mix and the concentrations of the primers and probes can be adjusted in order to improve the sensitivity or the specificity of the detection.

The reaction mix is then placed in the wells of a 96 well plate and the sample is then added. The plate is filmed, centrifuged, and inserted into a thermocycler.

The number of cycles is to be adapted depending on the enzyme present in the mix. The hybridization temperature is defined according to the fusion temperature of the primers and probes.

A positive control and negative controls for the extraction and for the qPCR are advantageously added.

### Example 7: Primer and probe design variations

Further to the demonstration in the preceding examples, that a defined set of primers and probes can detect specifically and exhaustively the *rrs* gene of all mycobacteria, the inventors have then demonstrated that variations of the primers and probes can be designed, without losing the specificity and exhaustivity of the initial primers and probes.

The inventors have thus studied *in silico* different variations, and more specifically addition or suppression of 1 to 4 bases, at each extremity. The study aims at defining the impact of these modifications on the specificity and exhaustivity of the initial primers and probes. In this regard, primer design software such as Primer Expresss and international database, such as PrimerBlast, have been used. The inventors have moreover tested combinations of the primer variants, and of probes.

### Materials & Methods

As an initial step, the 217 *rrs* gene sequences of mycobacterial origin, corresponding to 200 different species and 217 species, subspecies or variants in total, have been aligned with the software Geneious (10.2.6) and the program ClustalW. On this alignment, the 3 pairs of primers identified in example 2, i.e. F1-1 & R1-1, F3-1 & R3-1, and F4-1 & R4-1, are positioned.

Modifications likely to be introduced into the primers without compromising the exhaustivity are defined on the basis of this alignment view. Regarding the probes, the acceptable modifications are far less extensive, insofar as the probes are necessarily based on the sequences amplified by the primers, and thus limited to the sequence lying between the primer sequence (1 or 2 bases apart). This point also emphasizes that the sequences of the pair of primers are closely linked to the sequence of the associated probe.

### Design of the primer variations:

The primer variations are initially designed on the Primer Blast data, using the program Primer3. They are named F1-2, F1-3 for the primer F1, etc... ; F1-1, R1-1, F3-1, R3-1, F4-1 and R4-1 corresponding to the initial design. For each variation, the content of GC (percentage) and the Tm (temperature melting) are calculated. These variations are then analyzed with the software Primer Express, set with a quantification with a probe MGB (Minor Groove Binder), i.e. the same software as for the initial primers, for consistency.

For each tested primer variation, the following parameters/criteria, set by the program, are to be fulfilled in order to retain the variation:
- Primer Tm (Temperature melting) must be between 58°C and 60°C;
- Probe Tm (Temperature melting) must be between 68°C and 71°C;
- The content of GC must be between 40% and 70%, in particular around 50%.

Moreover, the difference between the Tm of a pair of primers must not exceed 2°C and the difference between the Tm of probe and of primer must be around 10°C.

In a first step, different pairs of primers have been tested, on the basis of this criteria, with the associated initial probes, i.e. S1-0, S3-0 and S4-0 without variations.

In a second step, the probe variations are then tested in combination with the pairs of primers (variations) retained and selected at the first step.

Two different types of results have finally been retained:
- The combinations of primers and probes entirely fulfilling all the criteria, as does the initial design of primers and probe. As demonstrated for the initial design, these combinations should be adapted for the selective and exhaustive amplification.
- The combinations for which one requirement is not perfectly fulfilled, for example a primer Tm comprised between 57°C and 58°C or between 60°C and 61°C, i.e. differing by 1°C of less from the set criteria; the other criteria being entirely fulfilled. These combinations are expected to be also perfectly adapted to the intended purpose.

### Specificity analysis:

Once different combinations are retained, on the basis of the criteria and selections mentioned above, the specificity of the different pairs of primers and associated probe are tested, in order to ensure that the specificity is not lost.

### Results:

### 1. Combination of primers

The different variations with respect to the primer sequences are detailed in table 6 below, specifying the modifications at the 5' terminus and 3' terminus, with respect to the initial design.

**Table 6:**

| Primers | Sequences (5'→3') | Modifications | Tm (°C) | %GC | SEQ ID |
|---|---|---|---|---|---|
| F1- 1 | CCTGGTAGTCCACGCCGTAA | Initial design | 59,6 | 60 | 1 |
| F1-2 | CCTGGTAGTCCACGCCGTAAA | 0 nt at 5' / +1 nt at 3' | 60,8 | 57 | 10 |
| F1-3 | CTGGTAGTCCACGCCGTAAA | -1 nt at 5' / +1 nt at 3' | 57,4 | 55 | 11 |
| F1-4 | TGGTAGTCCACGCCGTAAA | -2 nt at 5' / +1 nt at 3' | 56,3 | 53 | 29 |
| R1-1 | CGGATCCCAAGGAAGGAAAC | Initial design | 59,3 | 55 | 2 |
| R1-2 | GCACGGATCCCAAGGAAGGAAA | +3 nt at 5' / -1 nt at 3' | 65,1 | 55 | 30 |
| R1-3 | CACGGATCCCAAGGAAGGAAAC | +2 nt at 5' / +0 nt at 3' | 62,6 | 55 | 27 |
| R1-4 | ACGGATCCCAAGGAAGGAAACC | +1 nt at 5' / +1 nt at 3' | 63,2 | 55 | 31 |
| R1-5 | ACGGATCCCAAGGAAGGAAAC | +1 nt at 5' / +0 nt at 3' | 60,1 | 52 | 12 |
| R1-6 | GGATCCCAAGGAAGGAAACC | -1 nt at 5' / +1 nt at 3' | 58,3 | 55 | 13 |
| F3-1 | CACAGGACGCCGGTAGAGAT | Initial design | 58,8 | 60 | 4 |
| F3-2 | ACAGGACGCCGGTAGAGATA | -1 nt at 5' / +1 nt at 3' | 56 | 55 | 32 |
| F3-3 | CACAGGACGCCGGTAGAGATA | 0 nt at 5' / +1 nt at 3' | 58,8 | 57 | 14 |
| F3-4 | CAGGACGCCGGTAGAGATA | -2 nt at 5' / +1 nt at 3' | 55 | 58 | 33 |
| R3-1 | CATGCACCACCTGCACACA | Initial design | 58,7 | 58 | 5 |
| R3-2 | CATGCACCACCTGCACACAG | +0 nt at 5' / +1 nt at 3' | 60,8 | 60 | 15 |
| R3-3 | ATGCACCACCTGCACACAG | -1 nt at 5' / +1 nt at 3' | 57,6 | 58 | 16 |
| R3-4 | TGCACCACCTGCACACAG | -2 nt at 5' / +1 nt at 3' | 57,1 | 61 | 17 |
| F4-1 | CCCTTATGTCCAGGGCTTCA | Initial design | 58,6 | 55 | 7 |
| F4-2 | CCCCTTATGTCCAGGGCTTCA | +1 nt at 5' / +0 nt at 3' | 62 | 57 | 34 |
| F4-3 | CCCCTTATGTCCAGGGCTTC | +1 nt at 5' / -1 nt at 3' | 59,4 | 60 | 18 |
| F4-4 | GCCCCTTATGTCCAGGGCTTC | +2 nt at 5' / -1 nt at 3' | 62,7 | 62 | 35 |
| F4-5 | GCCCCTTATGTCCAGGGC | +2 nt at 5' / -4 nt at 3' | 58,9 | 67 | 19 |
| R4-1 | GGCATCGCAGCCCTTTG | Initial design | 59,9 | 65 | 8 |
| R4-2 | CGGCATCGCAGCCCTTT | +1 nt at 5' / -1 nt at 3' | 61,8 | 65 | 36 |
| R4-3 | CGGCATCGCAGCCCTT | +1 nt at 5' / -2 nt at 3' | 60,5 | 69 | 20 |
| R4-4 | GCATCGCAGCCCTTTGT | -1 nt at 5' / +1 nt at 3' | 56,8 | 59 | 37 |
| R4- 5 | GCATCGCAGCCCTTTGTA | -1 nt at 5' / +2 nt at 3' | 57 | 56 | 28 |
| R4-6 | GGCATCGCAGCCCTTTGTA | +0 nt at 5' / +2 nt at 3' | 60,7 | 58 | 21 |

For each system, i.e. system 1, system 3 and system 4, which amplifies a distinct region of the *rrs* gene, each variation of the forward primer is tested with each variation of the reverse primer, including the original design, with the PrimerExpress software.

F1-1 is thus tested independently with R1-1 (initial pair, without variation), R1-2, R1-3, R1-4, R1-5 and R1-6. The same applies to F1-2, F1-3 and F1-4.

The same strategy of combinations is applied to systems 3 and 4.

It is stressed in this respect that, a given variation of the forward primer may indeed be suitable in combination with a specific variation of the reverse primer and unsuitable in combination with another variation of the reverse primer. This is the reason why the variations of the forward and reverse primers are tested in pairs.

The following tables (tables 7, 8 and 9) report, for each system, the combinations for which either all the criteria are met (++), those for which only one parameter does not completely fulfil one of the criteria, but the difference remains acceptable (+), generally the Tm differs by 1°C from the targeted Tm , and those for which one or more of the criteria are not fulfilled (-), which represent less favorable primer sequences.

**Table 7: System 1**

| 1 | F1-1 | F1-2 | F1-3 | F1-4 |
|---|---|---|---|---|
| R1-1 | Combination 1 (++) | Combination 7 (+) | Combination 13 (-) | Combination 19 (-) |
| R1-2 | Combination 2 (-) | Combination 8 (-) | Combination 14 (-) | Combination 20 (-) |
| R1-3 | Combination 3 (-) | Combination 9 (+) | Combination 15 (-) | Combination 21 (-) |
| R1-4 | Combination 4 (-) | Combination 10 (-) | Combination 16 (-) | Combination 22 (-) |
| R1-5 | Combination 5 (+) | Combination11 (+) | Combination 17 (-) | Combination 23 (-) |
| R1-6 | Combination 6 (++) | Combination 12 (+) | Combination 18 (+) | Combination 24 (-) |

**Table 8: System 3**

| 1 | F3-1 | F3-2 | F3-3 | F3-4 |
|---|---|---|---|---|
| R3-1 | Combination 1 (++) | Combination 5 (-) | Combination 9 (++) | Combination 13 (-) |
| R3-2 | Combination 2 (+) | Combination 6 (-) | Combination 10 (+) | Combination 14 (-) |
| R3-3 | Combination 3 (+) | Combination 7 (-) | Combination 11 (+) | Combination 15 (-) |
| R3-4 | Combination 4 (+) | Combination 8 (-) | Combination 12 (+) | Combination 16 (-) |

**Table 9: System 4**

| 1 | F4-1 | F4-2 | F4-3 | F4-4 | F4-5 |
|---|---|---|---|---|---|
| R4-1 | Combination 1 (++) | Combination 7 (-) | Combination 13 (+) | Combination 19 (-) | Combination 25 (++) |
| R4-2 | Combination 2 (-) | Combination 8 (-) | Combination 14 (-) | Combination 20 (-) | Combination 26 (-) |
| R4-3 | Combination 3 (+) | Combination 9 (-) | Combination 15 (+) | Combination 21 (-) | Combination 27 (+) |
| R4-4 | Combination 4 (-) | Combination 10 (-) | Combination 16 (-) | Combination 22 (-) | Combination 28 (-) |
| R4-5 | Combination 5 (+) | Combination11 (-) | Combination 17 (-) | Combination 23 (-) | Combination 29 (-) |
| R4-6 | Combination 6 (-) | Combination 12 (-) | Combination 18 (-) | Combination 24 (-) | Combination 30 (+) |

### 2. Combination of probes

The variations of the probes S1-0, S3-0 and S4-0 which have been tested are reported in table 10, with the modifications at the 5' and 3' termini.

**Table 10:**

| Probes | Sequences (5'→3') | Modifications | Tm (°C) | % GC | SEQ ID |
|---|---|---|---|---|---|
| S1-0 | CGGTGGGTACTAGGTGTG | Initial design | 70 | 61 | 3 |
| S1-1 | CGGTGGGTACTAGGTGT | -1 nt at 3' term | 68 | 59 | 22 |
| S1-2 | CGGTGGGTACTAGGTG | -2 nt at 3' term | 69 | 63 | 23 |
| S3-0 | TCGGTTCCCTTGTGGC | Initial design | 69 | 63 | 6 |
| S3-1 | ATCGGTTCCCTTGTGGC | +1 nt at 5' term | 70 | 59 | 24 |
| S3-2 | CGGTTCCCTTGTGGC | -1 nt at 5' term | 68 | 67 | 25 |
| S4-0 | ACATGCTACAATGGCCGG | Initial design | 69 | 56 | 9 |
| S4-1 | ACATGCTACAATGGCCGGT | +1 nt at 3' term | 70 | 53 | 26 |
| S4-2 | CATGCTACAATGGCCGG | -1 nt at 5' term | 68 | 59 | 38 |
| S4-3 | CATGCTACAATGGCCG | -1 nt at 5' term / -1 nt at 3' term | 70 | 56 | 39 |

Different associations have been made, between the combinations of primers detailed in tables 7 to 9, for which the result is (++) or (+), and the associated probes and variations of table 10.

The following tables (tables 11-13) report, for each system, the associations for which either all the criteria are met (++), those for which one parameter does not completely fulfil one of the criteria, but the difference remains acceptable (+), and those associations for which one or more of the criteria are not fulfilled (-), which represent less favorable associations.

**Table 11: System 1:**

| | Combinations of primers for system 1 | | | | | | | |
|---|---|---|---|---|---|---|---|---|
| | 1 | 6 | 5 | 7 | 9 | 11 | 12 | 18 |
| S1-0 | ++ | ++ | ++ | ++ | ++ | ++ | ++ | ++ |
| S1-1 | ++ | ++ | + | + | - | - | - | + |
| S1-2 | ++ | ++ | + | + | - | + | - | + |

**Table 12: System 3:**

| | Combinations of primers for system 3 | | | | | | | |
|---|---|---|---|---|---|---|---|---|
| | 1 | 9 | 2 | 3 | 4 | 10 | 11 | 12 |
| S3-0 | ++ | ++ | ++ | ++ | ++ | ++ | ++ | ++ |
| S3-1 | ++ | ++ | + | - | - | + | + | - |
| S3-2 | ++ | ++ | + | + | - | - | + | + |

**Table 13: System 4:**

| | Combinations of primers for system 4 | | | | | | | |
|---|---|---|---|---|---|---|---|---|
| | 1 | 13 | 25 | 3 | 5 | 15 | 27 | 30 |
| S4-0 | ++ | ++ | ++ | ++ | ++ | ++ | ++ | ++ |
| S4-1 | ++ | ++ | ++ | + | - | + | + | + |
| S4-2 | ++ | ++ | ++ | - | + | + | - | - |
| S4-2 | ++ | ++ | ++ | + | - | + | + | + |

### 3. Specificity

The inventors have checked in the NCBI database, with the tool Primer BLAST, whether the combination of primers which have been retained are likely to amplify sequences other than mycobacterial sequences.

### System 1

For the combinations 6, 5, 7, 11 and 18, detailed above, the primer BLAST results show the same specificity as combination 1 (corresponding to the original primers). These variations therefore do not compromise the specificity.

### System 3:

For the combinations 9, 2, 3, 10, 11 and 12, detailed above, the primer BLAST results show the same specificity as combination 1 (corresponding to the original primers). These variations therefore do not compromise the specificity.

### System 4:

For this system, for the pair of primers disclosed in example 2, it had already been shown that the specificity was provided by the probe S4-0. In order to preserve this specificity provided by the probe, the inventors came to the conclusion that the 5' end of the probe could not be shorten, i.e. S4-2 and S4-3 were not acceptable; only the variation corresponding to S4-1 is thus retained.

For the combinations 13, 25, 3, 15, 27 and 30, detailed above, the primer BLAST results show the same specificity as combination 1 (corresponding to the original primers). These variations therefore do not compromise the specificity.

The combination 5 amplifies 13 additional species, not of mycobacterial origin; in association with probe S4-0 (initial design) the specificity can however be preserved.

These results thus show that not only the 3 pairs of primers and associated probes corresponding to
F1-1 & R1-1 with probe S1-0
F3-1 & R3-1 with probe S3-0
F4-1 & R4-1 with probe S4-0
have shown their capacity to specifically and exhaustively detect mycobacterial by an amplification method based on PCR with a detection by a probe, but that the same capacity is shared by minor variations of the primers and of the probes.

### Example 8: Further validation of the specificity.

The specificity of the method disclosed in examples 4 and 5, demonstrated in Example 2, has been validated by the inventors, especially specificity with regard to other non-mycobacterial bacteria, and specificity also in presence of matrices.

### 8.1. Tests on other species.

Four other species are selected to evaluate the specificity of the qPCR in the conditions detailed in examples 4 and 5, with the primers and probes F1-1, R1-1, S1-0, F3-1, R3-1, S3-0, F4-1, R4-1 and S4-0. The species chosen by the inventors in this regard are species considered as very close phylogenetically to the *Mycobacterium* genus (actinomyces class), namely:
- *Nocardia asteroides*
- *Cutibacterium acnes*
- *Corynebacterium diphteriae*
- *Dietzia papillomatosis*

Each strain was characterized by a viable concentration of 100 CFU/mL.

The mycobacteria qPCR testing is performed on the mycobacteria strain *M. tuberculosis* var BCG (positive control) and on the different actinomyces strains, at the same time and with the same experimental conditions.

The results obtained for *N. asteroides* at 100 CFU/mL and for *D*. *papillomatosis* at 100 CFU/mL are illustrated in FIG 4A and 4B respectively. Comparable results are obtained with *C*. *acnes* at 100 CFU/mL and *C*. *diphteriae* at 100 CFU/mL.

These results show a specific detection of mycobacteria strain while no detection for the other 4 actinomycetales strains, at a number of cycles between 37 and 40, depending on the strains. For the experimental conditions of this test, the cycle threshold is thus about 37 cycles.

These results demonstrate that the qPCR, with the primers and probes designed by the inventors, is specific for the detection of mycobacteria strains.

### Comparison with the compendial assay:

In order to compare the specificity of the qPCR method of the invention with the compendial method, based on culture, the compendial assay is performed with the same strains, at the same concentrations. For all four actinomycetales strains, a positive result is obtained on spiked liquid media.

*N. asteroides, C*. *acnes, C. diphteriae, D. papillomatosis* are thus detected by the mycobacteria testing on media (corresponding to the compendial assay) but not by the qPCR of the invention ("qPCR mycobacteria"), detecting specifically mycobacteria.

The method according to the invention thus allows a specific detection, more specific that the classical method implying culture.

### 8.2. Tests on matrices.

The qPCR mycobacteria according to the invention can be used for pharmaceutical product testing.

The inventors have indeed validated that this technique can be implemented with different matrices, confirming both the absence of non-specific cross detection, and the absence of PCR inhibition by the matrix components. The qPCR mycobacteria technique can thus be implemented for pharmaceutical product testing. The qPCR mycobacteria technique can thus be implemented in a process for producing a biopharmaceutical product as a step of testing for mycobacterial contamination.

The matrices tested by the inventors are the following:
- Supernatant of CHO cells producing recombinant cytomegalovirus protein antigens;
- HAV Hepatitis A virus crude harvest produced on MRC5 cells;
- Yellow Fever virus crude harvest produced on Vero cells;
- Rabies virus crude harvest produced on Vero cells.

The mycobacteria qPCR testing is performed on the matrices, spiked with mycobacteria strain *M*. *tuberculosis* var BCG either at different concentrations from 10⁵ to 10 viable mycobacteria per mL, or at the two concentrations 10⁵ (to simulate a high level of contamination) and 10 (to simulate a low level of contamination) viable mycobacteria per mL, in the experimental conditions detailed in examples 4 and 5, with the primers and probes F1-1, R1-1, S1-0, F3-1, R3-1, S3-0, F4-1, R4-1 and S4-0.

The results obtained are similar on matrices and on PBS samples; some of them are presented in FIG 5A, 5B and 5C.

Conclusion: the qPCR mycobacteria according to the invention allows the detection of *M*. *tuberculosis* var BCG on different matrices. This confirms that the matrices do not induce PCR inhibition and there is no non-specific amplification on non-spiked matrices. The qPCR of the invention is thus suitable for pharmaceutical product testing, at any step of the pharmaceutical product production, inter alia on the final product, on any intermediate matrices, on virus seeds, and on starting material, such as cell culture or media.

### Example 9: Demonstration of qPCR sensitivity.

### 9.1. Mycobacteria samples tested:

| Species | ATCC^{®} number | Viable mycobacteria/mL | Copy of Genome/mL | Ratio CG/viable mycobacteria |
|---|---|---|---|---|
| *M. tuberculosis* var BCG | N/A * | 1,16.10⁷ | 8,40. 10⁶ | **<1** |
| *M. avium* | 15769^{™} | 5,95.10⁷ | 8,07. 10⁶ | **<1** |
| *M. phlei* | 11758^{™} | 1,25.10⁶ | 2.39. 10⁷ | **19,1** |
| *M. hiberniae* | 49874^{™} | 1.10⁷ | 4,44.10⁷ | **4,44** |
| *M. xenopi* | 19970^{™} | 8,79.10⁵ | 8,97.10⁶ | **10,2** |
| *M. flavescens* | 23033^{™} | 5,32.10⁶ | 2,39. 10⁷ | **4,49** |
| *M. fortuitum* | 19542^{™} | 4,31.10⁷ | 1,38. 10⁷ | **<1** |
| *M. kansasii* | 21982^{™} | 9,69.10⁷ | 5,68. 10⁶ | **<1** |
| *M*. *abscessus* | 23040^{™} | 1,88.10⁷ | 1,15. 10⁶ | **<1** |

| | | | | |
|---|---|---|---|---|
| *= internal strain | | | | |

### 9.2. Matrices tested.

For *M*. *tuberculosis* var BCG, *M*. *avium, M. hiberniae*, *M. xenopi* and *M. phlei:* Vero cells suspension at the high concentration of >10⁶ cells/mL, in a medium containing serum and antibiotics, which are molecules well known as interfering with nucleic acid-based assays, corresponding to the "worst-case scenario" likely to be encountered in pharmaceutical product production.

For *M*. *flavescens, M. fortuitum, M. abscessus* and *M*. *kansasii:* the Vero Yellow Fever virus crude harvest matrix.

### 9.3. Method.

A dilution range of each strain detailed in section 9.1 is carried out in PBS, up to 10, or 1 viable mycobacteria per mL (depending on the tested strain). Samples with matrices (see 9.2) are spiked with the appropriate dilutions of mycobacteria strains, according to table 14.

**Table 14: Final dilutions in the matrices per mycobacteria strain**

| Species | Dilution 1 | Dilution 2 | Dilution 3 |
|---|---|---|---|
| *M. tuberculosis var BCG* | 100 | 10 | 1 |
| *M. avium* | 100 | 10 | 1 |
| *M. hiberniae* | 100 | 10 | 1 |
| *M. xenopi* | 10 | 1 | 0,1 |
| *M. phlei* | 10 | 1 | 0,1 |
| *M. flavescens* | 10 | 1 | NA |
| *M. fortuitum* | 10 | 1 | NA |
| *M. kansasii* | 100 | 10 | NA |
| *M*. *abscessus* | 100 | 10 | NA |

The volume of the sample to be analyzed is 1,5mL. Given the extraction volume and the fact that the whole extraction product is to be analyzed by qPCR, there are 6 determinations for one sample.

The sample is considered as positive for the presence of a mycobacterial strain, if one of the 6 determination is positive (significative amplification).

### 9.4. Results

### • M. tuberculosis var BCG at 1 viable mycobacteria/mL:

4/6 determinations show a positive and significant PCR amplification (i.e. above the threshold, at around 37-40 PCR cycles, contrary to the negative (non-spiked) control). See FIG. 6A.

The test is thus positive for this concentration and 1 viable mycobacteria/mL constitutes the limit of detection.

### • M. avium at 1 viable mycobacteria/mL:

4/6 determinations show a positive and significant PCR amplification (i.e. above the threshold, at around 37-40 PCR cycles, contrary to the negative (non-spiked) control).

The test is positive for this concentration and 1 viable mycobacteria/mL constitutes the limit of detection.

### • M. hiberniae at 1 viable mycobacteria/mL:

6/6 determinations show a positive and significant PCR amplification (i.e. above the threshold, at around 37-40 PCR cycles, contrary to the negative (non-spiked) control). See FIG. 6B.

The test is positive for this concentration and 1 viable mycobacteria/mL constitutes the limit of detection.

### • M. xenopi at 0,1 viable mycobacteria/mL:

6/6 determinations show a positive and significant PCR amplification (i.e. above the threshold, at around 37-40 PCR cycles, contrary to the negative (non-spiked) control).

The test is positive for this concentration and 0.1 viable mycobacteria/mL constitutes the limit of detection.

### • M. phlei at 0,1 viable mycobacteria/mL:

6/6 determinations show a positive and significant PCR amplification (i.e. above the threshold, at around 37-40 PCR cycles, contrary to the negative (non-spiked) control). See FIG. 6C.

The test is positive for this concentration and 0.1 viable mycobacteria/mL constitutes the limit of detection.

### • M. flavescens at 10 viable mycobacteria/mL:

4/6 determinations have positive and significant PCR amplifications (i.e. above the threshold, at around 37-40 PCR cycles, contrary to the negative (non-spiked) control).

The test is positive for this concentration and 10 viable mycobacteria/mL constitutes the limit of detection.

### • M. fortuitum at 10 viable mycobacteria/mL:

3/6 determinations have positive and significant PCR amplifications (i.e. above the threshold, at around 37-40 PCR cycles, contrary to the negative (non-spiked) control).

The test is positive for this concentration and 10 viable mycobacteria/mL constitutes the limit of detection.

### • M. abscessus at 10 viable mycobacteria/mL:

2/6 determinations have positive and significant PCR amplifications (i.e. above the threshold, at around 37-40 PCR cycles, contrary to the negative (non-spiked) control).

The test is positive for this concentration and 10 viable mycobacteria/mL constitutes the limit of detection.

### • M. kansasii at 10 viable mycobacteria/mL:

5/6 determinations have positive and significant PCR amplifications (i.e. above the threshold, at around 37-40 PCR cycles, contrary to the negative (non-spiked) control).

The test is positive for this concentration and 10 viable mycobacteria/mL constitutes the limit of detection.

### 9.5. Conclusion

The Limit of detection is at 10 viable mycobacteria/mL for: *M. flavescens, M. fortuitum, M*. *abscessus* and *M. kansasii* in a complex matrix (crude harvest of Vero Yellow Fever virus).

The limit of detection is at 1 viable mycobacteria/mL for: *M. tuberculosis* var BCG, *M*. *avium* and *M*. *hiberniae* in a complex matrix (highly concentrated Vero cells culture).

The limit of detection is at 0.1 viable mycobacteria/mL for: *M. xenopi* and *M. phlei* in a complex matrix (highly concentrated Vero cells culture).

This confirms the very high sensitivity of the method, which allows detection of representative mycobacterial strains at concentrations as low as 10 or 1 viable mycobacteria per mL, and even lower for some strains, even in complex matrices, such as highly concentrated Vero cell culture or viral crude harvest vaccine. The qPCR mycobacteria technique can thus be implemented in a process for producing a biopharmaceutical product as a step of testing for mycobacterial contamination.

### List of references:

Bhatnagar, J. et al. (2017). Improved Detection and Accuracy of Mycobacterium Species Identification from Paraffin Embedded Tissues of Patients by Using Multigene Targeted PCR and Sequencing. Open Forum Infectious Diseases 4, S620-S621.
Euzéby, J.P. (1997). List of Bacterial Names with Standing in Nomenclature: a Folder Available on the Internet. International Journal of Systematic Bacteriology, 47, 590-592.
Gupta, R.S., Lo, B., and Son, J. (2018). Phylogenomics and Comparative Genomic Studies Robustly Support Division of the Genus Mycobacterium into an Emended Genus Mycobacterium and Four Novel Genera. Frontiers in Microbiology 9, 67.
Haig, S.-J., Kotlarz, N., LiPuma, J.J., and Raskin, L. (2018). A High-Throughput Approach for Identification of Nontuberculous Mycobacteria in Drinking Water Reveals Relationship between Water Age and Mycobacterium avium. MBio 9. 9:e02354-17.
Meehan, C.J., Barco, R.A., Loh, Y.-H.E., Cogneau, S., and Rigouts, L. (2021). Reconstituting the genus Mycobacterium. International Journal of Systematic and Evolutionary Microbiology 71:004922.
Parte, A.C. (2014). LPSN - List of Prokaryotic names with Standing in Nomenclature. Nucleic Acids Research, 42, Issue D1, D613-D616
Parte, A.C. (2018). LPSN - List of Prokaryotic names with Standing in Nomenclature (bacterio.net), 20 years on. International Journal of Systematic and Evolutionary Microbiology, 68, 1825-1829.
Parte, A.C., et al. (2020). List of Prokaryotic names with Standing in Nomenclature (LPSN) moves to the DSMZ. International Journal of Systematic and Evolutionary Microbiology, 70, 5607-5612.
Riojas, M.A. et al. (2018). Phylogenomic analysis of the species of the Mycobacterium tuberculosis complex demonstrates that Mycobacterium africanum, Mycobacterium bovis, Mycobacterium caprae, Mycobacterium microti and Mycobacterium pinnipedii are later heterotypic synonyms of Mycobacterium tuberculosis. Int J Syst Evol Microbiol. 68(1):324-332.
Tortoli E, Brown-Elliott BA, Chalmers JD, et al. (2019) Same meat, different graw: ignore the new names of mycobacteria. Eur Respir J 2019; 54.

## Claims

1. A set of primers for amplifying by Polymerase Chain Reaction (PCR) a sequence of any mycobacterial *rrs* gene present in a sample, wherein said set comprises at least the three following pairs of primers:
1^{st} Pair:
Forward primer F1 having the sequence: 5'-CCTGGTAGTCCACGCCGTAA-3' (SEQ ID NO:1) or modifications thereof,
Reverse primer R1 having the sequence: 5'-CGGATCCCAAGGAAGGAAAC-3' (SEQ ID NO:2) or modifications thereof;
2^{nd} Pair:
Forward primer F3 having the sequence: 5'-CACAGGACGCCGGTAGAGAT-3' (SEQ ID NO:4) or modifications thereof,
Reverse primer R3 having the sequence: 5'-CATGCACCACCTGCACACA-3' (SEQ ID NO:5) or modifications thereof;
3^{rd} Pair:
Forward primer F4 having the sequence: 5'-CCCTTATGTCCAGGGCTTCA-3' (SEQ ID NO:7) or modifications thereof,
Reverse primer R4 having the sequence: 5'-GGCATCGCAGCCCTTTG-3' (SEQ ID NO:8) or modifications thereof;
wherein said modifications are the addition and/or the deletion of 1 to 4 nucleotides, at the 3' and/or 5' terminus, and/or the substitution of 1 or 2 nucleotides, and
wherein said three pairs of primers allow the amplification of a sequence from the *rrs* gene of any mycobacterial species.

2. The set of primers according to claim 1, wherein the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
5) Forward primer F1-2: 5'-CCTGGTAGTCCACGCCGTAAA-3' (SEQ ID NO:10) Reverse primer R1-3: 5'- CACGGATCCCAAGGAAGGAAAC-3' (SEQ ID NO:27);
6) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12);
7) Forward primer F1-2: 5'-CCTGGTAGTCCACGCCGTAAA-3' (SEQ ID NO:10) Reverse primer R1-6: 5'-GGATCCCAAGGAAGGAAACC-3' (SEQ ID NO:13); and
8) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13).

3. The set of primers according to any one of claims 1 to 2, wherein the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
5) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15);
6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
7) Forward primer F3-1: 5'-CACAGGACGCCGGTAGAGAT-3' (SEQ ID NO:4) Reverse primer R3-4: 5'-TGCACCACCTGCACACAG-3' (SEQ ID NO:17). and
8) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17).

4. The set of primers according to any one of claims 1 to 3, wherein the third pair of primers is chosen from:
1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
5) Forward primer F4-1: 5'-CCCTTATGTCCAGGGCTTCA-3' (SEQ ID NO:7), Reverse primer R4-5: 5'- GCATCGCAGCCCTTTGTA-3' (SEQ ID NO:28);
6) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
7) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
8) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

5. A kit for amplifying by PCR mycobacterial *rrs* gene sequences in a sample comprising a set of primers according to any one of claims 1-4.

6. The kit according to claim 5, for the detection of mycobacterial *rrs* gene sequences amplified by PCR in a sample, said kit further comprising the following associated probes for the detection of the amplified product(s):
- Probe S1 having the sequence 5'-CGGTGGGTACTAGGTGTG-3' (SEQ ID NO:3) or modifications thereof, for detection of the sequences amplified by said primers F1 and R1;
- Probe S3 having the sequence: 5'-TCGGTTCCCTTGTGGC-3' (SEQ ID NO:6) or modifications thereof, for detection of the sequences amplified by said primers F3 and R3;
- Probe S4 having the sequence: 5'-ACATGCTACAATGGCCGG-3' (SEQ ID NO:9) or modifications thereof, for detection of the sequences amplified by said primers F4 and R4,
wherein said modifications are the addition and/or the deletion of 1 to 2 nucleotides, at the 3' and/or 5' terminus.

7. The kit according to claim 6, wherein the probes are chosen:
a) For the Probe S1, from the group consisting of :
Probe S1-0: 5'-CGGTGGGTACTAGGTGTG-3' (SEQ ID NO:3),
Probe S1-1: 5'-CGGTGGGTACTAGGTGT-3' (SEQ ID NO:22) and
Probe S1-2: 5'-CGGTGGGTACTAGGTG-3' (SEQ ID NO:23);
wherein when the Probe S1 is S1-1, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2); and
5) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
when the Probe S1 is S1-2, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
5) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12); and
6) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
b) For the Probe S3, from the group consisting of :
Probe S3-0: 5'-TCGGTTCCCTTGTGGC-3' (SEQ ID NO:6),
Probe S3-1: 5'-ATCGGTTCCCTTGTGGC-3' (SEQ ID NO:24) and
Probe S3-2: 5'-CGGTTCCCTTGTGGC-3' (SEQ ID NO:25);
wherein when the Probe S3 is S3-1, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15); and
5) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
when the Probe S3 is S3-2, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
5) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17); and
6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
c) For the Probe S4, from the group consisting of :
Probe S4-0: 5'-ACATGCTACAATGGCCGG-3' (SEQ ID NO:9), and
Probe S4-1: 5'-ACATGCTACAATGGCCGGT-3' (SEQ ID NO:26);
wherein when the Probe S4 is S4-1, the third pair of primers is chosen from:
1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
5) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
6) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
7) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

8. The kit according to any one of claims 5 to 7, for amplification by quantitative Polymerase Chain Reaction (qPCR) or by digital Polymerase Chain Reaction (dPCR).

9. A method for amplifying and detecting sequences of the *rrs* gene of any mycobacteria present in a sample, comprising the following steps, carried out simultaneously or sequentially:
a) Carrying out *in vitro* PCR on the nucleic acids extracted from said sample, with at least the set of three pairs of primers according to any one of claims 1-4;
b) Detecting the presence or absence of an amplified product.

10. The method according to claim 9, wherein the amplification step a) is carried out by quantitative PCR or digital PCR.

11. The method according to any one of claims 9 to 10, wherein the detection step b) is carried out with the following associated probes:
i) Probe S1 selected from the group consisting of:
Probe S1-0: 5'-CGGTGGGTACTAGGTGTG-3' (SEQ ID NO:3),
Probe S1-1: 5'-CGGTGGGTACTAGGTGT-3' (SEQ ID NO:22) and
Probe S1-2: 5'-CGGTGGGTACTAGGTG-3' (SEQ ID NO:23);
Wherein when the Probe S1 is S1-1, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2); and
5) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
when the Probe S1 is S1-2, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
5) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12); and
6) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
ii) Probe S3 selected from the group consisting of:
Probe S3-0: 5'-TCGGTTCCCTTGTGGC-3' (SEQ ID NO:6),
Probe S3-1: 5'-ATCGGTTCCCTTGTGGC-3' (SEQ ID NO:24) and
Probe S3-2: 5'-CGGTTCCCTTGTGGC-3' (SEQ ID NO:25);
wherein when the Probe S3 is S3-1, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15); and
5) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
when the Probe S3 is S3-2, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
5) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17); and
6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
iii) Probe S4 selected from the group consisting of :
Probe S4-0: 5'-ACATGCTACAATGGCCGG-3' (SEQ ID NO:9), and
Probe S4-1: 5'-ACATGCTACAATGGCCGGT-3' (SEQ ID NO:26);
wherein when the Probe S4 is S4-1, the third pair of primers is chosen from:
1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
5) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
6) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
7) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

12. The method according to claim 11, wherein the amplification step a) is carried out simultaneously with said three pairs of primers and said associated probes, in a single reaction mix.

13. The method according to any one of claims 9-12, wherein the method comprises an initial step of extracting the nucleic acids from the sample, before carrying out step a).

14. Method for detecting the presence of mycobacterial contamination in a pharmaceutical product, without any step of culture, comprising the simultaneous following steps:
a) amplification *in vitro* by quantitative PCR (qPCR) or dPCR, carried out on the nucleic acids extracted from a sample of said product, with at least the three pairs of primers according to any one of claims 1-4, wherein said primers hybridize to the *rrs* gene,
b) detection of the presence or absence of an amplified product by the following probes:
i. Probe S1 selected from the group consisting of:
Probe S1-0: 5'-CGGTGGGTACTAGGTGTG-3' (SEQ ID NO:3),
Probe S1-1: 5'-CGGTGGGTACTAGGTGT-3' (SEQ ID NO:22) and
Probe S1-2: 5'-CGGTGGGTACTAGGTG-3' (SEQ ID NO:23);
wherein when the Probe S1 is S1-1, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2); and
5) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
when the Probe S1 is S1-2, the first pair of primers is chosen from:
1) Forward primer F1-1: 5'-CCTGGTAGTCCACGCCGTAA-3' (SEQ ID NO:1) Reverse primer R1-1: 5'-CGGATCCCAAGGAAGGAAAC-3' (SEQ ID NO:2);
2) Forward primer F1-1: 5'-CCTGGTAGTCCACGCCGTAA-3' (SEQ ID NO:1) Reverse primer R1-6: 5'-GGATCCCAAGGAAGGAAACC-3' (SEQ ID NO:13);
3) Forward primer F1-1: 5'-CCTGGTAGTCCACGCCGTAA-3' (SEQ ID NO:1) Reverse primer R1-5: 5'-ACGGATCCCAAGGAAGGAAAC-3' (SEQ ID NO:12);
4) Forward primer F1-2: 5'-CCTGGTAGTCCACGCCGTAAA-3' (SEQ ID NO:10) Reverse primer R1-1: 5'-CGGATCCCAAGGAAGGAAAC-3' (SEQ ID NO:2);
5) Forward primer F1-2: 5'-CCTGGTAGTCCACGCCGTAAA-3' (SEQ ID NO:10) Reverse primer R1-5: 5'-ACGGATCCCAAGGAAGGAAAC-3' (SEQ ID NO:12); and
6) Forward primer F1-3: 5'-CTGGTAGTCCACGCCGTAAA-3' (SEQ ID NO:11) Reverse primer R1-6: 5'-GGATCCCAAGGAAGGAAACC-3' (SEQ ID NO:13);
ii. Probe S3 selected from the group consisting of:
Probe S3-0: 5'-TCGGTTCCCTTGTGGC-3' (SEQ ID NO:6),
Probe S3-1: 5'-ATCGGTTCCCTTGTGGC-3' (SEQ ID NO:24) and
Probe S3-2: 5'-CGGTTCCCTTGTGGC-3' (SEQ ID NO:25);
wherein when the Probe S3 is S3-1, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-3: 5'-CACAGGACGCCGGTAGAGATA-3' (SEQ ID NO:14) Reverse primer R3-2: 5'-CATGCACCACCTGCACACAG-3' (SEQ ID NO:15); and
5) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
When the Probe S3 is S3-2, the second pair of primers is chosen from:
1) Forward primer F3-1: 5'-CACAGGACGCCGGTAGAGAT-3' (SEQ ID NO:4) Reverse primer R3-1: 5'-CATGCACCACCTGCACACA-3' (SEQ ID NO:5);
2) Forward primer F3-3: 5'-CACAGGACGCCGGTAGAGATA-3' (SEQ ID NO:14) Reverse primer R3-1: 5'-CATGCACCACCTGCACACA-3' (SEQ ID NO:5);
3) Forward primer F3-1: 5'-CACAGGACGCCGGTAGAGAT-3' (SEQ ID NO:4) Reverse primer R3-2: 5'-CATGCACCACCTGCACACAG-3' (SEQ ID NO:15);
4) Forward primer F3-1: 5'-CACAGGACGCCGGTAGAGAT-3' (SEQ ID NO:4) Reverse primer R3-3: 5'-ATGCACCACCTGCACACAG-3' (SEQ ID NO:16);
5) Forward primer F3-3: 5'-CACAGGACGCCGGTAGAGATA-3' (SEQ ID NO:14) Reverse primer R3-4: 5'-TGCACCACCTGCACACAG-3' (SEQ ID NO:17); and
6) Forward primer F3-3: 5'-CACAGGACGCCGGTAGAGATA-3' (SEQ ID NO:14) Reverse primer R3-3: 5'-ATGCACCACCTGCACACAG-3' (SEQ ID NO:16);
iii. Probe S4 selected from the group consisting of :
Probe S4-0: 5'-ACATGCTACAATGGCCGG-3' (SEQ ID NO:9), and
Probe S4-1: 5'-ACATGCTACAATGGCCGGT-3' (SEQ ID NO:26);
wherein when the Probe S4 is S4-1, the third pair of primers is chosen from:
1) Forward primer F4-1: 5'-CCCTTATGTCCAGGGCTTCA-3' (SEQ ID NO:7), Reverse primer R4-1: 5'-GGCATCGCAGCCCTTTG-3' (SEQ ID NO:8);
2) Forward primer F4-3: 5'-CCCCTTATGTCCAGGGCTTC-3' (SEQ ID NO:18), Reverse primer R4-1: 5'-GGCATCGCAGCCCTTTG-3' (SEQ ID NO:8);
3) Forward primer F4-5: 5'-GCCCCTTATGTCCAGGGC-3' (SEQ ID NO:19), Reverse primer R4-1: 5'-GGCATCGCAGCCCTTTG-3' (SEQ ID NO:8);
4) Forward primer F4-1: 5'-CCCTTATGTCCAGGGCTTCA-3' (SEQ ID NO:7), Reverse primer R4-3: 5'-CGGCATCGCAGCCCTT-3' (SEQ ID NO:20);
5) Forward primer F4-3: 5'-CCCCTTATGTCCAGGGCTTC-3' (SEQ ID NO:18), Reverse primer R4-3: 5'-CGGCATCGCAGCCCTT-3' (SEQ ID NO:20);
6) Forward primer F4-5: 5'-GCCCCTTATGTCCAGGGC-3' (SEQ ID NO:19), Reverse primer R4-3: 5'-CGGCATCGCAGCCCTT-3' (SEQ ID NO:20); and
7) Forward primer F4-5: 5'-GCCCCTTATGTCCAGGGC-3' (SEQ ID NO:19), Reverse primer R4-6: 5'-GGCATCGCAGCCCTTTGTA-3' (SEQ ID NO:21).

15. A process for producing a biopharmaceutical product comprising a step of testing for mycobacterial contamination by carrying out the method according to any one of claims 9 to 14.

## Patentansprüche

1. Satz von Primern zum Amplifizieren einer Sequenz eines beliebigen mykobakteriellen rrs-Gens, das in einer Probe vorhanden ist, durch Polymerase-Kettenreaktion (PCR), wobei der Satz wenigstens die drei folgenden Paare von Primern umfasst:
1. Paar:
Vorwärtsprimer F1 mit der Sequenz: 5'-CCTGGTAGTCCACGCCGTAA-3' (SEQ ID NO:1) oder Modifikationen davon,
Rückwärtsprimer R1 mit der Sequenz: 5'-CGGATCCCAAGGAAGGAAAC-3' (SEQ ID NO:2) oder Modifikationen davon;
2. Paar:
Vorwärtsprimer F3 mit der Sequenz: 5'-CACAGGACGCCGGTAGAGAT-3' (SEQ ID NO:4) oder Modifikationen davon,
Rückwärtsprimer R3 mit der Sequenz: 5'-CATGCACCACCTGCACACA-3' (SEQ ID NO:5) oder Modifikationen davon;
3. Paar:
Vorwärtsprimer F4 mit der Sequenz: 5'-CCCTTATGTCCAGGGCTTCA-3' (SEQ ID NO:7) oder Modifikationen davon,
Rückwärtsprimer R4 mit der Sequenz: 5'-GGCATCGCAGCCCTTTG-3' (SEQ ID NO:8) oder Modifikationen davon;
wobei die Modifikationen die Addition und/oder die Deletion von 1 bis 4 Nukleotiden an dem 3'- und/oder 5'-Terminus und/oder die Substitution von 1 oder 2 Nukleotiden sind, und
wobei die drei Paare von Primern die Amplifikation einer Sequenz aus dem rrs-Gen einer beliebigen mykobakteriellen Spezies ermöglichen.

2. Satz von Primern nach Anspruch 1, wobei das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
5) Vorwärtsprimer F1-2: 5'-CCTGGTAGTCCACGCCGTAAA-3' (SEQ ID NO:10)
Rückwärtsprimer R1-3: 5'-CACGGATCCCAAGGAAGGAAAC-3' (SEQ ID NO:27);
6) Vorwärtsprimer F1-2: 5'-CCTGGTAGTCCACGCCGTAAA-3' (SEQ ID NO:10)
Rückwärtsprimer R1-5: 5'-ACGGATCCCAAGGAAGGAAAC-3' (SEQ ID NO:12);
7) Vorwärtsprimer F1-2: 5'-CCTGGTAGTCCACGCCGTAAA-3' (SEQ ID NO:10)
Rückwärtsprimer R1-6: 5'-GGATCCCAAGGAAGGAAACC-3' (SEQ ID NO:13);
und
8) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13).

3. Satz von Primern nach einem der Ansprüche 1 bis 2, wobei das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-1: 5'-CACAGGACGCCGGTAGAGAT-3' (SEQ ID NO:4)
Rückwärtsprimer R3-3: 5'-ATGCACCACCTGCACACAG-3' (SEQ ID NO:16);
5) Vorwärtsprimer F3-3: 5'-CACAGGACGCCGGTAGAGATA-3' (SEQ ID NO:14)
Rückwärtsprimer R3-2: 5'-CATGCACCACCTGCACACAG-3' (SEQ ID NO:15);
6) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
7) Vorwärtsprimer F3-1: 5'-CACAGGACGCCGGTAGAGAT-3' (SEQ ID NO:4)
Rückwärtsprimer R3-4: 5'-TGCACCACCTGCACACAG-3' (SEQ ID NO:17).
und
8) Vorwärtsprimer F3-3: 5'-CACAGGACGCCGGTAGAGATA-3' (SEQ ID NO:14)
Rückwärtsprimer R3-4: 5'-TGCACCACCTGCACACAG-3' (SEQ ID NO:17).

4. Satz von Primern nach einem der Ansprüche 1 bis 3, wobei das dritte Paar von Primern ausgewählt ist aus:
1) Vorwärtsprimer F4-1: 5'-CCCTTATGTCCAGGGCTTCA-3' (SEQ ID NO:7),
Rückwärtsprimer R4-1: 5'-GGCATCGCAGCCCTTTG-3' (SEQ ID NO:8);
2) Vorwärtsprimer F4-3: 5'-CCCCTTATGTCCAGGGCTTC-3' (SEQ ID NO:18),
Rückwärtsprimer R4-1: 5'-GGCATCGCAGCCCTTTG-3' (SEQ ID NO:8);
3) Vorwärtsprimer F4-5: 5'-GCCCCTTATGTCCAGGGC-3' (SEQ ID NO:19),
Rückwärtsprimer R4-1: 5'-GGCATCGCAGCCCTTTG-3' (SEQ ID NO:8);
4) Vorwärtsprimer F4-1: 5'-CCCTTATGTCCAGGGCTTCA-3' (SEQ ID NO:7),
Rückwärtsprimer R4-3: 5'-CGGCATCGCAGCCCTT-3' (SEQ ID NO:20);
5) Vorwärtsprimer F4-1: 5'-CCCTTATGTCCAGGGCTTCA-3' (SEQ ID NO:7),
Rückwärtsprimer R4-5: 5'-GCATCGCAGCCCTTTGTA-3' (SEQ ID NO:28);
6) Vorwärtsprimer F4-3: 5'-CCCCTTATGTCCAGGGCTTC-3' (SEQ ID NO:18),
Rückwärtsprimer R4-3: 5'-CGGCATCGCAGCCCTT-3' (SEQ ID NO:20);
7) Vorwärtsprimer F4-5: 5'-GCCCCTTATGTCCAGGGC-3' (SEQ ID NO:19),
Rückwärtsprimer R4-3: 5'-CGGCATCGCAGCCCTT-3' (SEQ ID NO:20);
und
8) Vorwärtsprimer F4-5: 5'-GCCCCTTATGTCCAGGGC-3' (SEQ ID NO:19),
Rückwärtsprimer R4-6: 5'-GGCATCGCAGCCCTTTGTA-3' (SEQ ID NO:21).

5. Kit zur Amplifikation mykobakterieller rrs-Gensequenzen in einer Probe umfassend einen Satz von Primern nach einem der Ansprüche 1-4 durch PCR.

6. Kit nach Anspruch 5 zum Nachweisen von durch PCR amplifizierten mykobakteriellen rrs-Gensequenzen in einer Probe, wobei das Kit ferner die folgenden zugehörigen Sonden zum Nachweisen des oder der amplifizierten Produkte umfasst:
- Sonde S1 mit der Sequenz 5'-CGGTGGGTACTAGGTGTG-3' (SEQ ID NO:3) oder Modifikationen davon zum Nachweisen der durch die Primer F1 und R1 amplifizierten Sequenzen;
- Sonde S3 mit der Sequenz: 5'-TCGGTTCCCTTGTGGC-3' (SEQ ID NO:6) oder Modifikationen davon zum Nachweisen der durch die Primer F3 und R3 amplifizierten Sequenzen;
- Sonde S4 mit der Sequenz: 5'-ACATGCTACAATGGCCGG-3' (SEQ ID NO:9) oder Modifikationen davon zum Nachweisen der durch die Primer F4 und R4 amplifizierten Sequenzen, wobei die Modifikationen die Addition und/oder die Deletion von 1 bis 2 Nukleotiden an dem 3'- und/oder 5'-Terminus sind.

7. Kit nach Anspruch 6, wobei die Sonden ausgewählt sind:
a) für die Sonde S1 aus der Gruppe bestehend aus:
Sonde S1-0: 5'-CGGTGGGTACTAGGTGTG-3' (SEQ ID NO:3),
Sonde S1-1: 5'-CGGTGGGTACTAGGTGT-3' (SEQ ID NO:22) und
Sonde S1-2: 5'-CGGTGGGTACTAGGTG-3' (SEQ ID NO:23);
wobei, wenn die Sonde S1 S1-1 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5¹-CGGATCCCAAGGAAGGAAAC-3¹ (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
und
5) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
wenn die Sonde S1 S1-2 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5¹-GGATCCCAAGGAAGGAAACC-3¹ (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
5) Vorwärtsprimer F1-2: 5'-CCTGGTAGTCCACGCCGTAAA-3' (SEQ ID NO:10)
Rückwärtsprimer R1-5: 5'-ACGGATCCCAAGGAAGGAAAC-3' (SEQ ID NO:12);
und
6) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
b) für die Sonde S3 aus der Gruppe bestehend aus:
Sonde S3-0: 5'-TCGGTTCCCTTGTGGC-3' (SEQ ID NO:6),
Sonde S3-1: 5'-ATCGGTTCCCTTGTGGC-3' (SEQ ID NO:24) und
Sonde S3-2: 5'-CGGTTCCCTTGTGGC-3' (SEQ ID NO:25);
wobei, wenn die Sonde S3 S3-1 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-3: 5'-CACAGGACGCCGGTAGAGATA-3' (SEQ ID NO:14)
Rückwärtsprimer R3-2: 5'-CATGCACCACCTGCACACAG-3' (SEQ ID NO:15);
und
5) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
wenn die Sonde S3 S3-2 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-1: 5'-CACAGGACGCCGGTAGAGAT-3' (SEQ ID NO:4)
Rückwärtsprimer R3-3: 5'-ATGCACCACCTGCACACAG-3' (SEQ ID NO:16);
5) Vorwärtsprimer F3-3: 5'-CACAGGACGCCGGTAGAGATA-3' (SEQ ID NO:14)
Rückwärtsprimer R3-4: 5'-TGCACCACCTGCACACAG-3' (SEQ ID NO:17);
und
6) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
c) für die Sonde S4 aus der Gruppe bestehend aus:
Sonde S4-0: 5'-ACATGCTACAATGGCCGG-3' (SEQ ID NO:9), und Sonde S4-1: 5'-ACATGCTACAATGGCCGGT-3' (SEQ ID NO:26);
wobei, wenn die Sonde S4 S4-1 ist, das dritte Paar von Primern ausgewählt ist aus:
1) Vorwärtsprimer F4-1: 5'-CCCTTATGTCCAGGGCTTCA-3' (SEQ ID NO:7),
Rückwärtsprimer R4-1: 5'-GGCATCGCAGCCCTTTG-3' (SEQ ID NO:8);
2) Vorwärtsprimer F4-3: 5'-CCCCTTATGTCCAGGGCTTC-3' (SEQ ID NO:18),
Rückwärtsprimer R4-1: 5'-GGCATCGCAGCCCTTTG-3' (SEQ ID NO:8);
3) Vorwärtsprimer F4-5: 5'-GCCCCTTATGTCCAGGGC-3' (SEQ ID NO:19),
Rückwärtsprimer R4-1: 5'-GGCATCGCAGCCCTTTG-3' (SEQ ID NO:8);
4) Vorwärtsprimer F4-1: 5'-CCCTTATGTCCAGGGCTTCA-3' (SEQ ID NO:7),
Rückwärtsprimer R4-3: 5'-CGGCATCGCAGCCCTT-3' (SEQ ID NO:20);
5) Vorwärtsprimer F4-3: 5'-CCCCTTATGTCCAGGGCTTC-3' (SEQ ID NO:18),
Rückwärtsprimer R4-3: 5'-CGGCATCGCAGCCCTT-3' (SEQ ID NO:20);
6) Vorwärtsprimer F4-5: 5'-GCCCCTTATGTCCAGGGC-3' (SEQ ID NO:19),
Rückwärtsprimer R4-3: 5'-CGGCATCGCAGCCCTT-3' (SEQ ID NO:20);
und
7) Vorwärtsprimer F4-5: 5'-GCCCCTTATGTCCAGGGC-3' (SEQ ID NO:19),
Rückwärtsprimer R4-6: 5'-GGCATCGCAGCCCTTTGTA-3' (SEQ ID NO:21).

8. Kit nach einem der Ansprüche 5 bis 7 zur Amplifikation durch quantitative Polymerase-Kettenreaktion (qPCR) oder durch digitale Polymerase-Kettenreaktion (dPCR).

9. Verfahren zum Amplifizieren und Nachweisen von Sequenzen des rrs-Gens beliebiger in einer Probe vorhandener Mykobakterien, umfassend die folgenden Schritte, gleichzeitig oder nacheinander durchgeführt:
a) Durchführen einer In-vitro-PCR an den aus der Probe extrahierten Nukleinsäuren mit wenigstens dem Satz von drei Paaren von Primern nach einem der Ansprüche 1-4;
b) Nachweisen des Vorhandenseins oder Nichtvorhandenseins eines amplifizierten Produkts.

10. Verfahren nach Anspruch 9, wobei der Amplifikationsschritt a) durch quantitative PCR oder digitale PCR durchgeführt wird.

11. Verfahren nach einem der Ansprüche 9 bis 10, wobei der Nachweisschritt b) mit den folgenden zugehörigen Sonden durchgeführt wird:
i) Sonde S1 ausgewählt aus der Gruppe bestehend aus:
Sonde S1-0: 5'-CGGTGGGTACTAGGTGTG-3' (SEQ ID NO:3),
Sonde S1-1: 5'-CGGTGGGTACTAGGTGT-3' (SEQ ID NO:22) und
Sonde S1-2: 5'-CGGTGGGTACTAGGTG-3' (SEQ ID NO:23);
wobei, wenn die Sonde S1 S1-1 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
und
5) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
wenn die Sonde S1 S1-2 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
5) Vorwärtsprimer F1-2: 5'-CCTGGTAGTCCACGCCGTAAA-3' (SEQ ID NO:10)
Rückwärtsprimer R1-5: 5'-ACGGATCCCAAGGAAGGAAAC-3' (SEQ ID NO:12);
und
6) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
ii) Sonde S3 ausgewählt aus der Gruppe bestehend aus:
Sonde S3-0: 5'-TCGGTTCCCTTGTGGC-3' (SEQ ID NO:6),
Sonde S3-1: 5'-ATCGGTTCCCTTGTGGC-3' (SEQ ID NO:24) und Sonde S3-2: 5'-CGGTTCCCTTGTGGC-3' (SEQ ID NO:25);
wobei, wenn die Sonde S3 S3-1 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-3: 5'-CACAGGACGCCGGTAGAGATA-3' (SEQ ID NO:14)
Rückwärtsprimer R3-2: 5'-CATGCACCACCTGCACACAG-3' (SEQ ID NO:15);
und
5) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
wenn die Sonde S3 S3-2 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-1: 5'-CACAGGACGCCGGTAGAGAT-3' (SEQ ID NO:4)
Rückwärtsprimer R3-3: 5'-ATGCACCACCTGCACACAG-3' (SEQ ID NO:16);
5) Vorwärtsprimer F3-3: 5'-CACAGGACGCCGGTAGAGATA-3' (SEQ ID NO:14)
Rückwärtsprimer R3-4: 5'-TGCACCACCTGCACACAG-3' (SEQ ID NO:17);
und
6) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
iii) Sonde S4 ausgewählt aus der Gruppe bestehend aus Sonde S4-0: 5'-ACATGCTACAATGGCCGG-3' (SEQ ID NO:9), und Sonde S4-1: 5'-ACATGCTACAATGGCCGGT-3' (SEQ ID NO:26);
wobei, wenn die Sonde S4 S4-1 ist, das dritte Paar von Primern ausgewählt ist aus:
1) Vorwärtsprimer F4-1: 5'-CCCTTATGTCCAGGGCTTCA-3' (SEQ ID NO:7),
Rückwärtsprimer R4-1: 5'-GGCATCGCAGCCCTTTG-3' (SEQ ID NO:8);
2) Vorwärtsprimer F4-3: 5'-CCCCTTATGTCCAGGGCTTC-3' (SEQ ID NO:18),
Rückwärtsprimer R4-1: 5'-GGCATCGCAGCCCTTTG-3' (SEQ ID NO:8);
3) Vorwärtsprimer F4-5: 5'-GCCCCTTATGTCCAGGGC-3' (SEQ ID NO:19),
Rückwärtsprimer R4-1: 5'-GGCATCGCAGCCCTTTG-3' (SEQ ID NO:8);
4) Vorwärtsprimer F4-1: 5'-CCCTTATGTCCAGGGCTTCA-3' (SEQ ID NO:7),
Rückwärtsprimer R4-3: 5'-CGGCATCGCAGCCCTT-3' (SEQ ID NO:20);
5) Vorwärtsprimer F4-3: 5'-CCCCTTATGTCCAGGGCTTC-3' (SEQ ID NO:18),
Rückwärtsprimer R4-3: 5'-CGGCATCGCAGCCCTT-3' (SEQ ID NO:20);
6) Vorwärtsprimer F4-5: 5'-GCCCCTTATGTCCAGGGC-3' (SEQ ID NO:19),
Rückwärtsprimer R4-3: 5'-CGGCATCGCAGCCCTT-3' (SEQ ID NO:20);
und
7) Vorwärtsprimer F4-5: 5'-GCCCCTTATGTCCAGGGC-3' (SEQ ID NO:19),
Rückwärtsprimer R4-6: 5'-GGCATCGCAGCCCTTTGTA-3' (SEQ ID NO:21).

12. Verfahren nach Anspruch 11, wobei der Amplifikationsschritt a) mit den drei Paaren von Primern und den zugehörigen Sonden in einem einzigen Reaktionsgemisch gleichzeitig durchgeführt wird.

13. Verfahren nach einem der Ansprüche 9-12, wobei das Verfahren einen Anfangsschritt des Extrahierens der Nukleinsäuren aus der Probe vor der Durchführung von Schritt a) umfasst.

14. Verfahren zum Nachweisen des Vorhandenseins einer mykobakteriellen Kontamination in einem pharmazeutischen Produkt ohne einen Kulturschritt, umfassend die gleichzeitigen folgenden Schritte:
a) Amplifikation in vitro durch quantitative PCR (qPCR) oder dPCR, durchgeführt an den aus einer Probe des Produkts extrahierten Nukleinsäuren, mit wenigstens den drei Paaren von Primern nach einem der Ansprüche 1-4, wobei die Primer an das rrs-Gen hybridisieren,
b) Nachweisen des Vorhandenseins oder Nichtvorhandenseins eines amplifizierten Produkts durch die folgenden Sonden:
i. Sonde S1 ausgewählt aus der Gruppe bestehend aus
Sonde S1-0: 5'-CGGTGGGTACTAGGTGTG-3' (SEQ ID NO:3),
Sonde S1-1: 5'-CGGTGGGTACTAGGTGT-3' (SEQ ID NO:22) und
Sonde S1-2: 5'-CGGTGGGTACTAGGTG-3' (SEQ ID NO:23);
wobei, wenn die Sonde S1 S1-1 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
und
5) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
wenn die Sonde S1 S1-2 ist, das erste Paar von Primern ausgewählt ist aus:
1) Vorwärtsprimer F1-1: 5'-CCTGGTAGTCCACGCCGTAA-3' (SEQ ID NO:1)
Rückwärtsprimer R1-1: 5'-CGGATCCCAAGGAAGGAAAC-3' (SEQ ID NO:2);
2) Vorwärtsprimer F1-1: 5'-CCTGGTAGTCCACGCCGTAA-3' (SEQ ID NO:1)
Rückwärtsprimer R1-6: 5'-GGATCCCAAGGAAGGAAACC-3' (SEQ ID NO:13);
3) Vorwärtsprimer F1-1: 5'-CCTGGTAGTCCACGCCGTAA-3' (SEQ ID NO:1)
Rückwärtsprimer R1-5: 5'-ACGGATCCCAAGGAAGGAAAC-3' (SEQ ID NO:12);
4) Vorwärtsprimer F1-2: 5'-CCTGGTAGTCCACGCCGTAAA-3' (SEQ ID NO:10)
Rückwärtsprimer R1-1: 5'-CGGATCCCAAGGAAGGAAAC-3' (SEQ ID NO:2);
5) Vorwärtsprimer F1-2: 5'-CCTGGTAGTCCACGCCGTAAA-3' (SEQ ID NO:10)
Rückwärtsprimer R1-5: 5'-ACGGATCCCAAGGAAGGAAAC-3' (SEQ ID NO:12);
und
6) Vorwärtsprimer F1-3: 5'-CTGGTAGTCCACGCCGTAAA-3' (SEQ ID NO:11)
Rückwärtsprimer R1-6: 5'-GGATCCCAAGGAAGGAAACC-3' (SEQ ID NO:13);
ii. Sonde S3 ausgewählt aus der Gruppe bestehend aus:
Sonde S3-0: 5'-TCGGTTCCCTTGTGGC-3' (SEQ ID NO:6),
Sonde S3-1: 5'-ATCGGTTCCCTTGTGGC-3' (SEQ ID NO:24) und
Sonde S3-2: 5'-CGGTTCCCTTGTGGC-3' (SEQ ID NO:25);
wobei, wenn die Sonde S3 S3-1 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-3: 5'-CACAGGACGCCGGTAGAGATA-3' (SEQ ID NO:14)
Rückwärtsprimer R3-2: 5'-CATGCACCACCTGCACACAG-3' (SEQ ID NO:15);
und
5) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
wenn die Sonde S3 S3-2 ist, das zweite Paar von Primern ausgewählt ist aus:
1) Vorwärtsprimer F3-1: 5'-CACAGGACGCCGGTAGAGAT-3' (SEQ ID NO:4)
Rückwärtsprimer R3-1: 5'-CATGCACCACCTGCACACA-3' (SEQ ID NO:5);
2) Vorwärtsprimer F3-3: 5'-CACAGGACGCCGGTAGAGATA-3' (SEQ ID NO:14)
Rückwärtsprimer R3-1: 5'-CATGCACCACCTGCACACA-3' (SEQ ID NO:5);
3) Vorwärtsprimer F3-1: 5'-CACAGGACGCCGGTAGAGAT-3' (SEQ ID NO:4)
Rückwärtsprimer R3-2: 5'-CATGCACCACCTGCACACAG-3' (SEQ ID NO:15);
4) Vorwärtsprimer F3-1: 5'-CACAGGACGCCGGTAGAGAT-3' (SEQ ID NO:4)
Rückwärtsprimer R3-3: 5'-ATGCACCACCTGCACACAG-3' (SEQ ID NO:16);
5) Vorwärtsprimer F3-3: 5'-CACAGGACGCCGGTAGAGATA-3' (SEQ ID NO:14)
Rückwärtsprimer R3-4: 5'-TGCACCACCTGCACACAG-3' (SEQ ID NO:17);
und
6) Vorwärtsprimer F3-3: 5'-CACAGGACGCCGGTAGAGATA-3' (SEQ ID NO:14)
Rückwärtsprimer R3-3: 5'-ATGCACCACCTGCACACAG-3' (SEQ ID NO:16);
iii. Sonde S4 ausgewählt ist aus der Gruppe bestehend aus
Sonde S4-0: 5'-ACATGCTACAATGGCCGG-3' (SEQ ID NO:9), und
Sonde S4-1: 5'-ACATGCTACAATGGCCGGT-3' (SEQ ID NO:26);
wobei, wenn die Sonde S4 S4-1 ist, das dritte Paar von Primern ausgewählt ist aus:
1) Vorwärtsprimer F4-1: 5'-CCCTTATGTCCAGGGCTTCA-3' (SEQ ID NO:7),
Rückwärtsprimer R4-1: 5'-GGCATCGCAGCCCTTTG-3' (SEQ ID NO:8);
2) Vorwärtsprimer F4-3: 5'-CCCCTTATGTCCAGGGCTTC-3' (SEQ ID NO:18),
Rückwärtsprimer R4-1: 5'-GGCATCGCAGCCCTTTG-3' (SEQ ID NO:8);
3) Vorwärtsprimer F4-5: 5'-GCCCCTTATGTCCAGGGC-3' (SEQ ID NO:19),
Rückwärtsprimer R4-1: 5'-GGCATCGCAGCCCTTTG-3' (SEQ ID NO:8);
4) Vorwärtsprimer F4-1: 5'-CCCTTATGTCCAGGGCTTCA-3' (SEQ ID NO:7),
Rückwärtsprimer R4-3: 5'-CGGCATCGCAGCCCTT-3' (SEQ ID NO:20);
5) Vorwärtsprimer F4-3: 5'-CCCCTTATGTCCAGGGCTTC-3' (SEQ ID NO:18),
Rückwärtsprimer R4-3: 5'-CGGCATCGCAGCCCTT-3' (SEQ ID NO:20);
6) Vorwärtsprimer F4-5: 5'-GCCCCTTATGTCCAGGGC-3' (SEQ ID NO:19),
Rückwärtsprimer R4-3: 5'-CGGCATCGCAGCCCTT-3' (SEQ ID NO:20);
und
7) Vorwärtsprimer F4-5: 5'-GCCCCTTATGTCCAGGGC-3' (SEQ ID NO:19),
Rückwärtsprimer R4-6: 5'-GGCATCGCAGCCCTTTGTA-3' (SEQ ID NO:21).

15. Verfahren zur Herstellung eines biopharmazeutischen Produkts, umfassend einen Schritt des Testens auf mykobakterielle Kontamination durch Durchführen des Verfahrens nach einem der Ansprüche 9 bis 14.

## Revendications

1. Ensemble d'amorces pour amplifier, par réaction en chaîne par polymérase (PCR), une séquence de tout gène rrs mycobactérien présent dans un échantillon, ledit ensemble comprenant au moins les trois paires d'amorces suivantes :
1^{re} paire :
l'amorce sens F1 ayant la séquence : 5'-CCTGGTAGTCCACGCCGTAA-3' (SEQ ID N° : 1) ou des modifications de celle-ci,
l'amorce antisens R1 ayant la séquence : 5'-CGGATCCCAAGGAAGGAAAC-3' (SEQ ID N° : 2) ou des modifications de celle-ci ;
2^{e} paire :
l'amorce sens F3 ayant la séquence : 5'-CACAGGACGCCGGTAGAGAT-3' (SEQ ID N° : 4) ou des modifications de celle-ci,
l'amorce antisens R3 ayant la séquence : 5'-CATGCACCACCTGCACACA-3' (SEQ ID N° : 5) ou des modifications de celle-ci ;
3^{e} paire :
l'amorce sens F4 ayant la séquence : 5'-CCCTTATGTCCAGGGCTTCA-3' (SEQ ID N° : 7) ou des modifications de celle-ci,
l'amorce antisens R4 ayant la séquence : 5'-GGCATCGCAGCCCTTTG-3' (SEQ ID N° : 8) ou des modifications de celle-ci ;
dans lequel lesdites modifications sont l'addition et/ou la délétion de 1 à 4 nucléotides, à l'extrémité 3' et/ou 5', et/ou la substitution de 1 ou 2 nucléotides et
dans lequel lesdites trois paires d'amorces permettent l'amplification d'une séquence du gène *rrs* de toute espèce mycobactérienne.

2. Ensemble d'amorces selon la revendication 1, dans lequel la première paire d'amorces est choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
5) l'amorce sens F1-2 : 5'-CCTGGTAGTCCACGCCGTAAA-3' (SEQ ID N° : 10)
l'amorce antisens R1-3 : 5'-CACGGATCCCAAGGAAGGAAAC-3' (SEQ ID N° : 27) ;
6) l'amorce sens F1-2 : 5'-CCTGGTAGTCCACGCCGTAAA-3' (SEQ ID N° : 10)
l'amorce antisens R1-5 : 5'-ACGGATCCCAAGGAAGGAAAC-3' (SEQ ID N° : 12) ;
7) l'amorce sens F1-2 : 5'-CCTGGTAGTCCACGCCGTAAA-3' (SEQ ID N° : 10)
l'amorce antisens R1-6 : 5'-GGATCCCAAGGAAGGAAACC-3' (SEQ ID N° : 13) ;
et
8) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13).

3. Ensemble d'amorces selon l'une quelconque des revendications 1 à 2, dans lequel la deuxième paire d'amorces est choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-1 : 5'-CACAGGACGCCGGTAGAGAT-3' (SEQ ID NO : 4)
l'amorce antisens R3-3 : 5'-ATGCACCACCTGCACACAG-3' (SEQ ID N° : 16) ;
5) l'amorce sens F3-3 : 5'-CACAGGACGCCGGTAGAGATA-3' (SEQ ID N° : 14)
l'amorce antisens R3-2 : 5'-CATGCACCACCTGCACACAG-3' (SEQ ID N° : 15) ;
6) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
7) l'amorce sens F3-1 : 5'-CACAGGACGCCGGTAGAGAT-3' (SEQ ID N° : 4)
l'amorce antisens R3-4 : 5'-TGCACCACCTGCACACAG-3' (SEQ ID N° : 17)
et
8) l'amorce sens F3-3 : 5'-CACAGGACGCCGGTAGAGATA-3' (SEQ ID N° : 14)
l'amorce antisens R3-4 : 5'-TGCACCACCTGCACACAG-3' (SEQ ID N° : 17)

4. Ensemble d'amorces selon l'une quelconque des revendications 1 à 3, dans lequel la troisième paire d'amorces est choisie parmi :
1) l'amorce sens F4-1 : 5'-CCCTTATGTCCAGGGCTTCA-3' (SEQ ID N° : 7),
l'amorce antisens R4-1 : 5'-GGCATCGCAGCCCTTTG-3' (SEQ ID N° : 8) ;
2) l'amorce sens F4-3 : 5'-CCCCTTATGTCCAGGGCTTC-3' (SEQ ID N° : 18),
l'amorce antisens R4-1 : 5'-GGCATCGCAGCCCTTTG-3' (SEQ ID N° : 8) ;
3) l'amorce sens F4-5 : 5'-GCCCCTTATGTCCAGGGC-3' (SEQ ID N° : 19),
l'amorce antisens R4-1 : 5'-GGCATCGCAGCCCTTTG-3' (SEQ ID N° : 8) ;
4) l'amorce sens F4-1 : 5'-CCCTTATGTCCAGGGCTTCA-3' (SEQ ID NO : 7),
l'amorce antisens R4-3 : 5'-CGGCATCGCAGCCCTT-3' (SEQ ID N° : 20) ;
5) l'amorce sens F4-1 : 5'-CCCTTATGTCCAGGGCTTCA-3' (SEQ ID N° : 7),
l'amorce antisens R4-5 : 5'-GCATCGCAGCCCTTTGTA-3' (SEQ ID N° : 28) ;
6) l'amorce sens F4-3 : 5'-CCCCTTATGTCCAGGGCTTC-3' (SEQ ID N° : 18),
l'amorce antisens R4-3 : 5'-CGGCATCGCAGCCCTT-3' (SEQ ID N° : 20) ;
7) l'amorce sens F4-5 : 5'-GCCCCTTATGTCCAGGGC-3' (SEQ ID N° : 19),
l'amorce antisens R4-3 : 5'-CGGCATCGCAGCCCTT-3' (SEQ ID N° : 20) ;
et
8) l'amorce sens F4-5 : 5'-GCCCCTTATGTCCAGGGC-3' (SEQ ID N° : 19),
l'amorce antisens R4-6 : 5'-GGCATCGCAGCCCTTTGTA-3' (SEQ ID N° : 21).

5. Kit pour amplifier par PCR des séquences de gène *rrs* mycobactérien dans un échantillon, comprenant un ensemble d'amorces selon l'une quelconque des revendications 1 à 4.

6. Kit selon la revendication 5, pour la détection de séquences de gène *rrs* mycobactérien amplifiées par PCR dans un échantillon, ledit kit comprenant en outre les sondes associées suivantes pour la détection du ou des produits amplifiés :
- la sonde S1 ayant la séquence 5'-CGGTGGGTACTAGGTGTG-3' (SEQ ID N° : 3) ou des modifications de celle-ci, pour la détection des séquences amplifiées par lesdites amorces F1 et R1 ;
- la sonde S3 ayant la séquence : 5'-TCGGTTCCCTTGTGGC-3' (SEQ ID N° : 6) ou des modifications de celle-ci, pour la détection des séquences amplifiées par lesdites amorces F3 et R3 ;
- la sonde S4 ayant la séquence : 5'-ACATGCTACAATGGCCGG-3' (SEQ ID N° : 9) ou des modifications de celle-ci, pour la détection des séquences amplifiées par lesdites amorces F4 et R4, dans lequel lesdites modifications sont l'addition et/ou la délétion de 1 à 2 nucléotides, à l'extrémité 3' et/ou 5'.

7. Kit selon la revendication 6, dans lequel les sondes sont choisies :
a) pour la sonde S1, dans le groupe constitué par :
la sonde S1-0 : 5'-CGGTGGGTACTAGGTGTG-3' (SEQ ID N° : 3),
la sonde S1-1 : 5'-CGGTGGGTACTAGGTGT-3' (SEQ ID N° : 22) et
la sonde S1-2 : 5'-CGGTGGGTACTAGGTG-3' (SEQ ID N° : 23) ;
lorsque la sonde S1 est S1-1, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
et
5) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
lorsque la sonde S1 est S1-2, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
5) l'amorce sens F1-2 : 5'-CCTGGTAGTCCACGCCGTAAA-3' (SEQ ID N° : 10)
l'amorce antisens R1-5 : 5'-ACGGATCCCAAGGAAGGAAAC-3' (SEQ ID N° : 12) ;
et
6) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
b) pour la sonde S3, dans le groupe constitué par :
la sonde S3-0 : 5'-TCGGTTCCCTTGTGGC-3' (SEQ ID N° : 6), la sonde S3-1 : 5'-ATCGGTTCCCTTGTGGC-3' (SEQ ID N° : 24) et
la sonde S3-2 : 5'-CGGTTCCCTTGTGGC-3' (SEQ ID N° : 25) ; lorsque la sonde S3 est S3-1, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-3 : 5'-CACAGGACGCCGGTAGAGATA-3' (SEQ ID N° : 14)
l'amorce antisens R3-2 : 5'-CATGCACCACCTGCACACAG-3' (SEQ ID N° : 15) ;
et
5) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
lorsque la sonde S3 est S3-2, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-1 : 5'-CACAGGACGCCGGTAGAGAT-3' (SEQ ID N° : 4)
l'amorce antisens R3-3 : 5'-ATGCACCACCTGCACACAG-3' (SEQ ID N° : 16) ;
5) l'amorce sens F3-3 : 5'-CACAGGACGCCGGTAGAGATA-3' (SEQ ID N° : 14)
l'amorce antisens R3-4 : 5'-TGCACCACCTGCACACAG-3' (SEQ ID N° : 17) ;
et
6) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
c) pour la sonde S4, dans le groupe constitué par :
la sonde S4-0 : 5'-ACATGCTACAATGGCCGG-3' (SEQ ID N° : 9) et
la sonde S4-1 : 5'-ACATGCTACAATGGCCGGT-3' (SEQ ID N° : 26) ;
lorsque la sonde S4 est S4-1, la troisième paire d'amorces étant choisie parmi :
1) l'amorce sens F4-1 : 5'-CCCTTATGTCCAGGGCTTCA-3' (SEQ ID N° : 7),
l'amorce antisens R4-1 : 5'-GGCATCGCAGCCCTTTG-3' (SEQ ID N° : 8) ;
2) l'amorce sens F4-3 : 5'-CCCCTTATGTCCAGGGCTTC-3' (SEQ ID N° : 18),
l'amorce antisens R4-1 : 5'-GGCATCGCAGCCCTTTG-3' (SEQ ID N° : 8) ;
3) l'amorce sens F4-5 : 5'-GCCCCTTATGTCCAGGGC-3' (SEQ ID N° : 19),
l'amorce antisens R4-1 : 5'-GGCATCGCAGCCCTTTG-3' (SEQ ID N° : 8) ;
4) l'amorce sens F4-1 : 5'-CCCTTATGTCCAGGGCTTCA-3' (SEQ ID N° : 7),
l'amorce antisens R4-3 : 5'-CGGCATCGCAGCCCTT-3' (SEQ ID N° : 20) ;
5) l'amorce sens F4-3 : 5'-CCCCTTATGTCCAGGGCTTC-3' (SEQ ID N° : 18),
l'amorce antisens R4-3 : 5'-CGGCATCGCAGCCCTT-3' (SEQ ID N° : 20) ;
6) l'amorce sens F4-5 : 5'-GCCCCTTATGTCCAGGGC-3' (SEQ ID N° : 19),
l'amorce antisens R4-3 : 5'-CGGCATCGCAGCCCTT-3' (SEQ ID N° : 20) ;
et
7) l'amorce sens F4-5 : 5'-GCCCCTTATGTCCAGGGC-3' (SEQ ID N° : 19),
l'amorce antisens R4-6 : 5'-GGCATCGCAGCCCTTTGTA-3' (SEQ ID N° : 21).

8. Kit selon l'une quelconque des revendications 5 à 7, pour l'amplification par réaction en chaîne par polymérase quantitative (qPCR) ou par réaction en chaîne par polymérase digitale (dPCR).

9. Procédé pour amplifier et détecter des séquences du gène *rrs* de toute mycobactérie présente dans un échantillon, comprenant les étapes suivantes, réalisées simultanément ou séquentiellement :
a) la réalisation d'une PCR *in vitro* sur les acides nucléiques extraits dudit échantillon, avec au moins l'ensemble de trois paires d'amorces selon l'une quelconque des revendications 1-4 ;
b) la détection de la présence ou de l'absence d'un produit amplifié.

10. Procédé selon la revendication 9, dans lequel l'étape d'amplification a) est réalisée par PCR quantitative ou PCR digitale.

11. Procédé selon l'une quelconque des revendications 9 à 10, dans lequel l'étape de détection b) est réalisée avec les sondes associées suivantes :
i) la sonde S1 choisie dans le groupe constitué par :
la sonde S1-0 : 5'-CGGTGGGTACTAGGTGTG-3' (SEQ ID N° : 3),
la sonde S1-1 : 5'-CGGTGGGTACTAGGTGT-3' (SEQ ID N° : 22) et
la sonde S1-2 : 5'-CGGTGGGTACTAGGTG-3' (SEQ ID N° : 23) ;
lorsque la sonde S1 est S1-1, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
et
5) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
lorsque la sonde S1 est S1-2, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
5) l'amorce sens F1-2 : 5'-CCTGGTAGTCCACGCCGTAAA-3' (SEQ ID N° : 10)
l'amorce antisens R1-5 : 5'-ACGGATCCCAAGGAAGGAAAC-3' (SEQ ID N° : 12) ;
et
6) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
ii) la sonde S3 choisie dans le groupe constitué par :
la sonde S3-0 : 5'-TCGGTTCCCTTGTGGC-3' (SEQ ID N° : 6),
la sonde S3-1 : 5'-ATCGGTTCCCTTGTGGC-3' (SEQ ID N° : 24) et
la sonde S3-2 : 5'-CGGTTCCCTTGTGGC-3' (SEQ ID N° : 25) ; lorsque la sonde S3 est S3-1, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-3 : 5'-CACAGGACGCCGGTAGAGATA-3' (SEQ ID N° : 14)
l'amorce antisens R3-2 : 5'-CATGCACCACCTGCACACAG-3' (SEQ ID N° : 15) ;
et
5) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
lorsque la sonde S3 est S3-2, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-1 : 5'-CACAGGACGCCGGTAGAGAT-3' (SEQ ID N° : 4)
l'amorce antisens R3-3 : 5'-ATGCACCACCTGCACACAG-3' (SEQ ID N° : 16) ;
5) l'amorce sens F3-3 : 5'-CACAGGACGCCGGTAGAGATA-3' (SEQ ID N° : 14)
l'amorce antisens R3-4 : 5'-TGCACCACCTGCACACAG-3' (SEQ ID N° : 17) ;
et
6) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
iii) la sonde S4 choisie dans le groupe constitué par :
la sonde S4-0 : 5'-ACATGCTACAATGGCCGG-3' (SEQ ID N° : 9) et
la sonde S4-1 : 5'-ACATGCTACAATGGCCGGT-3' (SEQ ID N° : 26) ;
lorsque la sonde S4 est S4-1, la troisième paire d'amorces étant choisie parmi :
1) l'amorce sens F4-1 : 5'-CCCTTATGTCCAGGGCTTCA-3' (SEQ ID N° : 7),
l'amorce antisens R4-1 : 5'-GGCATCGCAGCCCTTTG-3' (SEQ ID N° : 8) ;
2) l'amorce sens F4-3 : 5'-CCCCTTATGTCCAGGGCTTC-3' (SEQ ID N° : 18),
l'amorce antisens R4-1 : 5'-GGCATCGCAGCCCTTTG-3' (SEQ ID N° : 8) ;
3) l'amorce sens F4-5 : 5'-GCCCCTTATGTCCAGGGC-3' (SEQ ID N° : 19),
l'amorce antisens R4-1 : 5'-GGCATCGCAGCCCTTTG-3' (SEQ ID N° : 8) ;
4) l'amorce sens F4-1 : 5'-CCCTTATGTCCAGGGCTTCA-3' (SEQ ID N° : 7),
l'amorce antisens R4-3 : 5'-CGGCATCGCAGCCCTT-3' (SEQ ID N° : 20) ;
5) l'amorce sens F4-3 : 5'-CCCCTTATGTCCAGGGCTTC-3' (SEQ ID N° : 18),
l'amorce antisens R4-3 : 5'-CGGCATCGCAGCCCTT-3' (SEQ ID N° : 20) ;
6) l'amorce sens F4-5 : 5'-GCCCCTTATGTCCAGGGC-3' (SEQ ID N° : 19),
l'amorce antisens R4-3 : 5'-CGGCATCGCAGCCCTT-3' (SEQ ID N° : 20) ;
et
7) l'amorce sens F4-5 : 5'-GCCCCTTATGTCCAGGGC-3' (SEQ ID N° : 19),
l'amorce antisens R4-6 : 5'-GGCATCGCAGCCCTTTGTA-3' (SEQ ID N° : 21).

12. Procédé selon la revendication 11, dans lequel l'étape d'amplification a) est réalisée simultanément avec lesdites trois paires d'amorces et lesdites sondes associées, dans un unique mélange réactionnel.

13. Procédé selon l'une quelconque des revendications 9 à 12, dans lequel le procédé comprend une étape initiale d'extraction des acides nucléiques de l'échantillon, avant la réalisation de l'étape a).

14. Procédé pour détecter la présence de contamination mycobactérienne dans un produit pharmaceutique, sans une quelconque étape de culture, comprenant les étapes simultanées suivantes :
a) une amplification *in vitro* par PCR quantitative (qPCR) ou dPCR, réalisée sur les acides nucléiques extraits d'un échantillon dudit produit, avec au moins les trois paires d'amorces selon l'une quelconque des revendications 1-4,
dans lequel lesdites amorces s'hybrident avec le gène *rrs,*
b) la détection de la présence ou de l'absence d'un produit amplifié par les sondes suivantes :
i. la sonde S1 choisie dans le groupe constitué par :
la sonde S1-0 : 5'-CGGTGGGTACTAGGTGTG-3' (SEQ ID N° : 3),
la sonde S1-1 : 5'-CGGTGGGTACTAGGTGT-3' (SEQ ID N° : 22) et
la sonde S1-2 : 5'-CGGTGGGTACTAGGTG-3' (SEQ ID N° : 23) ;
lorsque la sonde S1 est S1-1, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
et
5) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
lorsque la sonde S1 est S1-2, la première paire d'amorces étant choisie parmi :
1) l'amorce sens F1-1 : 5'-CCTGGTAGTCCACGCCGTAA-3' (SEQ ID N° : 1)
l'amorce antisens R1-1 : 5'-CGGATCCCAAGGAAGGAAAC-3' (SEQ ID N° : 2) ;
2) l'amorce sens F1-1 : 5'-CCTGGTAGTCCACGCCGTAA-3' (SEQ ID N° : 1)
l'amorce antisens R1-6 : 5'-GGATCCCAAGGAAGGAAACC-3' (SEQ ID N° : 13) ;
3) l'amorce sens F1-1 : 5'-CCTGGTAGTCCACGCCGTAA-3' (SEQ ID N° : 1)
l'amorce antisens R1-5 : 5'-ACGGATCCCAAGGAAGGAAAC-3' (SEQ ID N° : 12) ;
4) l'amorce sens F1-2 : 5'-CCTGGTAGTCCACGCCGTAAA-3' (SEQ ID N° : 10)
l'amorce antisens R1-1 : 5'-CGGATCCCAAGGAAGGAAAC-3' (SEQ ID N° : 2) ;
5) l'amorce sens F1-2 : 5'-CCTGGTAGTCCACGCCGTAAA-3' (SEQ ID N° : 10)
l'amorce antisens R1-5 : 5'-ACGGATCCCAAGGAAGGAAAC-3' (SEQ ID N° : 12) ;
et
6) l'amorce sens F1-3 : 5'-CTGGTAGTCCACGCCGTAAA-3' (SEQ ID N° : 11)
l'amorce antisens R1-6 : 5'-GGATCCCAAGGAAGGAAACC-3' (SEQ ID N° : 13) ;
ii. la sonde S3 choisie dans le groupe constitué par :
la sonde S3-0 : 5'-TCGGTTCCCTTGTGGC-3' (SEQ ID N° : 6),
la sonde S3-1 : 5'-ATCGGTTCCCTTGTGGC-3' (SEQ ID N° : 24) et
la sonde S3-2 : 5'-CGGTTCCCTTGTGGC-3' (SEQ ID N° : 25) ; lorsque la sonde S3 est S3-1, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-3 : 5'-CACAGGACGCCGGTAGAGATA-3' (SEQ ID N° : 14)
l'amorce antisens R3-2 : 5'-CATGCACCACCTGCACACAG-3' (SEQ ID N° : 15) ;
et
5) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
lorsque la sonde S3 est S3-2, la deuxième paire d'amorces étant choisie parmi :
1) l'amorce sens F3-1 : 5'-CACAGGACGCCGGTAGAGAT-3' (SEQ ID N° : 4)
l'amorce antisens R3-1 : 5'-CATGCACCACCTGCACACA-3' (SEQ ID N° : 5) ;
2) l'amorce sens F3-3 : 5'-CACAGGACGCCGGTAGAGATA-3' (SEQ ID N° : 14)
l'amorce antisens R3-1 : 5'-CATGCACCACCTGCACACA-3' (SEQ ID N° : 5) ;
3) l'amorce sens F3-1 : 5'-CACAGGACGCCGGTAGAGAT-3' (SEQ ID N° : 4)
l'amorce antisens R3-2 : 5'-CATGCACCACCTGCACACAG-3' (SEQ ID N° : 15) ;
4) l'amorce sens F3-1 : 5'-CACAGGACGCCGGTAGAGAT-3' (SEQ ID N° : 4)
l'amorce antisens R3-3 : 5'-ATGCACCACCTGCACACAG-3' (SEQ ID N° : 16) ;
5) l'amorce sens F3-3 : 5'-CACAGGACGCCGGTAGAGATA-3' (SEQ ID N° : 14)
l'amorce antisens R3-4 : 5'-TGCACCACCTGCACACAG-3' (SEQ ID N° : 17) ;
et
6) l'amorce sens F3-3 : 5'-CACAGGACGCCGGTAGAGATA-3' (SEQ ID N° : 14)
l'amorce antisens R3-3 : 5'-ATGCACCACCTGCACACAG-3' (SEQ ID N° : 16) ;
iii. la sonde S4 choisie dans le groupe constitué par :
la sonde S4-0 : 5'-ACATGCTACAATGGCCGG-3' (SEQ ID N° : 9) et
la sonde S4-1 : 5'-ACATGCTACAATGGCCGGT-3' (SEQ ID N° : 26) ;
lorsque la sonde S4 est S4-1, la troisième paire d'amorces étant choisie parmi :
1) l'amorce sens F4-1 : 5'-CCCTTATGTCCAGGGCTTCA-3' (SEQ ID N° : 7),
l'amorce antisens R4-1 : 5'-GGCATCGCAGCCCTTTG-3' (SEQ ID N° : 8) ;
2) l'amorce sens F4-3 : 5'-CCCCTTATGTCCAGGGCTTC-3' (SEQ ID N° : 18),
l'amorce antisens R4-1 : 5'-GGCATCGCAGCCCTTTG-3' (SEQ ID N° : 8) ;
3) l'amorce sens F4-5 : 5'-GCCCCTTATGTCCAGGGC-3' (SEQ ID N° : 19),
l'amorce antisens R4-1 : 5'-GGCATCGCAGCCCTTTG-3' (SEQ ID N° : 8) ;
4) l'amorce sens F4-1 : 5'-CCCTTATGTCCAGGGCTTCA-3' (SEQ ID N° : 7),
l'amorce antisens R4-3 : 5'-CGGCATCGCAGCCCTT-3' (SEQ ID N° : 20) ;
5) l'amorce sens F4-3 : 5'-CCCCTTATGTCCAGGGCTTC-3' (SEQ ID N° : 18),
l'amorce antisens R4-3 : 5'-CGGCATCGCAGCCCTT-3' (SEQ ID N° : 20) ;
6) l'amorce sens F4-5 : 5'-GCCCCTTATGTCCAGGGC-3' (SEQ ID N° : 19),
l'amorce antisens R4-3 : 5'-CGGCATCGCAGCCCTT-3' (SEQ ID N° : 20) ;
et
7) l'amorce sens F4-5 : 5'-GCCCCTTATGTCCAGGGC-3' (SEQ ID N° : 19),
l'amorce antisens R4-6 : 5'-GGCATCGCAGCCCTTTGTA-3' (SEQ ID N° : 21).

15. Procédé pour produire un produit biopharmaceutique comprenant une étape de test de la contamination mycobactérienne par la réalisation du procédé selon l'une quelconque des revendications 9 à 14.
